# EUROPÄISCHE PATENTSCHRIFT

(11) **EP 2 931 918 B1**
(45) Veröffentlichungstag und Bekanntmachung des Hinweises auf die Patenterteilung: **12.07.2017**
(21) Anmeldenummer: 13821429.1
(22) Anmeldetag: 15.11.2013
(51) Int. Cl.: C12Q 1/68, C12N 15/63, C12P 13/08

(54) **VERFAHREN ZUR IDENTIFIZIERUNG EINER ZELLE MIT GEGENÜBER IHREM WILDTYP ERHÖHTEN INTRAZELLULÄREN KONZENTRATION EINES BESTIMMTEN METABOLITEN, WOBEI DIE VERÄNDERUNG DER ZELLE DURCH REKOMBINEERING ERREICHT WIRD, SOWIE EIN VERFAHREN ZUR HERSTELLUNG EINER GEGENÜBER IHREM WILDTYP GENETISCH VERÄNDERTEN PRODUKTIONSZELLE MIT OPTIMIERTER PRODUKTION EINES BESTIMMTEN METABOLITEN, EIN VERFAHREN ZUR HERSTELLUNG DIESES METABOLITEN, SOWIE DAFÜR GEEIGNETE NUKLEINSÄUREN.**
A METHOD OF IDENTIFYING A CELL WITH INCREASED CONCENTRATION OF A PARTICULAR METABOLITE COMPARED TO THE CORRESPONDING WILDTYPE CELL, WHEREIN THE CELL IS MODIFIED VIA RECOMBINEERING, A METHOD FOR THE PRODUCTION OF A GENETICALLY MODIFIED CELL WITH OPTIMISED PRODUCTION OF A PARTICULAR METABOLITE, A METHOD FOR THE PRODUCTION OF SAID METABOLITE AND SUITABLE NUCEIC ACIDS.
PROCÉDÉ D'IDENTIFICATION D'UNE CELLULE PRÉSENTANT, COMPARATIVEMENT À SON TYPE SAUVAGE, UNE CONCENTRATION INTRACELLULAIRE AUGMENTÉE D'UN MÉTABOLITE DÉTERMINÉ, LA MODIFICATION DE LA CELLULE S'EFFECTUANT PAR RECOMBINAISON, UN PROCÉDÉ POUR PRODUIRE UNE CELLULE DE PRODUCTION D'UN MÉTABOLITE DÉTERMINÉ, GÉNÉTIQUEMENT MODIFIÉE COMPARATIVEMENT À SON TYPE SAUVAGE, LADITE CELLULE ÉTANT À PRODUCTION OPTIMISÉE, UN PROCÉDÉ POUR PRODUIRE LEDIT MÉTABOLITE AINSI QUE DES ACIDES NUCLÉIQUES REQUIS À CET EFFET

(30) Priorität: 14.12.2012 DE 102012024435
(43) Veröffentlichungstag der Anmeldung: 21.10.2015
(73) Patentinhaber: Forschungszentrum Jülich GmbH, 52425 Jülich (DE)
(72) Erfinder: BINDER, Stephan, 52249 Eschweiler (DE); EGGELING, Lothar, 52428 Jülich (DE); BOTT, Michael, 52428 Jülich (DE)
(86) Internationale Anmeldenummer: PCT/DE2013/000683
(87) Internationale Veröffentlichungsnummer: WO 2014/090208

(56) Entgegenhaltungen:
- WO-A1-2011/138006
- WO-A2-03/040290
- US-A1- 2005 191 732
- S. BINDER ET AL: "Recombineering in Corynebacterium glutamicum combined with optical nanosensors: a general strategy for fast producer strain generation", NUCLEIC ACIDS RESEARCH, Bd. 41, Nr. 12, 28. April 2013 (2013-04-28), Seiten 6360-6369, XP055111472, ISSN: 0305-1048, DOI: 10.1093/nar/gkt312
- MICHAEL BOTT: "Single-cell metabolite sensors and recombineering as novel tools for strain and enzyme development", SYMPOSIUM ON BIOCATALYSIS, 5. November 2013 (2013-11-05), XP055112427, Mühlheim an der Ruhr
- STEPHAN BINDER ET AL: "A high-throughput approach to identify genomic variants of bacterial metabolite producers at the single-cell level", GENOME BIOLOGY, BIOMED CENTRAL LTD., LONDON, GB, Bd. 13, Nr. 5, 28. Mai 2012 (2012-05-28), Seite R40, XP021127741, ISSN: 1465-6906, DOI: 10.1186/GB-2012-13-5-R40
- ELIZABETH E. REGULSKI ET AL: "A widespread riboswitch candidate that controls bacterial genes involved in molybdenum cofactor and tungsten cofactor metabolism", MOLECULAR MICROBIOLOGY, Bd. 68, Nr. 4, 1. Mai 2008 (2008-05-01), Seiten 918-932, XP055112439, ISSN: 0950-382X, DOI: 10.1111/j.1365-2958.2008.06208.x
- STEFANIE HAMPEL ET AL: "Drosophila Brainbow: a recombinase-based fluorescence labeling technique to subdivide neural expression patterns", NATURE METHODS, Bd. 8, Nr. 3, 1. März 2011 (2011-03-01), Seiten 253-259, XP055112440, ISSN: 1548-7091, DOI: 10.1038/nmeth.1566
- DATABASE ENA [Online] 29. November 2012 (2012-11-29), Trost E, Gotker S et al: ""Complete genome sequence and lifestyle of black-pigmented Corynebacterium aurimucosum ATCC 700975 (formerly C. nigricans CN-1) isolated from a vaginal swab of a woman with spontaneous abortion"", XP002722943, Database accession no. CP001601
- DATABASE UniProt [Online] 16. Juni 2009 (2009-06-16), "SubName: Full=Putative phage recombinase; TVRVDLSESA LDYPQHVDGE VVDSKPAEGE AA", XP002725931, gefunden im EBI accession no. UNIPROT:C3PIA1 Database accession no. C3PIA1 & TROST EVA ET AL: "Complete genome sequence and lifestyle of black-pigmented Corynebacterium aurimucosum ATCC 700975 (formerly C. nigricans CN-1) isolated from a vaginal swab of a woman with spontaneous abortion", BMC GENOMICS, BIOMED CENTRAL LTD, LONDON, UK, Bd. 11, Nr. 1, 5. Februar 2010 (2010-02-05), Seite 91, XP021066220, ISSN: 1471-2164
- YUEJU WANG ET AL: "Recombinase technology: applications and possibilities", PLANT CELL REPORTS, SPRINGER, BERLIN, DE, Bd. 30, Nr. 3, 24. Oktober 2010 (2010-10-24), Seiten 267-285, XP019880902, ISSN: 1432-203X, DOI: 10.1007/S00299-010-0938-1
- SAWITZKE JAMES A ET AL: "Recombineering: in vivo genetic engineering in E. coli, S. enterica, and beyond", METHODS IN ENZYMOLOGY, ACADEMIC PRESS, US, Bd. 421, 1. Januar 2007 (2007-01-01), Seiten 171-199, XP008124026, ISSN: 0076-6879, DOI: 10.1016/S0076-6879(06)21015-2 [gefunden am 2007-03-09]
- NURIJE MUSTAFI ET AL: "The development and application of a single-cell biosensor for the detection of-methionine and branched-chain amino acids", METABOLIC ENGINEERING, ACADEMIC PRESS, US, Bd. 14, Nr. 4, 6. Februar 2012 (2012-02-06), Seiten 449-457, XP028429545, ISSN: 1096-7176, DOI: 10.1016/J.YMBEN.2012.02.002 [gefunden am 2012-02-15]
- PAUL HINDE ET AL: "The Potential of Site-Specific Recombinases as Novel Reporters in Whole-Cell Biosensors of Pollution", ADVANCES IN APPLIED MICROBIOLOGY, ACADEMIC PRESS, UNITED STATES, Bd. 52, 1. Januar 2003 (2003-01-01), Seiten 29-74, XP009177362, ISSN: 0065-2164
- KATASHKINA JOANNA I ET AL: "Use of the Î Red-recombineering method for genetic engineering of Pantoea ananatis", BMC MOLECULAR BIOLOGY, BIOMED CENTRAL LTD, GB, Bd. 10, Nr. 1, 23. April 2009 (2009-04-23) , Seite 34, XP021048298, ISSN: 1471-2199, DOI: 10.1186/1471-2199-10-34
- PFLEGER ET AL: "Microbial sensors for small molecules: Development of a mevalonate biosensor", METABOLIC ENGINEERING, ACADEMIC PRESS, US, Bd. 9, Nr. 1, 23. Dezember 2006 (2006-12-23), Seiten 30-38, XP005733339, ISSN: 1096-7176, DOI: 10.1016/J.YMBEN.2006.08.002

## Beschreibung

Die Erfindung betrifft ein Verfahren zur Identifizierung einer Zelle mit gegenüber ihrem Wildtyp erhöhten intrazellulären Konzentration eines bestimmten Metaboliten, wobei die Veränderung der Zelle durch Rekombineering erreicht wird, sowie ein Verfahren zur Herstellung einer gegenüber ihrem Wildtyp genetisch veränderten Produktionszelle mit optimierter Produktion eines bestimmten Metaboliten, ein Verfahren zur Herstellung dieses Metaboliten, sowie dafür geeignete Nukleinsäuren.

Mikroorganismen werden seit Jahrzehnten großtechnisch zur Produktion niedermolekularer Moleküle benutzt. Niedermolekulare Moleküle sind zum Beispiel natürliche bakterielle Metabolite wie Aminosäuren (EP 1070132 B1, WO 2008/006680 A8), Nukleoside und Nukleotide (EP 2097512 C1, CA 2297613 C1), Fettsäuren (WO 2009/071878 C1, WO 2011/064393 C1), Vitamine (EP 0668359 C1), organische Säuren (EP 0450491 B1, EP 0366922 B1) oder Zucker (EP 0861902 C1, US 3642575 A). Durch Bakterien produzierte niedermolekuläre Moleküle sind auch solche Moleküle, die durch Expression heterologer Gene, die zum Beispiel aus Pflanzen stammen, gebildet werden. Dies sind pflanzliche Wirkstoffe. Dazu gehören beispielsweise Taxol (WO 1996/032490 C1, WO 1993/021338 C1), Artemisinin (WO 2009/088404 C1), und weitere Moleküle, die zu den Klassen der Isoprenoide, Phenylpropanoide oder Alkaloide gehören (Marienhagen J, Bott M, 2012, J Biotechnol., doi.org/10.1016/j.jbiotec.2012.06.001). Generell können neben Molekülen, oder Vorstufen von Molekülen pflanzlichen Ursprungs auch solche Moleküle mit Mikroorganismen gewonnen werden, die von wirtschaftlichem Interesse sind. Dazu gehören zum Beispiel Hydroxyisobuttersäure zur Herstellung von Methacrylaten (PCT/EP2007/055394), Diamine zur Herstellung von Kunststoffen (JP 2009-284905 A), oder auch Alkohole zur Verwendung als Treibstoff (WO 2011/069105 C2, WO 2008/137406 C1).

Als Mikroorganismen zur Produktion niedermolekularer Moleküle sind Gram-negative Bakterien, Gram-positive Bakterien und Hefen geeignet. Geeignete Bakterien sind beispielsweise zum Genus Enterobacteria gehörende Escherichia species wie Escherichia coli, oder zum Genus Firmicutes gehörende Bacillus species wie Bacillus subtilis, oder zum Genus Firmicutes gehörende Lactococcus species wie Lactococcus lactis oder Lactobacillus species wie Lactobacillus casei, oder zum Genus Ascomycetes gehörende Saccharomyces species wie Saccharomyces cerevisiae, oder Yarrowia species wie Yarrowia lipolytica, oder zum Genus Corynebacterium gehörende Corynebacterium species.

Unter den Corynebacterien ist bevorzugt Corynebacterium efficiens (DSM44549), Corynebacterium thermoaminogenes (FERM BP-1539) und Corynebacterium ammoniagenes (ATCC6871), besonders aber Corynebacterium glutamicum (ATCC13032). Einige Spezies von Corynebacterium glutamicum sind auch unter anderem Namen dem Stand der Technik entsprechend bekannt. So zum Beispiel Corynebacterium acetoacidophilum ATCC13870, Corynebacterium lilium DSM20137, Corynebacterium melassecola ATCC17965, Brevibacterium flavum ATCC14067, Brevibacterium lactofermentum ATCC13869, Brevibacterium divaricatum ATCC14020, und Microbacterium ammoniaphilum ATCC15354.

Um die Bildung und Produktion der niedermolekularen Moleküle zu erreichen, werden eigene Gene des Mikoorganismus, oder homologe Gene oder heterologe Gene der Synthesewege der niedermolekularen Moleküle exprimiert oder verstärkt exprimiert, oder es wird deren mRNA Stabilität erhöht. Dazu können die Gene auf Plasmiden oder Vektoren in die Zelle eingebracht werden, oder sie können auf Episomen vorliegen, oder ins Chromosom integriert werden. Auch können die zelleigenen chromosomal kodierten Gene in ihrer Expression gesteigert werden. Dies wird beispielsweise durch geeignete Mutationen im Chromosom im Bereich des Promoters erreicht. Es können auch andere Mutationen, die zur Produksteigerung führen, in das Chromosom eingeführt werden, die beispielsweise die mRNA Stabilität beeinflussen, oder die osmotische Stabilität, die Resistenz gegenüber pH Schwankungen, oder auch Gene, deren Funktion nicht bekannt ist, die sich aber günstig auf die Produktbildung auswirken. Auch werden homologe Gene oder heterologe Gene in das Chromosom eingebaut oder sie werden so eingebaut, dass sie im Chromosom in mehrfachen Kopien vorliegen.

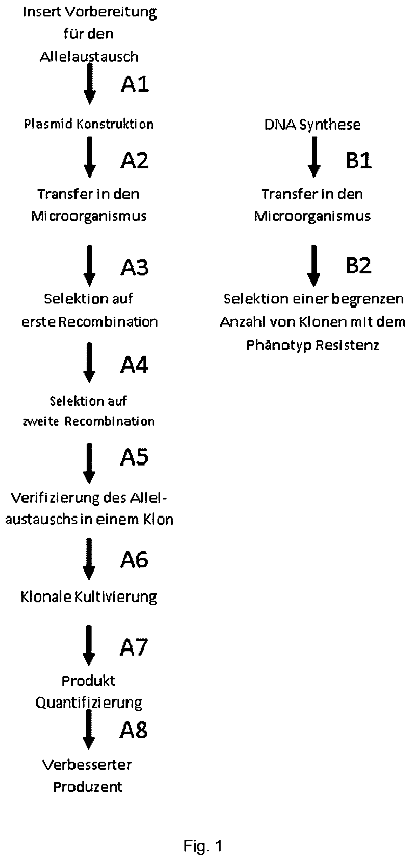

Der gezielten Einbau der Mutationen oder Gene in das Genom erfordert die Konstruktion eines Plasmids, das durch in vitro Rekombination von DNA Sequenzen unter Nutzung von Restriktionsendonukleasen und DNA Ligasen hergestellt wird. Die gesamte Prozedur, um gezielt chromosomale Mutationen einzuführen, umfasst ferner die nachfolgenden Schritte, um den in vivo Austausch zu erreichen, den Test auf erfolgreichen Austausch, und schließlich den Test auf gesteigerte Produktbildung. Dies erfordert eine Vielzahl von Schritten, A1 - A8, die in Figur 1 (links) schematisch angeführt sind. Diese Methodik wird für viele Bakterien, die zur Produktion kleiner Moleküle benutzt werden, angewandt. Beispiele sind Corynebacterium glutamicum (Small mobilizable multi-purpose cloning vectors derived from the Escherichia coli plasmids pK18 and pK19: selection of defined deletions in the chromosome of Corynebacterium glutamicum. Schäfer A, Tauch A, Jäger W, Kalinowski J, Thierbach G, Pühler A. Gene. 1994 Jul 22;145(1):69-73), oder Pseudomonas aeruginosa (Allelic exchange in Pseudomonas aeruginosa using novel ColE1-type vectors and a family of cassettes containing a portable oriT and the counter-selectable Bacillus subtilis sacB marker. Schweizer HP. Mol Microbiol. 1992 May;6(9):1195-204), oder Bacillus subtilis (Construction of a modular plasmid family for chromosomal integration in Bacillus subtilis. Gimpel M, Brantl S. J Microbiol. Methods. 2012 Nov;91(2):312-7), oder Clostridien (Novel system for efficient isolation of clostridium double-crossover allelic exchange mutants enabling markerless chromosomal gene deletions and DNA integration. Al-Hinai MA, Fast AG, Papoutsakis ET. Appl. Environ Microbiol. 2012 Nov;78(22):8112-21).

Die gezielte Einführung von Mutationen und Genen in das Chromosom erfordert die in vitro Rekombination von DNA Sequenzen unter Nutzung von Restriktionsendonukleasen und DNA Ligasen zur Herstellung eines Plasmids (Figur 1, A1). Bei den dazu erforderlichen Plasmiden handelt es sich um Plasmide, die im gewünschten Produzenten unter geeigneten Bedingungen nicht replizieren. Nach Einbringen des Plasmids in den Mikroorganismus durch Elektroporation, chemische oder ballistische Transformation (Figur 1, A2), erfolgt Integration in das Chromosom. Auf Integration wird durch Vektor-vermittelte Resistenz selektioniert (Figur 1, A3). Geeignete Plasmide sind beispielsweise pBRH1 (WO2003076452C2) oder pWV01(US6025190), die in Azetobacter beziehungsweise Bacillus nach Transformation (Figur 1, A2) durch Erhöhung der Temperatur in der Zelle nicht mehr replizieren können, so dass in resistenten Zellen der Einbau des Vektors in das Chromosom erfolgt. Das Plasmid pK19mobsacB kann von vornherein nicht in Corynebacterien, wie C. glutamicum, replizieren, so dass in Anwesenheit von Kanamycin nur Klone selektioniert werden, bei denen die Integration des Vektors in das Chromosom durch homologe Rekombination erfolgt (Schäfer et al., Gene 145, 69-73 (1994) (Figure 1, A3). Diese nicht-replizierenden Plasmide dienen als Vektor, um Gene im Chromosom gerichtet zu mutieren, Promotersequenzen zu mutieren, Sequenzen zu deletieren, oder Sequenzen auszutauschen, oder neue Gene in das Chromosom einzuführen. Dieses Verfahren ist aufwändig, da die Plasmide einzeln in vitro konstruiert werden müssen. Es ist ferner aufwändig, da über geeignete Selektionsverfahren, wie Selektion auf Antibiotikaresistenz oder die angeführte Temperaturerhöhung, zunächst der Einbau des Plasmids mitsamt der auszutauschenden Sequenzen in das Chromosom bewirkt wird, und in einem nachfolgenden Schritt wieder der Verlust des Plasmids aus dem Chromosom erreicht wird (Figur 1, A4). Durch nachfolgende Tests, üblicherweise PCR Amplifikationen, lässt sich dann erst prüfen, ob die auszutauschenden Sequenzen wie gewünscht tatsächlich im Chromosom verblieben sind (Figur 1, A5). So wird ein einzelner Klon konstruiert, der nachfolgend kultiviert wird (Figur 1, A6), dessen Produkt quantifiziert wird (Figur 1, A7), und so gegebenenfalls ein verbesserter Produzent erhalten wird (Figur 1, A8). Diese Technik der Plasmidkonstruktion und der homologen Rekombination zur Gewinnung von mikrobiellen Produzenten wird vielfach angewandt, wie beispielsweise um Allelaustausche oder Deletionen in C. glutamicum oder E. coli zu erreichen (US 8,293,514; US 8,257,943; US8,216,820; WO 2008/006680 A8; EP 2386650 C1).

In jüngster Zeit ist als weitere Methodik der gezielten Genommutation das sogenannte "Rekombineering" eingeführt. Die Mutationen einzuführen erfordert weit weniger Schritte als der Einbau von Mutationen mit Hilfe von Plasmiden (Figur 1, rechts, B1 - B2). Rekombineering nutzt Phagen- oder Prophagengene, die die homologe Rekombination zwischen der chromosomalen DNA und von außen zugeführter DNA bewirkt. Diese DNA wird im einfachsten Falle als kommerziell synthetisierte Einzelstrang-DNA eingesetzt. Auch kann mittels PCR amplifizierte Doppelstrang DNA verwendet werden. Wenn die geeigneten Phagen- oder Prophagengene vorliegen, erfordert diese Methodik nur wenige Schritte. Der Nachteil ist allerdings, dass diese Methodik im Wesentlichen auf Einführung von Mutationen, die Wachstum auf selektivem Medium erlauben, beschränkt ist, wie zum Beispiel die Einführung von Antibiotikaresistenzen, weil andere Mutationen nicht erkannt werden können. Die direkte Anwendung zur schnellen Generierung auf produktbildende Mikroorganismen ist deswegen äußerst beschränkt, und bisher nur für E. coli und das Produkt Lycopen beschrieben (Programming cells by multiplex genome engineering and accelerated evolution. Wang HH, Isaacs FJ, Carr PA, Sun ZZ, Xu G, Forest CR, Church GM. Nature. 2009;460(7257):894-8). In diesem besonderen Falle konnte aufgrund des gefärbten Lycopens anhand der Koloniefarbe auf Produktbildung geschlossen werden. Für andere Organismen und andere Produkte, wie beispielsweise Aminosäuren oder andere organische Säuren, ist eine direkte Erkennung gesteigerter Produktbildung bisher nicht möglich. Ein weiterer Nachteil ist es, dass Rekombineering auf E. coli und eine sehr begrenzte Zahl weiterer Mikroorganismen beschränkt ist, wie Salmonella enterica, Yersinia pseudotuberculosis, Lactobacillus, Bacillus subtilis und Mycobacterium.

Ein weiteres Problem besteht darin, dass es bisher kein generelles System gibt, um produktbildende Mikroorganismen unmittelbar nach dem Rekombineering in großen Zellpopulationen zu identifizieren und aus solchen Zellpopulationen zu isolieren. Die bisher beim Rekombineering angewandte Methodik der Selektion auf Petrischalen ist, wie erwähnt, auf sehr spezielle Anwendungen limitiert und darüber hinaus in der Zahl der Rekombinanten, die auf Petrischalen erhalten werden, limitiert, was das Verfahren für das screening großer Rekombinantenbibliotheken ungeeignet macht.

Rekombineering basiert auf der homologen Rekombination, die von Proteinen, die aus Phagen oder Prophagen stammen, vermittelt wird. Zwei homologe Systeme sind für Escherichia coli bekannt. Das RecE/RecT des Rac-Prophagen und das red-Operon, bestehend aus Red-Gamma, Red-Beta und Red-Alpha vom Bakteriophagen Lambda. Beide Systeme erlauben den Austausch frei wählbarer DNA-Abschnitte zwischen zwei unterschiedlichen DNA-Molekülen. Der Austausch von DNA erfolgt über zwei dem Zielfragment flankierende homologe (ähnliche oder identische) Bereiche mit einer Länge von 30-100 Basenpaaren. Zum Einführen chromosomaler Mutationen wird das DNA-Molekül, das die Mutation trägt, als Einzelstrang kommerziell synthetisiert (Figur 1, B1), und in E. coli, der das Protein Red-Beta exprimiert, eingebracht. Aufgrund der Homologie zwischen dem eingebrachten DNA-Molekül und dem Chromosom wird durch das Red-Beta Protein die Rekombination und der Austausch der Sequenzen vermittelt. Auf diese Weise sind Mutationen im galK Gen des Chromosoms von E. coli korrigiert worden. Da nur das intakte galK Gen die Galaktoseverwertung erlaubt, werden rekombinante Klone als Kolonien auf Petrischalen durch Wachstum selektioniert (Figur 1, B2) (Rekombineering: in vivo genetic engineering in E. coli, S. enterica, and beyond. Sawitzke JA, Thomason LC, Costantino N, Bubunenko M, Datta S, Court DL. Methods Enzymol. 2007;421:171-99). Ebenso können Resistenzgene eingeführt werden, oder entsprechende Mutationen korrigiert werden, so dass wiederum nach erfolgreichem Rekombineering auf Wachstum selektioniert werden kann. Dies ist auch mit Genen durchführbar, die für Resistenz gegenüber Chloramphenicol, Hygromycin, Streptomycin, Ampicillin oder Spectinomycin kodieren (Rekombineering: in vivo genetic engineering in E. coli, S. enterica, and beyond. Sawitzke JA, Thomason LC, Costantino N, Bubunenko M, Datta S, Court DL. Methods Enzymol. 2007;421:171-99). Auch andere Gene mit leicht selektierbarem Phänotyp lassen sich so durch Rekombineering in das Chromosom von E. coli einbringen oder im Chromosom mutieren. Falls keine Möglichkeit der Selektion besteht, kann dies durch verschiedene Techniken umgangen werden, wie Koselektion oder Koloniehybridisierung (Rekombineering: in vivo genetic engineering in E. coli, S. enterica, and beyond. Sawitzke JA, Thomason LC, Costantino N, Bubunenko M, Datta S, Court DL. Methods Enzymol. 2007;421:171-99), oder auch PCR-Analyse von Klonen, was allerdings zusätzliche Schritte erfordert und den Vorteil der schnellen gezielten Mutagenese des Chromosoms durch Rekombineering wieder zunichte macht. Außerdem setzt es eine außerordentlich hohe Rekombinationsfrequenz voraus, da andernfalls hunderte von Klonen einzeln getestet werden müssten. Deswegen ist Rekombineering des Chromosoms ohne klonale Kultivierungen zur Isolation eines verbesserten mikrobiellen Metabolitproduzenten noch nicht generell verwendbar. Ein Sonderfall ist das mikrobielle Produkt Lycopen, das zu auffallend rot gefärbten Kolonien führt. So wurde durch iteratives, mehrfach aufeinanderfolgendes Rekombineering mit E. coli und visuelle qualitative Bewertung der Farbintensität von Kolonien auf Petrischalen 20 chromosomale Genorte mutiert, um gesteigerte Lycopenbildung zu erreichen (Programming cells by multiplex genome engineering and accelerated evolution. Wang HH, Isaacs FJ, Carr PA, Sun ZZ, Xu G, Forest CR, Church GM. Nature. 2009;460(7257):894-8). Die Einschränkung der Anwendung des Rekombineering zur Entwicklung mikrobieller Produzenten beruht darauf, dass die allermeisten mikrobiell hergestellten niedermolekularen Moleküle keinen Phänotyp besitzen, auf den selektioniert werden kann.

Zum Stand der Technik des Produktnachweises gehören auch Metabolitsensoren - auch bekannt unter der Bezeichnung Nanosensoren - mit denen in einzelnen Bakterien erhöhte Produktbildung nachgewiesen werden kann. Solche Metabolitsensoren nutzen Transkriptionsfaktoren oder RNA Aptamere zur Detektion niedermolekularer Metabolite in Bakterien oder Hefen. Bekannte Metabolitsensoren, die auf Transkriptionsfaktoren beruhen, sind beispielsweise pSenLys, pSenArg, pSenSer, pSenOAS oder pJC1-Irp-brnF-eyfp (WO2011138006; DPA 102012 016 716.4). Der Metabolitsensor umfasst dabei eine für ein Autofluoreszenzprotein kodierende Gensequenz, wobei die Expression des Autofluoreszenzproteins von der intrazellulären Konzentration eines bestimmten Metaboliten abhängig ist. Dabei erfolgt die Kontrolle der Expression der für das Autofluoreszenzprotein kodierenden Gensequenz in Abhängigkeit von der intrazellulären Konzentration des bestimmten Metaboliten auf der Ebene der Transkription. In Abhängigkeit von der intrazellulären Konzentration des jeweiligen Metaboliten wird mithin mehr oder weniger mRNA gebildet, die in den Ribosomen unter Bildung des Autofluoreszenzproteins translatiert werden kann. Der Mikroorganismus, der den Metabolitsensor enthält, kann jeder beliebige Mikroorganismus sein. Beispielhaft können Bakterien, Hefen oder Enterobakterien, wie Escherichia coli, Corynebacterium glutamicum oder Saccharomyces cerevisiae genannt werden.

Die Nutzung von Metabolitsensoren, um Zellen mit gesteigerter Produktbildung einzusetzen, beruht darauf, dass bei gesteigerter Bildung eines Metaboliten nicht nur vermehrt Metabolit produziert und extrazellulär angehäuft wird, sondern auch eine erhöhte intrazelluläre Konzentration des Metaboliten gegenüber dem Wildtyp vorliegt (A high-throughput approach to identify genomic variants of bacterial metabolite producers at the single-cell level. Binder S, Schendzielorz G, Stäbler N, Krumbach K, Hoffmann K, Bott M, Eggeling L.Genome Biol. 2012 May 28;13(5):R40; Engineering microbial biofuel tolerance and export using efflux pumps. Dunlop MJ, Dossani ZY, Szmidt HL, Chu HC, Lee TS, Keasling JD, Hadi MZ, Mukhopadhyay A. Mol Syst Biol. 2011 May 10;7:487).

Metabolitsensoren sind beschrieben, um in Mutantenbibliotheken von Mikroorganismen Mutanten mit erhöhter Produktbildung zu erkennen, und diese Mutanten mittels Durchflusszytometrie und automatischer Sortiereinrichtung auszusortieren WO2011138006; DPA 102012 016 716.4). Die Mutantenbibliothek war in diesem Falle mit chemischer ungerichteter Mutagenese des Chromosoms hergestellt worden, beziehungsweise durch Einführung von Mutationen in ein plasmidkodiertes Gen durch fehlerhafte Polymerasekettenreaktion. Die vorliegende Erfindung bezieht sich nicht auf chemische ungerichtete Mutagenese oder Mutagenese durch fehlerhafte Polymerasekettenreaktion.

Die Veröffentlichung von Elizabeth E. Regulski et. al. "A widespread riboswitch candidate that controls bacterial genes involved in molybdenum cofactor and tungsten cofactor metabolism" in Molecular Microbiology, Bd. 68, Nr. 4, 1. Mai 2008 (2008-05-01), Seiten 918 - 932, offenbart eine gegenüber dem Wildtyp genetisch veränderte Zelle, die eine für eine nicht im Wildtyp vorhandene Rekombinase kodierende Gensequenz enthält.

Die Veröffentlichung von Stefanie Hampel et. al. "Drosophila Brainbow: a recombinasebased fluorescence labeling technique to subdivide neural expression patterns" in Nature Methods, Bd. 8, Nr. 3, 1. März 2011 (2011-03-01), Seiten 253 - 259) offenbart eine gegenüber dem Wildtyp genetisch veränderte Zelle, die für eine nicht im Wildtyp vorhandene Rekombinase kodierende Gensequenz und zusätzlich eine für einen Metabolitsensor kodierende Gensequenz enthält.

Die Schrift US2012/165387 A1 offenbart ein gegenüber seinem Wildtyp genetisch verändertes Bakterium, das eine für eine nicht im Wildtyp vorhandene Rekombinase kodierende Gensequenz und zusätzlich eine für einen Metabolitsensor kodierende Gensequenz umfasst.

Der Nachteil der bisherigen Techniken zur Stammentwicklung ist, dass bisher keine Technik zur Verfügung steht, um gezielt Mutationen in ein zelluläres Gen oder das Chromosom einzuführen, und gleichzeitig ohne klonale Kultivierungen (Petrischalen) nach Einführung der Mutation einen verbesserten Metabolitproduzenten direkt als Einzelzelle in Zellsuspensionen zu identifizieren und aus der Zellsuspensionen zu isolieren.

Es ist daher Aufgabe der Erfindung, ein derartiges Verfahren zur beschleunigten Entwicklung mikrobieller Produzenten kleiner Moleküle zur Verfügung zu stellen und die Nachteile des Standes der Technik zu überwinden.

Die Aufgabe wird erfindungsgemäß gelöst durch eine Zelle nach Anspruch 1, Verfahren nach den nebengeordneten Ansprüchen 4, 5.

Vorteilhafte Weiterbildungen der Erfindung sind in den Unteransprüchen angegeben.

Mit der Zelle und dem Verfahren ist es nunmehr möglich, auf besonders schnellem Weg für die gesteigerte Produktion von Metaboliten Zellen zu schaffen, mit denen eine gegenüber der Ausgangszelle gesteigerte Produktion von Metaboliten möglich ist.

In Folgenden soll die Erfindung in ihrer allgemeinen Form beschrieben werden.

Erfindungsgemäß wird eine gegenüber ihrem Wildtyp genetisch veränderte Zelle gemäß Anspruch 1, die eine für eine Rekombinase kodierende Gensequenz und zusätzlich einen Metabolitsensor kodierende Gensequenz enthält, zur Verfügung gestellt.

Bei der Zelle handelt es sich um einen Mikroorganismus, bevorzugt ein Bakterium, insbesondere der Gattung Corynebakterium, Enterobakterium oder der Gattung Escherichia, besonders bevorzugt Corynebacterium glutamicum oder Escherichia coli.

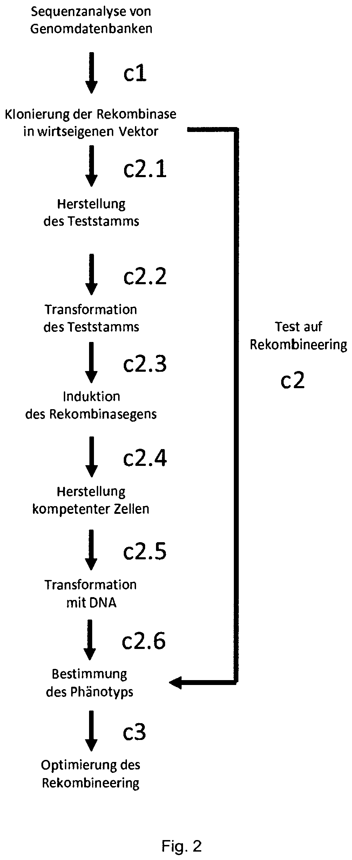

Bei der für eine Rekombinase kodierende Gensequenz kann es sich um eine Sequenz handeln, die eine verbesserte Funktionalität gegenüber einer bekannten Rekombinase in einem gewünschten Mikroorganismus hat. Es handelt sich um eine für eine Rekombinase kodierende Gensequenz, die für ein Protein kodiert, das extrazellulär zugesetzte DNA mit zelleigner DNA rekombiniert. Der Test auf Funktionalität kann durchgeführt werden, wie es in Figur 2 schematisch dargestellt ist. Als besonders geeignet hat sich eine Gensequenz nach SEQ. ID. Nr. 1 oder SEQ. ID Nr. 7 und 9 herausgestellt.

Die für die Rekombinase kodierende Gensequenz kann mittels eines Vektors, beispielsweise eines Plasmids, in die Zelle transformiert und exprimiert werden, wobei die Rekombinase gebildet wird.

Die in dem Verfahren eingesetzte Rekombinase ist dadurch charakterisiert, dass sie extrazellulär zugesetzte DNA mit der zelleigenen DNA rekombiniert. Die Rekombinase kann beispielsweise aus einem größeren Genpool, wie einem Metagenom, stammen, wo mögliche Rekombinasen durch Sequenzvergleiche mit bekannten Rekombinasen identifiziert werden. Durch solche Sequenzvergleiche lassen sich weiterhin mögliche Rekombinasen auch in bereits bestehenden Datenbanken identifizieren. Darüberhinaus können Proteine, denen Rekombinaseaktivität zugesprochen wird, oder von denen eine solche Aktivität vermutet wird, auch durch funktionelle Charakterisierung als Rekombinase erkannt werden. Bevorzugt können Rekombinasen aus Phagen oder Prophagen isoliert werden. So können Rekombinasen aus Prophagen der biotechnologisch relevanten Bakterien Leuconostoc, Clostridien, Thiobacillus, Alcanivorax, Azoarcus, Bacillus, Pseudomonas, Pantoea, Acinetobacter, Shewaniella, oder Corynebacterium und jeweils der verwandten Species isoliert und eingesetzt werden. Bevorzugt sind Rekombinasen, die zu der Rekombinase RecT des Prophagen Rac, oder der Rekombinase Bet des Phagen Lambda homolog sind. Besonders bevorzugt ist die Rekombinase RecT aus dem E. coli Prophagen Rac, die Rekombinase Bet aus dem E. coli Phagen Lambda, und die Rekombinase rCau (cauri_1962) aus Corynebacterium aurimucosum.

Bei der eingesetzten Gensequenz, die für den Metabolitsensor kodiert, handelt es sich um die Sequenz von Vektoren, beispielsweise Plasmiden, die für Proteine kodieren, welche Metabolite, wie Aminosäuren, organische Säuren, Fettsäuren, Vitamine oder pflanzliche Wirkstoffe erkennen und durch Fluoreszenz sichtbar machen. Je stärker die Fluoreszenz ist, desto höher ist die intrazelluläre Metabolikonzentration. Dadurch kann eine Zelle mit gegenüber der genetisch unveränderten Form erhöhter Fluoreszenz und damit erhöhter Produktbildung indentifiziert werden.

Die so modifizierte Zelle ist geeignet, von außen zugeführte DNA-Moleküle, die die Mutationen M1 bis Mm oder die mutierten Gene G1-Gn tragen, in ihre zelleigene DNA einzubauen und eine durch den Einbau der DNA bedingte erhöhte Produktion eines bestimmten Metaboliten durch Fluoreszenz kenntlich zu machen. Der Metabolisensor ist dabei so ausgewählt, dass er auf die Detektion des Metaboliten, der vermehrt gebildet werden soll, anspricht.

Weiterhin umfasst die Erfindung ein Verfahren gemäß Anspruch 4 zum Identifizieren einer Zelle mit gegenüber ihrem Wildtyp erhöhter intrazellulärer Konzentration eines bestimmten Metaboliten in einer Zellsuspension, beinhaltend die Verfahrensschritte:
i) Bereitstellen einer Zellsuspension beinhaltend Zellen der zuvor beschriebenen Art
ii) genetisches Verändern der Zellen durch Rekombineering unter Zugabe von DNA, die mindestens ein verändertes Gen G1 bis Gn oder mindestens eine Mutation M1 bis Mm enthält unter Erhalt einer Zellsuspension, in der sich die Zellen hinsichtlich der intrazellulären Konzentration eines bestimmten Metaboliten unterscheiden,
iii) identifizieren einzelner Zellen, in der Zellsuspension mit erhöhter intrazellulärer Konzentration eines bestimmten Metaboliten durch Fluoreszenzdetektion mittels eines Metabolitsensors.

Als Zellen werden Mikroorganismen der Gattung Corynebakterium, Enterobakterien oder der Gattung Escherichia, besonders bevorzugt Corynebacterium glutamicum, oder Escherichia coli eingesetzt.

Bei dem Rekombeneering handelt es sich um die nach dem Stand der Technik bekannten Methoden und die im speziellen Beschreibungsteil beispielhaft, aber nicht beschränkend, offenbarten Verfahren. Vorzugsweise wird das Rekombinasegen auf einem Plasmid in die Zelle eingeführt. Besonders bevorzugt wird ein Gen für eine Rekombinase nach SEQ. ID1 Nr.1 in die Zelle eingeführt.

Dazu werden die Zellen vorzugsweise mit Vektoren, besonders bevorzugt Plasmide, nach den Sequenzen SEQ.ID. Nr. 3 bis Nr. 9 transformiert.

Bei den Metaboliten die gegenüber dem Wildtyp in erhöhter intrazellulärer Konzentration auftreten, kann es sich beispielsweise um Aminosäuren, organische Säuren, Fettsäuren, Vitamine oder pflanzliche Wirkstoffe handeln. Es handelt sich dabei um die gewünschten Produkte, deren Produktion verbessert werden sollen.

Bei der Zellsuspension kann es sich um Zellen, die beispielsweise in einer salzhaltigen wässrigen Lösung vorliegen und die gegebenenfalls Nährstoffe enthält, handeln.

Bei der genetischen Veränderung der Zelle durch Rekombineering kann DNA als Einzelstrang oder Doppelstrang DNA benutzt werden, sowie synthetische DNA oder auch aus Zellen isolierte DNA. Die DNA kann 50 bp bis 3 Mb umfassen, bevorzugt DNA mit der Länge 50-150 bp. Die DNA kann für Proteine oder Teilen von Proteinen des gentechnisch zu verändernden Produzenten kodieren. Auch kann DNA eingesetzt werden, die homolog zu Promoterbereiche oder Bereichen unbekannter Funktion des gentechnisch zu verändernden Produzenten ist. Darüber hinaus kann die DNA auch für Gene oder regulatorische Elemente aus anderen Organismen kodieren, als die des gentechnisch zu verändernden Produzenten. Neben definierten DNA Molekülen können auch Mischungen von DNA Molekülen eingesetzt werden, was zum Beispiel zur Erzeugung großer genetischer Diversität vorteilhaft ist.

Die Insertion kann dabei in das Chrosom oder in ein Plasmid erfolgen.

Die Methoden Fluoreszenzdetektion mittels eines Metabolitsensors sind dem Fachmann bekannt.

Die Erfindung betrifft in einer Ausgestaltung auch ein Verfahren zur Herstellung einer gegenüber ihrem Wildtyp genetisch veränderten Produktionszelle mit optimierter Produktion eines bestimmten Metaboliten gemäß Anspruch 5, beinhaltend folgende Schritte:
i) Bereitstellen einer Zellsuspension beinhaltend Zellen der zuvor beschriebenen Art;
ii) genetisches Verändern der Zellen durch Rekombineering unter Zugabe von DNA, die mindestens ein verändertes Gen G1 bis Gn oder mindestens eine Mutation M1 bis Mm enthält. Erhalt einer Zellsuspension, in der sich die Zellen hinsichtlich der intrazellulären Konzentration eines bestimmten Metaboliten unterscheiden;
iii) Identifizieren einzelner Zellen in der Zellsuspension mit erhöhter intrazellulärer Konzentration eines bestimmten Metaboliten durch Fluoreszenzdetektion mittels eines Metabolitsensors;
iv) Abtrennen der identifizierten Zellen aus der Zellsuspension;
v) Identifizieren derjenigen genetisch veränderten Gene G1 bis Gn oder derjenigen Mutationen M1 bis Mm in den identifizierten und abgetrennten Zellen, welche für die erhöhte intrazelluläre Konzentration des bestimmten Metaboliten verantwortlich sind;
vi) Herstellung einer gegenüber ihrem Wildtyp genetisch veränderten Produktionszelle mit optimierter Produktion des bestimmten Metaboliten, deren Genom mindestens eines der Gene G1 bis Gn und/oder mindestens eine Mutation M1 bis Mm umfasst.

Für die Zellen, das Rekombineering, die Metaboliten, die Zellsuspension und die Methoden der Fluoreszenzdetektion, Vektoren, die in die Zelle einzuführende DNA der Schritte i), ii) und iii) gelten dieselben Zusammenhänge, wie für das Verfahren zum Identifizieren einer Zelle mit gegenüber ihrem Wildtyp erhöhter intrazellulärer Konzentration eines bestimmten Metaboliten in einer Zellsuspension.

Das Abtrennen der identifizierten Zellen kann mit bekannten Methoden erfolgen.

Für die Herstellung einer gegenüber dem Wildtyp veränderten Produktionszelle werden die Zellen, die zur Identifizierung der gesteigerten Produktion verwendet wurden und die eine gesteigerte Produktion von Metaboliten durch erhöhte Fluoreszenz anzeigen, isoliert.

In diesen Zellen wird die Mutation M1-Mm und/oder im Gen G1-Gn, oder die Mutationen M1-Mm in den Genen G1-Gn identifiziert. Dies kann durch PCR Amplifikation der Zielgene in den Genen G1 bis Gn und/oder den Mutationsorten M1 bis Mm, mit anschließender Sequenzierung erfolgen. Ebenso kann die Sequenzierung des Genoms erfolgen.

Die identifizierten produktsteigernden Mutationen M1-Mm und/oder Gene G1-Gn werden anschließend in die Produktionszelle übertragen. Dies kann mit dem Fachmann nach dem Stand der Technik bekannten Methoden erfolgen.

Die Bezeichnung G1 bis Gn richtet sich auf mindestens eines der Gene G1, G2, G3 bis Gn, das der Zelle im Rahmen des Rekombeneerings hinzugefügt wurde und das nun für eine besonders gute Steigerung der Produktion des Metaboliten verantwortlich gemacht wird.

Die Bezeichnung M1 bis Mm richtet sich auf Mutationen M1, M2 M3.... Mm, die in den Genen G1 bis Gn enthalten sind, welche der Zelle bei Verfahrensschritt ii) zugegeben wurde und die nun für eine besonders gute Steigerung der Produktion des Metaboliten verantwortlich gemacht wird.

Diese Gene oder diese Mutationen werden aus der Zelle isoliert und in das Genom der Produktionszelle nach bekannten Verfahren eingebracht. Dabei kann das Gen oder die Mutation, bzw. können die Gene oder die Mutationen in die chromosomale DNA oder ein Plasmid der Produktionszelle eingebracht werden.

Bei diesen Genen oder Mutationen handelt es sich um DNA Abschnitte, die vorzugsweise für Proteine der Schritte eines Biosyntheseweges des gewünschten Metaboliten oder gegebenenfalls eines damit im Zusammenhang stehenden Stoffwechselprozesses kodieren. Auch kann es sich um DNA handeln, mit der die Promoteraktivität von Genen oder die Stabilität der mRNA von Genen für die Produktbildung förderlicherweise beeinflußt wird.

Insbesondere wurden die Gene nach den Sequenzen SEQ. ID. Nr. 33 bis SEQ. ID. 44 aufgefunden, die geeignet sind, die Produktion von L-Lysin zu steigern.

Die Erfindung betrifft weiterhin ein Verfahren zur Herstellung von Metaboliten, beinhaltend die Verfahrensschritte:
a) Herstellung einer gegenüber ihrem Wildtyp genetisch veränderten Produktionszelle mit optimierter Produktion eines bestimmten Metaboliten durch ein Verfahren der oben angegebenen Art
b) Kultivieren der Zelle in einem Kulturmedium beinhaltend Nährstoffe unter Bedingungen, unter denen die Produktionszelle aus den Nährstoffen den bestimmten Metaboliten produziert.

Der auf diese Weise hergestellte Metabolit wird in das Kulturmedium sekretiert und kann aus dem Kulturmedium isoliert werden.

Das zu verwendende Kulturmedium beziehungsweise Fermentationsmedium muss in geeigneter Weise den Ansprüchen der jeweiligen Stämme genügen. Geeignete Kulturmedien sind dem Fachmann bekannt. Beschreibungen von Kulturmedien verschiedener Mikroorganismen sind im Handbuch &Idquor; Manual of Methods for General Bacteriology" der American Society for Bacteriology (Washington D.C., USA, 1981) enthalten. Die Begriffe Kulturmedium und Fermentationsmedium, beziehungsweise Medium, sind gegenseitig austauschbar.

Als Kohlenstoffquelle können Zucker und Kohlehydrate, wie z. B. Glucose, Saccharose, Lactose, Fructose, Maltose, Melasse, Saccharose-haltige Lösungen aus der Zuckerrüben-oder Zuckerrohrverarbeitung, Stärke, Stärkehydrolysat und Cellulose, Öle und Fette, wie zum Beispiel Sojaöl, Sonnenblumenöl, Erdnußöl und Kokosfett, Fettsäuren, wie zum Beispiel Palmitinsäure, Stearinsäure und Linolsäure, Alkohole wie zum Beispiel Glycerin, Methanol und Ethanol und organische Säuren, wie zum Beispiel Essigsäure oder Milchsäure verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Das Kulturmedium muß weiterhin Salze, beispielsweise in Form von Chloriden oder Sulfaten von Metallen, wie beispielsweise Natrium, Kalium, Magnesium, Calcium und Eisen enthalten, wie zum Beispiel Magnesiumsulfat oder Eisensulfat, die für das Wachstum notwendig sind. Schließlich können essentielle Wuchsstoffe wie Aminosäuren beispielsweise Homoserin und Vitamine, beispielsweise Thiamin, Biotin oder Pantothensäure, zusätzlich zu den oben genannten Stoffen eingesetzt werden.

Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak, beziehungsweise Ammoniakwasser oder saure Verbindungen wie Phosphorsäure oder Schwefelsäure in geeigneter Weise eingesetzt. Der pH-Wert wird im Allgemeinen auf einen Wert von 6,0 bis 8,5, vorzugsweise 6,5 bis 8, eingestellt. Zur Kontrolle der Schaumentwicklung können Antischaummittel, wie zum Beispiel Fettsäurepolyglykolester eingesetzt werden. Zur Aufrechterhaltung der Stabilität von Plasmiden können dem Medium geeignete, selektiv wirkende Stoffe, wie zum Beispiel Antibiotika, hinzugefügt werden. Die Fermentation wird bevorzugt unter aeroben Bedingungen durchgeführt. Um diese aufrechtzuerhalten, werden Sauerstoff oder Sauerstoff-haltige Gasmischungen, wie zum Beispiel Luft, in die Kultur eingetragen. Die Verwendung von Flüssigkeiten, die mit Wasserstoffperoxid angereichert sind, ist ebenfalls möglich. Gegebenenfalls wird die Fermentation bei Überdruck, beispielsweise bei einem Überdruck von 0,03 bis 0,2 MPa, gefahren. Die Temperatur der Kultur liegt normalerweise bei 20°C bis 45°C und vorzugsweise bei 25°C bis 40°C, besonders bevorzugt bei 30° bis 37°C. Bei batch-Verfahren wird die Kultivierung bevorzugt solange fortgesetzt, bis sich eine für die Maßnahme der Gewinnung des gewünschten Metaboliten wie z.B. einer Aminosäure, organischen Säure, eines Vitamins, oder eines pflanzlichen Wirkstoffs, in ausreichender Menge gebildet hat. Dieses Ziel wird normalerweise innerhalb von 10 bis 160 Stunden erreicht. Bei kontinuierlichen Verfahren sind längere Kultivierungszeiten möglich. Durch die Tätigkeit der Mikroorganismen kommt es zu einer Anreicherung (Akkumulation) des Metabolits im Fermentationsmedium und/oder in den Zellen der Mikroorganismen.

Beispiele für geeignete Fermentationsmedien finden sich unter anderem in den Patentschriften 5,770,409, US 5,990,350, US 5,275,940, WO 2007/012078, US 5,827,698, WO 2009/043803, US 5,756,345 oder US 7,138,266.

Mit dem erfindungsgemäßen Verfahren zur Herstellung von Metaboliten können beispielsweise Aminosäuren, organische Säuren, Vitamine, Kohlehydrate oder pflanzliche Wirkstoffe besonders effektiv hergestellt werden.

Vorzugsweise werden mit diesem Verfahren L-Aminosäuren, Nukleotide und pflanzliche Wirkstoffe produziert, besonders bevorzugt L-Lysin. Beschrieben ist auch ein Rekombinasegen nach SEQ. ID. Nr. 1 und deren Allele, mit einer mindestens 70% igen, vorzugsweise 80% igen, besonders bevorzugt 85% igen, und/oder 90% igen Homologie, insbesondere bevorzugt, einer Homologie, die 91%, 92, %, 93%, 94 %, 95%, 96%, 97%, 98%, oder 99% beträgt. Beschrieben ist auch eine Rekombinase nach SEQ. ID. Nr.2 und deren homologe Proteine, mit einer Homologie von 95%, 96%, 97%, vorzugsweise von 98% oder 99%. Weiterhin sind Nukleinsäuren nach den Sequenzen der SEQ. ID. Nr. 33 - SEQ. ID. Nr. 44 beschrieben, die für Gene kodieren, mit denen besonders produktive Produktionsstämme erhalten werden können und die ursprünglich aus der Zelle zur Identifikation von Mutationen stammen.

Im Folgenden wird die Erfindung im speziellen Beschreibungsteil genauer, jedoch nicht beschränkend, ausgeführt.

Einen Beitrag zur Lösung der gestellten Aufgabe leistet ein Verfahren in dem eine Rekombinase identifiziert und genutzt wird. Um Rekombinasen für biotechnologisch relevante Bakterien wie Leuconostoc, Clostridien, Thiobacillus, Alcanivorax, Azoarcus, Bacillus, Pseudomonas, Pantoea, Acinetobacter, Shewaniella und Corynebacterium species, insbesondere Corynebacterium glutamicum zu identifizieren, werden Genomdatenbanken nach bekannten Verfahren auf Proteine, die homolog zu bekannten Rekombinasen sind, und von denen eine bessere Funktion im gewünschten Organismus erwartet oder erhofft wird, als die der bekannten Rekombinasen, analysiert. Genomdatenbanken sind frei zugänglich. So zum Beispiel die Datenbank des European Molecular Biologies Laboratories (EMBL, Heidelberg, Germany and Cambridge, UK), die Datenbank des National Center for Biotechnology Information (NCBI, Bethesda, Md., USA), die Datenbank des Swiss Institute of Bioinformatics (Swissprot, Geneva, Switzerland), oder die Protein Information Resource Database (PIR, Washington, D.C., USA) und die DNA Data Bank of Japan (DDBJ, 1111 Yata, Mishima, 411-8540, Japan).

Die genannten Datenbanken werden genutzt, um nach Proteinen zu suchen, die homolog zu bereits bekannten Rekombinasen sind (Figur 2, c1), wie zum Beispiel RecT aus dem Prophagen Rac (Genetic and molecular analyses of the C-terminal region of the recE gene from the Rac prophage of Escherichia coli K-12 reveal the recT gene. Clark, A.J., Sharma, V., Brenowitz, S., Chu, C.C., Sandler, S., Satin, L., Templin, A., Berger, I., Cohen, A. J. Bacteriol. (1993)), Beta aus dem Pagen Lambda (Hendrix, R.W. (1999). All the world's a phage. Proc Nat Acad Sc USA 96: 2192-2197), gp61 aus Mycobacteriophage Che9c oder gp43 aus Mycobacteriophage Halo (Rekombineering mycobacteria and their phages. van Kessel JC, Marinelli LJ, Hatfull GF. Nat Rev Microbiol. 2008 Nov;6(11):851-857). Die Suche nach den homologen Proteinen erfolgt mit bekannten Algorithmen und Sequenzanalyseprogrammen nach bekannten Verfahren, die öffentlich zugänglich sind, wie zum Beispiel beschrieben bei Staden (Nucleic Acids Research 14, 217-232 (1986)), oder Marck (Nucleic Acids Research 16, 1829-1836 (1988)) oder unter Nutzung des GCG Programms von Butler (Methods of Biochemical Analysis 39, 74-97 (1998)).

Die beschriebenen Sequenzen umfassen auch solche Sequenzen, die eine Homologie (auf Aminosäureebene) bzw. Identität (auf Nukleinsäureebene, exklusive der natürlichen Degeneration) größer als 70%, vorzugsweise 80%, mehr bevorzugt 85% (in Bezug auf die Nukleinsäuresequenz) bzw. 90% (auch in Bezug auf die Polypeptide), bevorzugt größer als 91%, 92%, 93% oder 94%, mehr bevorzugt größer als 95% oder 96% und besonders bevorzugt größer als 97%, 98% oder 99% (in Bezug auf beide Arten von Sequenzen) zu einer dieser Sequenzen aufweisen, sofern die Wirkungsweise bzw. Funktion und Zweck einer solchen Sequenz erhalten bleibt. Der Ausdruck "Homologie" (oder Identität) wie hierin verwendet, kann durch die Gleichung H (%) = [1-V/X]x 100 definiert werden, worin H Homologie bedeutet, X die Gesamtzahl an Nukleobasen/Aminosäuren der Vergleichssequenz ist und V die Anzahl an unterschiedlichen Nukleobasen/Aminosäuren der zu betrachtenden Sequenz, bezogen auf die Vergleichssequenz ist. Auf jeden Fall sind mit dem Begriff Nukleinsäuresequenzen, welche für Polypeptide codieren, alle Sequenzen umfasst, die nach Maßgabe der Degeneration des genetischen Codes möglich erscheinen.

Die prozentuale Identität zu den Aminosäuresequenzen, die in dem Sequenzprotokoll angegeben sind, kann mit im Stand der Technik bekannten Verfahren vom Fachmann ohne weiteres ermittelt werden. Ein geeignetes Programm, das erfindungsgemäß eingesetzt werden kann, ist BLASTP (Altschul et al.. 1997. Gapped BLAST and PSI-BLAST: a new generation of protein database search programs. Nucleic Acids Res. 25(17): 3389-3402).

Die im Sequenzprotokoll angegebenen Sequenzen umfassen auch Nukleinsäuresequenzen, die mit den angegebenen hybridisieren. Anleitungen zur Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen Sonden, beispielsweise die zum Gen komplementäre Nukleotidsequenz, und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996). Für die Hybridisierungsreaktion kann beispielsweise ein Puffer entsprechend 5xSSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2xSSC und gegebenenfalls nachfolgend 0,5xSSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Vorzugsweise werden die Waschschritte bei Temperaturen von ca. 62°C - 68°C, bevorzugt von 64°C - 68°C oder ca. 66°C - 68°C, besonders bevorzugt von ca. 66°C - 68°C durchgeführt. Es ist gegebenenfalls möglich, die Salzkonzentration bis auf eine Konzentration entsprechend 0,2xSSC oder 0,1xSSC zu senken. Durch schrittweise Erhöhung der Hybridisierungstemperatur in Schritten von ca. 1 - 2°C von 50°C bis 68°C können Polynukleotidfragmente, die für Aminosäuresequenzen kodieren, isoliert werden, die beispielsweise mindestens 70% oder mindestens 80% oder mindestens 90% bis 95% oder mindestens 96% bis 98% oder mindestens 99% Identität zur Sequenz der eingesetzten Sonde besitzen. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558).

Die so ermittelte neue DNA Sequenz aus Corynebacterium aurimucosum, die das Rekombinasegen recT enthält (SEQ ID No. 1) und für die funktionelle Rekombinase rCau kodiert (SEQ ID No. 2), ist Bestandteil der vorliegenden Erfindung.

Identifizierte Rekombinasen werden in einen Vektor, beispielsweise ein Plasmid, kloniert, der die induzierbare Expression des Rekombinasegens im Wirt, in dem die Rekombination durchgeführt werden soll, erlaubt (Figur 2, c2.1). Expressionsvektoren sind Stand der Technik. So kann beispielsweise für Leuconostoc oder Lactobacillus der Vektor pCB42 benutzt werden (Construction of theta-type shuttle vector for Leuconostoc and other lactic acid bacteria using pCB42 isolated from kimchi. Eom HJ, Moon JS, Cho SK, Kim JH, Han NS. Plasmid. 2012 Jan;67(1):35-43), für Clostridien ptHydA (Girbal L, von Abendroth G, Winkler M, Benton PM, Meynial-Salles I, Croux C, Peters JW, Happe T, Soucaille P (2005) Homologous and heterologous over-expression in Clostridium acetobutylicum and characterization of purified clostridial and algal Fe-only hydrogenases with high specific activities. Appl. Environ Microbiol. 71:2777-2781) für Thiobacillus pTF-FC2 (Plasmid evolution and interaction between the plasmid addiction stability systems of two related broad-host-range IncQ-like plasmids. Deane SM, Rawlings DE. J Bacteriol. 2004 Apr;186(7):2123-33.), für Alcanivorax pRED (Appl Microbiol Biotechnol. 2006 Jul;71(4):455-62. Functional expression system for cytochrome P450 genes using the reductase domain of self-sufficient P450RhF from Rhodococcus sp. NCIMB 9784. Nodate M, Kubota M, Misawa N.), für Bacillus pMG (Construction of a modular plasmid family for chromosomal integration in Bacillus subtilis. Gimpel M, Brantl S. J Microbiol Methods. 2012;91 (2):312-7), für Pseudomonas pWW0 (Increasing Signal Specificity of the TOL Network of Pseudomonas putida mt-2 by Rewiring the Connectivity of the Master Regulator XylR. de Las Heras A, Fraile S, de Lorenzo V. PLoS Genet. 2012 Oct;8(10):e1002963), für Pantoea pAGA (Characterization of a small cryptic plasmid from endophytic Pantoea agglomerans and its use in the construction of an expression vector. de Lima Procöpio RE, Araüjo WL, Andreote FD, Azevedo JL. Genet Mol Biol. 2011 Jan; 34(1): 103-9), für Acinetobacter pRIO-5 (Complete sequence of broad-host-range plasmid pRIO-5 harboring the extended-spectrum-β-lactamase gene blaBES. Bonnin RA, Poirel L, Sampaio JL, Nordmann P. Antimicrob Agents Chemother. 2012 Feb; 56(2):1116-9), für Shewaniella pBBR1-MCS (Shewanella oneidensis: a new and efficient system for expression and maturation of heterologous [Fe-Fe] hydrogenase from Chlamydomonas reinhardtii.

Sybirna K, Antoine T, Lindberg P, Fourmond V, Rousset M, Mejean V, Bottin H. BMC Biotechnol. 2008 Sep 18;8:73), oder für Corynebacterium species, insbesondere C. glutamicum pZ1 (Menkel et al., Applied and Environmental Microbiology (1989) 64: 549-554) oder pCLTON (A tetracycline inducible expression vector for Corynebacterium glutamicum allowing tightly regulable gene expression. Lausberg F, Chattopadhyay AR, Heyer A, Eggeling L, Freudl R. Plasmid. 2012 68(2):142-7). Ein Übersichtsartikel zu Expressionsplasmiden in Corynebacterium glutamicum ist durch Tauch et al. (Journal of Biotechnology 104, 27-40 (2003)) geschrieben.

Gemäß einer bevorzugten Ausführungsform der erfindungsgemäßen Vektoren handelt es sich um die Vektoren pCLTON2-bet (SEQ ID No. 3), pCLTON2-recT (SEQ ID No. 4), pCLTON2-gp43 (SEQ ID No. 5), pCLTON2-gp61 (SEQ ID No. 6), pCLTON2-rCau (SEQ ID No. 7), pEKEx3-recT (SEQ ID No. 8), pEKEx3-bet (SEQ ID No. 9).

Die so hergestellten Vektoren werden auf Aktivität der Rekombinase im jeweiligen Wirt getestet (Figur 2, c2). Der Aktivitätstest umfasst die Herstellung eines Teststamms des Wirts bei dem durch Rekombineering ein einfach zu prüfender Phänotyp hergestellt werden soll (Figur 2, c2.1). Die weiteren Schritte beinhalten die Transformation des Teststamms (Figur 2, c2.2), Induktion der Expression des Rekombinasegens (Figur 2, c2.3), Herstellung kompetenter Zellen zur Aufnahme von linearer DNA (Figur 2, c2.4), Transformation der kompetenten Zellen mit linearer DNA (Figur 2, c2.5), und Test auf Herstellung des Phänotyps (Figur 2, c2.6). Lässt sich der erwartete Phänotyp herstellen, so ist Rekombineering erfolgt. Die einzelnen Schritte, c2.1 -c2.6, sind dem Fachmann bekannt. So wird in den Teststamm als einfach zu prüfender Phänotyp (Figur 2, c2.1) beispielsweise ein defektes Antibiotikaresistenzgen in das Chromosom eingebaut, dessen Funktion durch erfolgreiches Rekombineering wiederhergestellt wird. Als Antibiotikaresistenzgene stehen Gene, die Resistenz gegenüber Kanamycin, Chloramphenicol, Hygromycin, Streptomycin, Ampicillin oder Spectinomycin verleihen, zur Verfügung. Auch können Gene verwendet werden, die Wachstum auf einem bestimmten Substrat als Selektionsmarker erlauben, wie beispielsweise das für Galaktokinase kodierende galK Gen. Die Transformation des Teststamms mit dem Rekombinase exprimierenden Testplasmid (Figur 2, c2.2) erfolgt nach bekannten Verfahren, wie zum Beispiel Elektroporation, chemischer Transformation oder ballistischer Transformation. Zur Induktion des Rekombinasegens (Figur 2, c2.3) im Expressionsvektor wird der durch den Vektor vorgegebene Induktor dem Medium zugegeben. Diese Vorgehensweise ist dem Fachmann bekannt, und es handelt sich bei dem Induktor beispielsweise um Isopropyl-β-D-thiogalactopyranosid, Anhydrotetracyclin, Sakacin, oder Acetamid. Die weiteren Schritte, wie die Herstellung kompetenter Zellen (Figur 2, c2.4), die Transformation der Zellen (Figur 2, c2.5) sowie Test auf Herstellung des Phänotyps durch Ausplattieren auf Petrischalen (Figur 2, c2.6), sind mikrobiologische Standardverfahren und ebenfalls dem Fachmann bekannt. Wird der gewünschte Phänotyp nach dem beschriebenen Verfahren hergestellt, erfolgt anschließend vorzugsweise die Optimierung des Rekombineerings (Figur 2, c3). Dieses umfasst die Variation der Induktionszeit im Bereich von dreißig Minuten bis sechs Stunden, die Variation der zum Rekombineering eingesetzten DNA, und die Variation der Regenerations- und Seggregationszeit sowie gegebenenfalls weiterer Parameter, die dem Fachmann bekannt sind.

Bei der zum Rekombineering eingesetzten DNA handelt es sich um Einzelstrang DANN, die durch kommerzielle Anbieter synthetisiert wird, und bis zu 300 Basenpaare lang sein kann. Dabei liegt im Zentrum der DNA die gewünschte in das Chromosom einzuführende Mutation vor, und flankierend dazu enthält die DNA Sequenzen, die zur chromosomalen Sequenz des Wirts homolog sind (US 7,144,734). Die Optimierung beinhaltet den Test von DNA unterschiedlicher Länge. Als DNA wird DNA der Länge von 20-300 Basenpaaren, bevorzugt 100 Basenpaare, eingesetzt. Die Optimierung beinhaltet den Test von DNA in unterschiedlicher Menge, wobei 0,2 - 30 Mikrogramm zur Transformation, bevorzugt 10 Mikrogramm, eingesetzt werden. Die Optimierung beinhaltet ferner den Test von DNA die entweder zum Sinnstrang oder zum Gegenstrang homolog ist, wobei bevorzugt die zum Gegenstrang homologe DNA eingesetzt wird (US 7,674,621). Die einzelnen Optimierungsschritte sind dem Fachmann bekannt, und beispielsweise für E. coli (Rekombineering: in vivo genetic engineering in E. coli, S. enterica, and beyond. Sawitzke JA, Thomason LC, Costantino N, Bubunenko M, Datta S, Court DL. Methods Enzymol. 2007; 421:171-99), Bacillus subtilis (Bacillus subtilis genome editing using ssDNA with short homology regions. Wang Y, Weng J, Waseem R, Yin X, Zhang R, Shen Q. Nucleic Acids Res. 2012 Jul;40(12):e91), oder Lactococcus (High efficiency Rekombineering in lactic acid bacteria. van Pijkeren JP, Britton RA. Nucleic Acids Res. 2012, 40(10):e76) beschrieben.

Zur Durchführung des Rekombineerings zur Gewinnung eines mikrobiellen Produzenten (Figur 2, C1-C4), wird der zu mutierende Genort im Chromosom ausgewählt. Dies können bekannte Gene, Gene unbekannter Funktion oder intergenische Bereiche sein. Bei Produzenten sind es beispielsweise Gene oder Promoterbereiche von Genen, die am Anabolismus oder Katabolismus oder an Regulationsprozessen beteiligt sind, oder solche, die die Halbwertszeit von mRNA oder Proteinen beeinflussen.

Die zur Rekombination benutzte DNA wird synthetisiert oder durch PCR Amplifikation hergestellt. Sie ist 30-3000 Basenpaare lang, und hat die Organisationsstruktur A-B-C. Dabei ist B die im Zentrum liegende gewünschte Mutation. Dies kann bei einer Insertion eine Sequenz von 1-3000 Basenpaaren sein, bevorzugt eine von 1-1000, weiter bevorzugt eine von 1-100, besonders bevorzugt eine von 1. Die Sequenzen A und C sind homolog zu chromosomalen Sequenzen. Sie sind in der synthetisierten DNA jeweils 20-100 Basenpaare lang. Bei einer im Chromosom gewünschten Deletion ist B Null Basenpaare lang, und A und C sind homolog zu Sequenzen im Chromosom, die direkt benachbart zum zu deletierenden Bereich sind. Die Sequencen A und C sind in der synthetisierten DNA 20-100 Basenpaare lang. Die Deletion im Chromosom kann 1 Basenpaar oder bis zu 10 kb betragen. Zum Austausch von Basen im Chromosom stellt B den auszutauschenden Bereich dar, der 1-50 Basenpaare umfassen kann. Die Sequenzen A und C sind homolog zu chromosomalen Sequenzen, die benachbart zum auszutauschenden Bereich liegen. Sie sind in der synthetisierten DNA 20-100 Basenpaare lang. DNA Synthesen führt beispielsweise Genescript (GenScript USA Inc., 860 Centennial Ave., Piscataway, NJ 08854, USA), oder Eurofins (Eurofins MWG Operon, Anzingerstr. 7a, 85560 Ebersberg, Germany), oder DNA 2.0 (DNC2.0 Headquarters, 1140 O'Brien Drive, Suite A, Menlo Park, CA 94025, USA) durch.

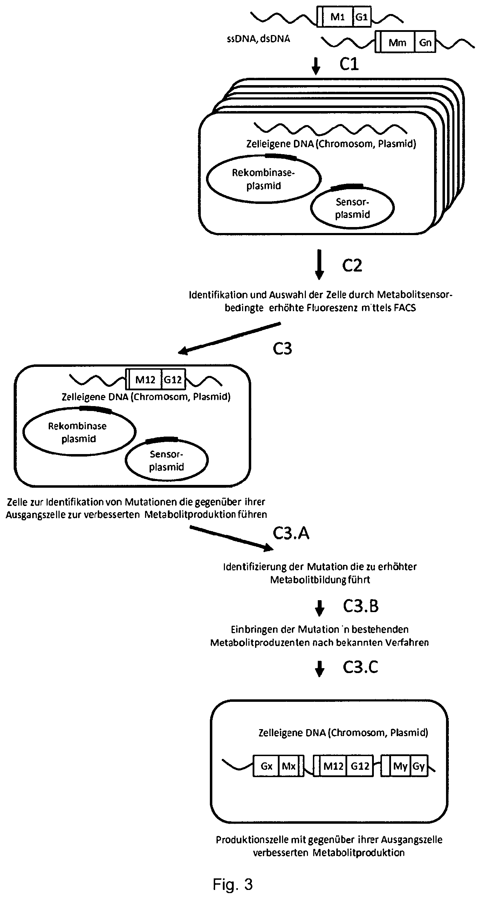

Die synthetisierte oder durch PCR Amplifikation hergestellte DNA wird durch Transformation, in den Mikroorganismus, der die Rekombinase exprimiert und der einen Metabolitsensor enthält, eingebracht (Figur 3, C1). Solche Metabolitsensoren enthaltende Mikroorganismen sind beschrieben (WO2011138006, DPA 102012 016 716.4, DPA 10 2012 017 026.2). Die zur Transformation und Rekombination benutzte DNA ist wie zuvor beschrieben eine definierte DNA Sequenz.

Zur Transformation und Rekombination können aber auch definierte Mischungen verschiedener DNA Sequenzen eingesetzt werden. Diese Mischungen werden bevorzugt beim Austausch von Basen im Chromosom im Bereich "B" eingesetzt, wo "B" bei der Organisationsstruktur A-B-C der DNA Sequenz den auszutauschenden Bereich darstellt. So können beispielsweise in einem Gen gleichzeitig in einer Population von Mikroorganismen verschiedene Aminosäuren an einer Position im Polypeptid ausgetauscht werden. Es können auch gleichzeitig verschiedene Aminosäuren an verschiedenen Positionen im Polypeptid ausgetauscht werden. Es können auch gleichzeitig in einem Promotorbereich verschiedene Nukleotide ausgetauscht werden. Die entsprechenden DNA Mischungen können selbst hergestellt werden durch Mischen einzelner definierter DNA Sequenzen, oder bereits beim Hersteller als Mischungen synthetisiert werden, wodurch bis zu mehrere Tausend verschiedener DNA Moleküle in einem Ansatz vorliegen, die auch in einem Ansatz dann zur Transformation und Rekombination (Figur 3, C1) benutzt werden. Solche DNA Mischungen mit verschiedensten Sequenzen können kommerziell bezogen werden. So werden beispielsweise "Combinatorial Libraries" oder "Controlled Randomized Libraries" oder "Truncated libraries" von Life Technologies GmbH, Frankfurter Straße 129B, 64293 Darmstadt angeboten, die direkt zum Rekombineering eingesetzt werden können.

Zur Transformation und Rekombination können ferner auch undefinierte DNA Sequenzen eingesetzt werden. Beispielsweise genomische DNA aus existierenden Produzenten. Auf diese Weise können DNA Abschnitte und/oder Mutationen und/oder Gene identifiziert werden die förderlich für die Produktbildung sind.

Im Anschluss an die Transformation der Rekombinase- und Nanosensor-enthaltenden Mikroorganismen erfolgt Regeneration in einem Komplexmedium, wie es dem Fachmann bekannt ist und beispielsweise für E. coli (Hanahan, D. Studies on transformation of Escherichia coli with plasmids. J Mol Biol. 1983; 166(4):557-80), oder Corynebacterium (Tauch A, Kirchner O, Löffler B, Götker S, Pühler A, Kalinowski J. Efficient electrotransformation of corynebacterium diphtheriae with a mini-replicon derived from the Corynebacterium glutamicum plasmid pGC1. Curr Microbiol. 2002; 45(5):362-7) beschrieben ist. Nach der Regeneration werden die Zellen gegebenfalls zur Seggregation in ein Minimalmedium überführt, worauf entsprechend des erfindungsgemäßen Verfahrens direkt die Produktanalyse in Einzelzellen durch Durchflusszytometrie und Selektion der Produzenten erfolgt (Figur 3, C2). Die selektierten Einzelzellen werden auf Medium in Petrischalen abgelegt, oder auch unmittelbar in Mikrotiterplatten mit Flüssigmedium zur weiteren Kultivierung abgelegt. Einzelheiten zur Analyse von Zellsuspensionen mittels Durchflusszytometrie können beispielsweise Sack U, Tarnok A, Rothe G (Hrsg): Zelluläre Diagnostik. Grundlagen, Methoden und klinische Anwendungen der Durchflusszytometrie, Basel, Karger, 2007, Seiten 27 - 70, entnommen werden. Geeignete Durchflusszytometer, die bis zu 100000 Zellen pro Sekunde analysieren und eine Sortiermöglichkeit besitzen, sind beispielsweise das Gerät Aria-III (BD Biosciences, 2350 Qume Drive, San Jose, California, USA, 95131, 877.232.8995) oder das Gerät MoFlo- XDP (Beckman Coulter GmbH, Europark Fichtenhain B 13, 47807 Krefeld, Germany).

Im Anschluss an die Produzentenisolation (Figur 3, C3) erfolgt die Verifizierung der Produktbildungseigenschaften in Schüttelkolben oder Mikrotiterplatten. Der besonders geeignete Produzent wird ausgewählt. Er produziert mehr von dem mikrobiell hergestellten Produkt als der Ausgangsstamm der in Schritt C1 (Figur 3) zum DNA Transfer eingesetzt wurde. Durch Sequenzierung des Genoms in Bereichen, die durch die im Schritt C1 zugesetzte DNA definiert sind, oder auch des gesamten Chromosoms, oder auch von plasmidkodierter DNA, kann die erfolgte produktsteigernde Mutation identifiziert werden (Figur 3, C3.A). Die entsprechenden Mutationen M1 bis Mm und/oder Gene G1 bis Gn werden gegebenfalls nach bekannten Verfahren in andere Produzentenstämme transferiert (Figur 3, C3.B), um so einen bereits bestehenden Metabolitproduzenten weiter zu verbessern (Figur 3, C3.C).

Die Erfindung wird nun anhand von Figuren und nicht limitierenden Beispielen näher erläutert.
Figur 1: (links) Die Figur zeigt zum Verständnis der Erfindung eine Darstellung des Ablaufs des Verfahrens nach dem Stand der Technik, nämlich das Prinzip der Herstellung einer chromosomalen Mutation durch die Schritte A1 - A8, ausgehend von der Konstruktion eines spezifischen Plasmides (A1) über zwei Selektionsschritte auf Petrischalen (A3 **-** A4), und klonale Kultivierung (A6) bis zum Test auf verbesserte Produktion (A7 - A8), sowie (rechts) das Prinzip der Herstellung einer chromosomalen Mutation durch Rekombineering, ausgehend von synthetischer DNA (B1) und Selektion resistenter Klone auf Petrischalen oder gefärbter Klone auf Petrischalen (B2).
Figur 2: Die Figur zeigt die erfindungsgemäße Entwicklung des Rekombineerings für einen für die biotechnologische Produktion niedermolekularer Moleküle relevanten Mikroorganismus. Durch Sequenzanalysen werden Rekombinasen identifiziert (c1), die in geeignete Expressionsvektoren eingebaut werden (c2.1). Nach Schritten, die im Wirt hohe Rekombinaseexpression bewirken und den Wirt zur DNA Aufnahme befähigen (c2.2 - c2.4), wird die DNA als Einzelstrang oder Doppelstrang DNA zugegeben (c2.5) und auf einen geeigneten Phänotyp des Teststamms selektioniert (c2.6). Abschließend erfolgt im gesamten Test auf Rekombineering (c2) noch dessen Optimierung (c3).
Figur 3: Das erfindungsgemäße Rekombineering gekoppelt mit zytometrischer Produktanalyse durch Metabolitsensoren zur Isolation mikrobieller Metabolitproduzenten und der weiteren Nutzung so identifizierter Mutationen zur Verbesserung bereits bestehender Metabolitproduzenten. DNA wird zu den Zellen zugegeben (C1), die Rekombinase exprimieren und das Sensorplasmid mit dem Metabolitsensor enthalten. In den Zellen erfolgt durch Rekombinase ein Einbau der zugegebenen DNA mit den mutierten Genen G1 - Gn mit den Mutationen M1-Mm. Zellen mit erhöhter Produktbildung und damit erhöhter Fluoreszenz werden mittels Hochdurchsatzdurchflusszytometrie und Selektion (FACS) isoliert (C2), und liefert so eine Zelle zur Identifikation der Mutationen, die zu verbesserter Metabolitbildung führt (C3). Gegebenenfalls stellt diese Zelle auch bereits einen verbesserten Metabolitproduzenten dar. Mit bekannten Verfahren wird das Genom oder Plasmid der aus dem Schritt C3 resultierenden Zelle zur Identifikation der Mutationen M1-Mm in den Genen G1-Gn sequenziert (C3.A), um diese nach bekannten Verfahren (C3.B) in bereits bestehende Metabolitproduzenten zu deren weiterer Verbesserung einzuführen (C3.C).

### Beispiel 1: Identifizierung einer Rekombinase

Mit der unter der Zugangsnummer CAD61789.1 in der Datenbank des National Center for Biotechnology Information (NCBI, Bethesda, Md., USA) hinterlegten Sequenz von RecT aus dem Prophagen Rac von Escherichia coli wurde eine Homologiesuche mit dem Programm Blast, BLAST 2.2.27+ durchgeführt (Wheeler, David; Bhagwat, Medha (2007). "Chapter 9 , BLAST QuickStart". In Bergman, Nicholas H. Comparative Genomics Volumes 1 and 2. Methods in Molecular Biology. 395-396. Totowa, NJ: Humana Press). Die Homologiesuche erfolgte gegenüber allen Proteinen, die in den Genomen der folgenden Corynebakterien species kodiert sind: C. accolens, C. ammoniagenes, C. amycolatum, C. aurimucosum, C. bovis, C. diphtheriae, C. efficiens, C. genitalium, C. glucuronolyticum, C. glutamicum, C. jeikeium, C. kroppenstedtii, C. lipophiloflavum, C. matruchotii, C. nuruki, C. pseudogenitalium, C. pseudotuberculosis, C. resistens, C. striatum, C. tuberculostearicum, C. ulcerans, C. urealyticum und C. variabile.

Als Ergebnis wurde die Sequenz cauri_1962 erhalten, die für ein Protein mit einer Länge von 272 Aminosäuren kodiert, wovon 41% identisch und 61% ähnlich zu der Sequenz von RecT sind. Die so ermittelte DNA Sequenz aus C. aurimucosum, die das Rekombinasegen recT enthält, ist als SEQ ID No. 1 angegeben und die Proteinsequenz als SEQ ID No. 2.

### Beispiel 2: Klonierung von Rekombinasen

Die Klonierung von Rekombinasen erfolgte im Expressionsvektor pCLTON2 (A tetracycline inducible expression vector for Corynebacterium glutamicum allowing tightly regulable gene expression. Lausberg F, Chattopadhyay AR, Heyer A, Eggeling L, Freudl R. Plasmid. 2012 68(2):142-7), sowie im Vektor pEKEx3 (The E2 domain of OdhA of Corynebacterium glutamicum has succinyltransferase activity dependent on lipoyl residues of the acetyltransferase AceF. Hoffelder M, Raasch K, van Ooyen J, Eggeling L. J Bacteriol. 2010; 192(19):5203-11).

### Beispiel 2a: Herstellung von pCLTON2-bet

Zur Klonierung von Bet wurde der Vektor pSIM8 (Rekombineering: in vivo genetic engineering in E. coli, S. enterica, and beyond. Sawitzke JA, Thomason LC, Costantino N, Bubunenko M, Datta S, Court DL. Methods Enzymol. 2007;421:171-99) mit dem QIAGEN Plasmid Plus Maxi Kit (Best. Nr. 12963) aus E. coli isoliert. Dieses Plasmid diente als Template zur PCR Amplifikation mit den Primerpaaren bet-F und bet-R.

| | |
|---|---|
| bet-F | aaggagatatagatATGAGTACTGCACTCGCAAC |
| bet-R | TCATGCTGCCACCTTCTGCTC |

Das resultierende Fragment von 0,8 kb wurde über Gelisolierung mit dem Minielute Extraktions Kit (Best. Nr. 28704) von Quiagen isoliert, mit dem Klenow Fragment aufgefüllt, und anschließend mit T4 Polynukleotidkinase von Fermentas (Best. Nr. EK0031 phosphoryliert. Der Vektor pCLTON2 (A tetracycline inducible expression vector for Corynebacterium glutamicum allowing tightly regulable gene expression. Lausberg F, Chattopadhyay AR, Heyer A, Eggeling L, Freudl R. Plasmid. 2012 68(2):142-7) wurde Smal geschnitten und mit Shrimp Alkaline Phosphatase von Fermentas (Best. Nr. EF0511) dephosphoryliert. Das Fragment und der Vektor wurden mit dem Rapid DNA Ligation Kit von Roche (Best. Nr. 11 635 379 001) ligiert, und es wurde E. coli DH5 damit transformiert. Transformierte Zellen wurden auf 100 ng/mL Spektinomycin enthaltendes Komplexmedium ausplattiert.

Zum Test auf gewünschte Ligationsprodukte wurde eine Kolonie-PCR mit den Primerpaaren Pcl_fw und Pcl_rv-pEKEx2_fw durchgeführt.

| | |
|---|---|
| Pcl_fw | GTAACTATTGCCGATGATAAGC |
| Pcl_rv-pEKEx2_fw | CGGCGTTTCACTTCTGAGTTCGGC |

Aus einem Klon, der ein PCR Produkt der Größe 1,17 kb ergab, wurde im größeren Maßstab Plasmid mit dem QIAGEN Plasmid Plus Maxi Kit (Best. Nr. 12963) präpariert. Das Plasmid wurde als pCLTON2-bet bezeichnet, dessen Sequenz als SEQ ID No. 3 bezeichnet wurde.

### Beispiel 2b: Herstellung von pCLTON2-recT

Zur Klonierung von recT wurde der Vektor pRAC3 (Roles of RecJ, RecO, and RecR in RecET-mediated illegitimate recombination in Escherichia coli. Shiraishi K, Hanada K, Iwakura Y, Ikeda H, J Bacteriol. 2002 Sep;184(17):4715-21) mit dem QIAGEN Plasmid Plus Maxi Kit (Best. Nr. 12963) aus E. coli isoliert. Dieses Plasmid diente als Template zur PCR Amplifikation mit den Primerpaaren recT-F und recT-R.

| | |
|---|---|
| recT-F | aaggagatatagatATGACTAAGCAACCACCAATC |
| recT-R | CGGTTATTCCTCTGAATTATCG |

Das resultierende Fragment von 0,8 kb wurde in Beispiel 2a beschrieben isoliert, mit pCLTON2 ligiert, und E. coli DH5 damit transformiert. Transformierte Zellen wurden auf 100 ng/mL Spektinomycin enthaltendes Komplexmedium ausplattiert.

Der Test auf gewünschte Ligationsprodukte wurde eine Kolonie-PCR wie in Beispiel 2a beschrieben durchgeführt. Aus einem Klon der ein PCR Produkt der Größe 1,194 kb ergab, wurde im größeren Maßstab Plasmid präpariert. Das Plasmid wurde als pCLTON2-recT bezeichnet, dessen Sequenz als SEQ ID No. 4 bezeichnet wurde.

### Beispiel 2c: Herstellung von pCLTON2-gp43

Zur Klonierung von gp43 wurde das Gen bei Eurofins-MWG-Operon (Anzingerstr. 7a, 85560 Ebersberg, Deutschland) synthetisiert. Die Sequenz des synthetisierten Fragments ist als SEQ ID No. 10 angeführt. Das Fragment wurde als 1407 bp Fragment mit den Restriktionsenzymen BglII und EcoRI präpariert, mit dem Klenow Fragment behandelt, und anschließend mit T4 Polynukleotidkinase von Fermentas (Best. Nr. EK0031) phosphoryliert. Das Fragment wurde in Beispiel 2a beschrieben isoliert, mit pCLTON2 ligiert, und E. coli DH5 damit transformiert. Transformierte Zellen wurden auf 100 ng/mL Spektinomycin enthaltendes Komplexmedium ausplattiert.

Zum Test auf gewünschte Ligationsprodukte wurde eine Kolonie-PCR wie in Beispiel 2a beschrieben durchgeführt. Aus einem Klon, der ein PCR Produkt der Größe 1,79 kb ergab, wurde im größeren Maßstab Plasmid präpariert. Das Plasmid wurde als pCLTON2-gp43 bezeichnet, dessen Sequenz als SEQ ID No. 5 bezeichnet wurde.

### Beispiel 2d: Herstellung von pCLTON2-gp61

Zur Klonierung von gp61 wurde das Gen bei Eurofins-MWG-Operon (Anzingerstr. 7a, 85560 Ebersberg, Deutschland) synthetisiert. Die Sequenz des synthetisierten Fragments ist als SEQ ID No. 11 angeführt. Das Fragment wurde als 1082 bp mit den Restriktionsenzymen BglII und MunI präpariert, mit dem Klenow Fragment behandelt, und anschließend mit T4 Polynukleotidkinase von Fermentas (Best. Nr. EK0031) phosphoryliert. Es wurde in Beispiel 2a beschrieben isoliert, mit pCLTON2 ligiert, und E. coli DH5 damit transformiert. Transformierte Zellen wurden auf 100 ng/mL Spektinomycin enthaltendes Komplexmedium ausplattiert.

Zum Test auf gewünschte Ligationsprodukte wurde eine Kolonie-PCR wie in Beispiel 2a beschrieben durchgeführt. Aus einem Klon, der ein PCR Produkt der Größe 1,45 kb ergab, wurde im größeren Maßstab Plasmid präpariert. Das Plasmid wurde als pCLTON2-gp61 bezeichnet, dessen Sequenz als SEQ ID No. 6 bezeichnet wurde.

### Beispiel 2e: Herstellung von pCLTON2-rCau

Zur Klonierung von rCau (cauri_1962) wurde das Gen bei Eurofins-MWG-Operon (Anzingerstr. 7a, 85560 Ebersberg, Deutschland) synthetisiert. Die Sequenz des synthetisierten Fragments ist als SEQ ID No. 1 angeführt. Das Fragment wurde als 839 bp mit den Restriktionsenzymen BglII und MunI präpariert, mit dem Klenow Fragment behandelt, und anschließend mit T4 Polynukleotidkinase von Fermentas (Best. Nr. EK0031) phosphoryliert. Es wurde wie in Beispiel 2a beschrieben isoliert, mit pCLTON2 ligiert, und E. coli DH5 damit transformiert. Transformierte Zellen wurden auf 100 ng/mL Spektinomycin enthaltendes Komplexmedium ausplattiert.

Zum Test auf gewünschte Ligationsprodukte wurde eine Kolonie-PCR wie in Beispiel 2a beschrieben durchgeführt. Aus einem Klon, der ein PCR Produkt der Größe 1,22 kb ergab, wurde im größeren Maßstab Plasmid präpariert. Das Plasmid wurde als pCLTON2-rCau bezeichnet, dessen Sequenz als SEQ ID No. 7 bezeichnet wurde.

### Beispiel 2f: Herstellung von pEKEx3-recT

Zur Klonierung von recT in pEKEx3 wurde als Template zur PCR Amplifikation pCLTON2-recT aus Beispiel 2b eingesetzt. Das Gen wurde mit den Primerpaaren Bglll-RBS-RecT-F und EcoRI-RecT-R amplifiziert.

| | |
|---|---|
| BglII-RBS-RecT-F | gcagatctaaggagatatacatATGACTAAGCAACCACCAATCG |
| EcoRI-RecT-R | gcgcgaattccaggCTGAATTATTCCTC |

Das resultierende Fragment von 0,84 kb wurde über Gelisolierung mit dem Minielute Extraktions KIT (Best. Nr. 28704) von Quiagen isoliert, mit dem Klenow Fragment behandelt, und anschließend mit T4 Polynukleotidkinase von Fermentas (Best. Nr. EK003) phosphoryliert. Der Vektor pEKEx3 wurde mit EcoRI und BamHI geschnitten, und das resultierende Fragment von 8298 bp mit Shrimp Alkaline Phosphatase von Fermentas (Best. Nr. EF0511) dephosphoryliert. Das Fragment und der Vektor wurden mit dem Rapid DNA Ligation Kit von Roche (Best. Nr. 11 635 379 001) ligiert, und es wurde E. coli DH5 damit transformiert. Transformierte Zellen wurden auf 100 ng/mL Spektinomycin enthaltendes Komplexmedium ausplattiert.

Zum Test auf gewünschte Ligationsprodukte wurde eine Kolonie-PCR mit den Primerpaaren col-pEKEx3-F und col-pEKEx3-R durchgeführt.

| | |
|---|---|
| col-pEKEx3-F | CGCCGACATCATAACGGTTCTG |
| col-pEKEx3-R | TTATCAGACCGCTTCTGCGTTC |

Aus einem Klon, der ein PCR Produkt der Größe 1,71 kb ergab, wurde im größeren Maßstab Plasmid mit dem QIAGEN Plasmid Plus Maxi Kit (Best. Nr. 12963) präpariert. Das Plasmid wurde als pEKEx3-recT bezeichnet, dessen Sequenz als SEQ ID No. 8 bezeichnet wurde.

### Beispiel 2g: Herstellung von pEKEx3-bet

Zur Klonierung der Rekombinase Bet wurde als Template zur PCR Amplifikation pCLTON2-rCau aus Beispiel 2e eingesetzt. Das Gen wurde mit den Primerpaaren BglII-RBS-bet-F und EcoRI-bet-R amplifiziert.

| | |
|---|---|
| BglII-RBS-bet-F | cggcagatctaaggagatatacatATGAGTACTGCACTCGCAAC |
| EcoRI-bet-R | gcgcggaattCATGCTGCCACCTTCTGC |

Das resultierende Fragment von 0,81 kb wurde über Gelisolierung mit dem Minielute Extraktions Kit (Best. Nr. 28704) von Quiagen isoliert, mit dem Klenow Fragment behandelt, und anschließend mit T4 Polynukleotidkinase von Fermentas (Best. Nr. EK0031) phosphoryliert. Der Vektor pEKEx3 wurde mit EcoRI und BamHI geschnitten, und das resultierende Fragment von 8298 bp mit Shrimp Alkaline Phosphatase von Fermentas (Best. Nr. EF0511) dephosphoryliert. Das Fragment und der Vektor wurden mit dem Rapid DNA Ligation Kit von Roche (Best. Nr. 11 635 379 001) ligiert, und es wurde E. coli DH5 damit transformiert. Transformierte Zellen wurden auf 100 ng/mL Spektinomycin enthaltendes Komplexmedium ausplattiert.

Zum Test auf gewünschte Ligationsprodukte wurde wie in Beispiel 2e eine Kolonie-PCR mit den Primerpaaren col-pEKEx3-F und col-pEKEx3-r durchgeführt. Aus einem Klon, der ein PCR Produkt der Größe 1,08 kb ergab, wurde im größeren Maßstab Plasmid mit dem QIAGEN Plasmid Plus Maxi Kit (Best. Nr. 12963) präpariert. Das Plasmid wurde als pEKEx3-bet bezeichnet, dessen Sequenz als SEQ ID No. 9 bezeichnet wurde.

### Beispiel 3: Herstellung eines Teststammes

Um eine nicht funktionale Kopie eines Kanamycinresistenz-verleihenden Gens in das Chromosom von C. glutamicum ATCC13032 einzubauen, wurde zunächst mit den Primerpaaren Scal-KanR-F / Kan(-)-L-R und Munl-R-R / Kan(-)-R-F zwei zu fusionierende PCR-Fragmente mit dem Vektor pJC1 (Cremer J, Treptow C, Eggeling L, Sahm H. Regulation of enzymes of lysine biosynthesis in Corynebacterium glutamicum. J Gen Microbiol. 1988;134(12):3221-9) als template hergestellt.

| | |
|---|---|
| Scal-KanR-F | CGAGTACTACAAACGCGGCCATAAC |
| Kan(-)-L-R | GTCGGAAGAGGCATAGAATTCCGTCAGCCAGTTTAG |
| Kan(-)-R-F | GCTGACGGAATTCTATGCCTCTTCCGACCATC |
| Munl-R-R | ATACAATTGAACAAAGCCGCCGTCC |

Beide resultierenden PCR-Fragmente wurden mit dem Minielute Extraktions Kit (Best. Nr. 28704) von Quiagen aufgereinigt und in einer Fusions-PCR mit den Primerpaaren Scal-KanR-F / Munl-R-R zum defekten Kanamycinresistenzgen fusioniert. Dies enthält in Position 234 als zusätzliches Nukleotid ein Cytosin, wodurch ein frame-shift vorliegt und das Gen nicht vollständig abgelesen wird. Das entstandene Produkt wurde mit ScaI und MunI restringiert und anschließend in den EcoRI und ScaI geschnittenen pK18mobsacB-lysOP7 (Acetohydroxyacid synthase, a novel target for improvement of L-lysine production by Corynebacterium glutamicum. Blombach B, Hans S, Bathe B, Eikmanns BJ. Appl Environ Microbiol. 2009 Jan;75(2):419-427) kloniert. In diesem Vektor ist das defekte Kanamycinresistenzgen von zwei nicht-codierenden Bereichen des C. glutamicum Genoms flankiert, über welche die homologe Integration in das Genom erfolgt. Anschließend wurde die gesamte Kassette in das C. glutamicum Genom zwischen Position 1.045.503 bis 1.045.596 nach bekannten Verfahren über zweifach positive Selektion integriert (SmaII mobilizable multi-purpose cloning vectors derived from the Escherichia coli plasmids pK18 and pK19: selection of defined deletions in the chromosome of Corynebacterium glutamicum. Schäfer A, Tauch A, Jäger W, Kalinowski J, Thierbach G, Pühler A Gene. 1994 Jul 22;145(1):69-73). Die korrekte Integration des defekten Kanamycinresistenzgens in das Chromosom wurde mit den Primerpaaren coINCR-L2 und coINCR-R2 überprüft. Die Größe des PCR-Fragments betrug 3937 bp.

| | |
|---|---|
| coINCR-L2: | CATTGGTCACCTTTGGCGTGTGG |
| colNCR-R2: | AATCAATGAGCGCCGTGAAGAAGG |

### Beispiel 4: Test auf Rekombinaseaktivität

Die Transformation des Teststammes erfolgte wie bei Tauch et al. für Corynebacterium diphtheriae and C. glutamicum beschrieben (Efficient electrotransformation of Corynebacterium diphtheriae with a mini-replicon derived from the Corynebacterium glutamicum plasmid pGC1. Tauch A, Kirchner O, Löffler B, Götker S, Pühler A, Kalinowski J. Curr Microbiol. 2002 Nov;45(5):362-367). Der Stamm wurde kompetent gemacht und jeweils 0.5 Mikrogramm des für die Rekombinase kodierenden Vektors zur Elektroporation eingesetzt. Spektinomycinresistente Klone wurden auf dem Komplexmedium Brain-Heart-Infusion-Sorbitol, BHIS, (High efficiency electroporation of intact Corynebacterium glutamicum cells. Liebl W, Bayerl A, Schein B, Stillner U, Schleifer KH. FEMS Microbiol Lett. 1989 Dec;53(3):299-303), das 100 Mikrogramm Spektinomycin (BHIS-Spec100) enthielt, selektiert.
Jeweils ein Klon des Teststammes, der den Vektor pCLTON2-bet, pCLTON2-recT, pCLTON2-gp43, pCLTON2-gp61, pCLTON2-rCau, pEKEx3-recT, bzw. pEKEx3-bet enthielt, wurde in 50 ml BHIS-Spec100 angeimpft und über Nacht bei 130 rpm und 30 °C im Erlenmeyerkolben kultiviert. Am nächsten Morgen wurden 500 ml BHIS-Spec100 + IPTG (0,5 mM bei Verwendung von pEKEx3-recT, pEKEx3-bet) oder Tetrazyklin (250 ng / ml bei Verwendung von pCLTON2-bet, pCLTON2-recT, pCLTON2-gp43, pCLTON2-gp61, pCLTON2-rCau) mit 10 ml aus dem über Nacht inkubierten Medium angeimpft und für 4 bis 6 h bis zum Erreichen einer OD von 1,5 bis 2 kultiviert. Anschließend wurde die Kultur für 30 min auf Eis gekühlt, zweimal mit 50 ml 10 % Glyzerin, 1 mM Tris pH 8 und anschließend zweimal mit 10 % Glyzerin gewaschen. Das Zellpellet wurde im Rücklauf und zusätzlichen 1 ml Glyzerin 10 % resuspendiert, zu je 150 µl aliquotiert, in flüssigem Stickstoff schockgefroren und bis zum Gebrauch bei -75 °C gelagert. Zum Gebrauch wurden die Zellen innerhalb von 20 min schonend auf Eis aufgetaut, und mit 1 µg DNA gemischt.

Als DNA wurde das oligo Kan100*, mit der Sequenz
ATGCATCATCAGGAGTACGGATAAAATGCTTGATGGTCGGAAGAGGCATAAATTCCGTC AGCCAGTTTAGTCTGACCATCTCATCTGTAACATCATTGGC eingesetzt. Diese DNA wurde bei Eurofins-MWG-Operon (Anzingerstr. 7a, 85560 Ebersberg, Deutschland) synthetisiert.

Die Suspension aus Zellen und 1 Mikrogramm DNA wurde in Elektroporationsküvetten übertragen und mit 800 µl eiskaltem 10 % Glyzerin vorsichtig überschichtet und anschließend elektroporiert. Zur Regeneration wurden die Zellen sofort in 4 ml auf 46 °C vortemperiertes BHIS überführt und für 6 min bei 46 °C inkubiert. Anschließend erfolgte eine 1 bis 6 stündige Kultivierung bei 30 °C und 170 rpm im 15 ml Falcon. Danach wurden die Zellen auf BHIS, das 50 Mikrogramm pro Milliliter Kanamycin enthielt, übertragen. Das Ergebnis ist in Tabelle 1 dargestellt. Es zeigt sich, dass pro Ansatz maximal 57 Zellen spontan Kanamycin resistent sind, die höchste Rekombinationsfrequenz von 20054 Klonen wurde mit pEKEx3-recT erhalten, und abnehmende Rekombinaseaktivität mit pCLTON2-recT, pEKEx3-bet, pCLTON2-rCau, und pCLTON2-gp61. Die Rekombinaseaktivität mit pCLTON2-gp43 liegt kaum über dem Hintergrund, und pCLTON2-bet ist nicht aktiv.

### Beispiel 5: Optimierung der Rekombinaseaktivität

Der Teststamm mit pEKEx3-recT wurde in 50 ml BHIS-Spec100 angeimpft und über Nacht bei 130 rpm und 30 °C im Erlenmeyerkolben kultiviert. Am nächsten Morgen wurden 500 ml BHIS-Spec100 + 0,5 mM IPTG mit 10 ml aus dem über Nacht inkubierten Medium angeimpft und 0, 1, oder 4 Stunden kultiviert. Der Teststamm mit pCLTON2-recT wurde in 50 ml BHIS-Spec100 angeimpft und über Nacht bei 130 rpm und 30 °C im Erlenmeyerkolben kultiviert. Am nächsten Morgen wurden 500 ml BHIS-Spec100 + 250 Nanogramm Tetracyklin mit 10 ml aus dem über Nacht inkubierten Medium angeimpft und 0, 1, oder 4 Stunden kultiviert. Anschließend wurde die Kultur für 30 min auf Eis gekühlt, zweimal mit 50 ml 10% Glyzerin, 1 mM Tris pH 8 und anschließend zweimal mit 10 % Glyzerin gewaschen. Das Zellpellet wurde im Rücklauf und zusätzlichen 1 ml Glyzerin 10 % resuspendiert, zu je 150 µl aliquotiert, in flüssigem Stickstoff schockgefroren und bis zum Gebrauch bei -75 °C gelagert. Zum Gebrauch wurden die Zellen innerhalb von 20 min schonend auf Eis aufgetaut, und mit 1 Mikrogramm DNA gemischt.

Die Elektroporation und Regeneration erfolgte wie in Beispiel 4 beschrieben. Tabelle 2 zeigt, dass nach 4 Stunden Induktion bei Nutzung der Rekombinase recT im Vektor pEKEx3-recT maximale Rekombinationsfrequenz vorliegt.

Zur weiteren Optimierung wurden Zellen des Teststamm mit pEKEx3-recT wie zuvor benutzt, aber steigende Mengen an DNA zugesetzt. Tabelle 3 zeigt, dass bei Nutzung der Rekombinase recT im Vektor pEKEx3-recT bei einer Konzentration von 10 Mikrogramm DNA maximale Rekombinationsfrequenz vorliegt.

### Beispiel 6: Gewinnung eines Lysinproduzenten durch Rekombineering im lysC Gen mit einem DNA Molekül

Zur direkten Isolation eines vermehrt Lysin produzierenden Stammes, ausgehend von einem Ausgangsstamm, wurde C. glutamicum ATCC13032 mit dem Nanosensor pSenLys transformiert. Der Nanosensor pSenLys ist in WO2011138006 beschrieben. Zellen des resultierenden Stammes wurden mit pEKEx3-recT transformiert und die Rekombinase wurde induziert, wie im Beispiel 4 beschrieben. Die DNA lysC-100* wurde synthetisiert bei Eurofins-MWG-Operon (Anzingerstr. 7a, 85560 Ebersberg, Deutschland). Sie ist als SEQ ID No. 32 hinterlegt.

IysC-100*:

10 Mikrogramm der DNA lysC-100* wurden durch Elektroporation, wie in Beispiel 4 beschrieben, in den Stamm transferiert (Figur 1, C1). Anschließend erfolgte Regeneration des Stammes für 4 Stunden in BHIS 100 Mikrogramm pro Milliliter Spektinomycin. Anschließend wurden die Zellen abzentrifugiert und in 700 Mikroliter CGXII-Glukose resuspendiert. Dieses Minimalmedium ist bei Keilhauer et al. beschrieben (Isoleucine synthesis in Corynebacterium glutamicum: molecular analysis of the ilvB-ilvN-ilvC operon. Keilhauer C, Eggeling L, Sahm H. J Bacteriol. 1993 Sep;175(17):5595-603). Die Zellen wurden bis zum Erreichen der stationären Phase für 40 Stunden bei 30 Grad Celsius inkubiert. Danach wurde 1:10 in neues CGXII-Glukose Medium überimpft, 4 Stunden inkubiert, und die Zellsuspension der zytometrischen Produktanalyse und Selektion von Einzelzellen zugeführt (Figur 2, C2).

Zur durchflusszytometrischen Analyse und Sortierung der Zellen mit hoher Fluoreszenz wurde die Zellsuspension in CGXII-Glukose Medium auf eine optische Dichte unter 0,1 eingestellt, und unmittelbar dem ARIA II high-speed cell sorter (Becton Dickinson GmbH, Tullastr. 8-12, 69126 Heidelberg) zugeführt. Die Analyse erfolgte mit den Anregungswellenlängen von 488 und 633 nm und die Detektion bei den Emissionswellenlängen von 530 ± 15 nm und 660 ± 10 nm bei einem Probendruck von 70 psi. Die Daten wurden mit der zum Gerät gehörenden Software-Version BD DIVA 6.1.3 analysiert. Das elektronische gating wurde anhand des forward und backward scatters eingestellt, um nicht-bakterielle Partikel auszuschließen. Um EYFP-positive Zellen zu sortieren, wurde die nächste Stufe des elektronischen gatings gewählt, um nicht-fluoreszierende Zellen auszuschließen. Auf diese Weise wurden 51 fluoreszierende Zellen auf Petrischalen, die BHIS Medium enthielten, aussortiert.

Die Petrischale wurde 30 Stunden bei 30 Grad Celsius inkubiert, und anschließend wurde mit je einem Klon eines der 46 Reaktionsgefäße der Mikrotiterplatte Flowerplate (48-well) des BioLector Kultivierungssystems beimpft (m2plabs GmbH, Aachen, Germany). Jedes Reaktionsgefäß enthielt 0,7 Mikroliter CGXII-Glukose. Eines der Reaktionsgefäße wurde mit einer Negativkontrolle, und eines mit einer Positivkontrolle beimpft. Anschließend wurde die Mikrotiterplatte bei 30°C, 1200 UpM, Schüttelradius 3 mm 2 Tage inkubiert. In dem BioLector Kultivierungssystem wurde online das Wachstum als Streulicht bei 620 nm aufgezeichnet, sowie die Fluoreszenz der Kultur bei einer Anregungswellenlänge von 485 nm und einer Emissionswellenlänge von 520 nm kontinuierlich aufgezeichnet.

Nach 2 Tagen wurde die spezifische Fluoreszenz der Kulturen aus den aufgezeichneten Daten ermittelt. Sie war in 33 klonalen Kulturen mindestens vierfach gegenüber der Negativkontrolle erhöht. Von 12 dieser Kulturen wurde die lysC Sequenz im Genom bestimmt. In allen Fällen war das Cytosin in Position 932 des Gens gegen ein Thymidin ausgetauscht. Die Sequenz entsprach somit dem Sequenzbereich der auf dem synthetisierten oligo lysC-100* vorlag und zur Lysinbildung bei C. glutamicum führt (Binder et al. Genome Biology 2012, 13:R40).

### Beispiel 7: Gewinnung eines Lysinproduzenten durch Rekombineering im murE Gen mit mehreren DNA Molekülen gleichzeitig

Zur direkten Isolation eines vermehrt Lysin produzierenden Stammes, ausgehend von einem Ausgangsstamm durch murE Mutationen, wurde der in Beispiel 6 beschriebene Ausgangsstamm C. glutamicum ATCC13032 mit pSenLys und pEKEx3-recT benutzt. Die einzelnen murE DNA oligos wurden durch Eurofins-MWG-Operon (Anzingerstr. 7a, 85560 Ebersberg, Deutschland) synthetisiert. Es wurden folgende murE Sequenzen benutzt: murEG81amb*, SEQ ID No. 12; murEG81A*, SEQ ID No. 13; murEG81C*, SEQ ID No. 14; murE G81D*, SEQ ID No. 15; murEG81E*, SEQ ID No. 16; murEG81F*, SEQ ID No. 17; murEG81H*, SEQ ID No. 18; murEG81I*, SEQ ID No. 19; murEG81K*, SEQ ID No. 20; murEG81L*, SEQ ID No. 21; murEG81M*, SEQ ID No. 22; murEG81N*, SEQ ID No. 23; murEG81P*, SEQ ID No. 24; murEG81Q*, SEQ ID No. 25; murEG81R*, SEQ ID No. 26; murEG81S*, SEQ ID No. 27; murEG81T*, SEQ ID No. 28; murEG81V*, SEQ ID No. 29; murEG81W*, SEQ ID No. 30; murEG81Y*, SEQ ID No. 31.

Je 1 Mikrogramm dieser DNA oligos wurde entnommen, und die resultierenden 20 Mikrogramm mit einem Aliquot Zellen vermischt und durch Elektroporation wie in Beispiel 4 beschrieben in den Stamm transferiert (Figur 2, C1). Anschließend erfolgte Regeneration der Zellen mit den anschließenden Kultivierungen und der durchflusszytometrischen Analyse und Sortierung der Zellen (Figur 2, C2) wie in Beispiel 5 beschrieben.

Auf diese Weise wurden 62 fluoreszierende Zellen auf Petrischalen, die BHIS Medium enthielten, aussortiert. Die Petrischale wurde 30 Stunden bei 30 Grad Celsius inkubiert, und anschließend wurde mit je einem Klon eines der 46 Reaktionsgefäße der Mikrotiterplatte Flowerplate (48-well) des BioLector Kultivierungssystems beimpft (m2plabs GmbH, Aachen, Germany). Jedes Reaktionsgefäß enthielt 0,7 Mikroliter CGXII-Glukose. Eines der Reaktionsgefäße wurde mit einer Negativkontrolle, und eines mit einer Positivkontrolle beimpft. Anschließend wurde die Mikrotiterplatte bei 30°C, 1200 UpM, Schüttelradius 3 mm 2 Tage inkubiert. In dem BioLector Kultivierungssystem wurde online das Wachstum als Streulicht bei 620 nm aufgezeichnet, sowie die Fluoreszenz der Kultur bei einer Anregungswellenlänge von 485 nm und einer Emissionswellenlänge von 520 nm kontinuierlich aufgezeichnet.

Nach 2 Tagen wurde die spezifische Fluoreszenz der Kulturen aus den aufgezeichneten Daten ermittelt. Sie war in 33 klonalen Kulturen mindestens zwölffach gegenüber der Negativkontrolle erhöht. Von 21 Kulturen erfolgte zur Verfizierung der Produktbildung (Figur 2, C3) noch eine L-Lysinbestimmung im Medium. Die Lysinbestimmung erfolgte als o-Phthdialdehyd Derivat mittels Hochdruckflüssigchromatografie mit einem uHPLC 1290 Infinity system (Agilent) auf einer Zorbax Eclipse AAA C18 3.5 micron 4.6 x 75 mm Revered Phase Säule und einem Fluoreszenzdetektor. Als Eluenz wurde ein Gradient aus 0.01 M Na-Borat pH 8.2 mit steigender Methanolkonzentration benutzt und Detektion der fluoreszierenden Isoindolderivate erfolgte bei einer Anregungswellenlänge von 230 nm und einer Emissionswellenlänge von 450 nm. Es wurden die in Tabelle 4 gezeigten L-Lysinwerte bestimmt, die gegenüber dem Ausgangsstamm eine Verbesserung der L-Lysinbildung zeigen.

Von diesen 21 Klonen wurde die murE Sequenz im Genom bestimmt. Die Sequenzierung erfolgte nach PCR Amplifikation bei dem Unternehmen Eurofins-MWG-Operon (Anzingerstr. 7a, 85560 Ebersberg, Deutschland). Die erhaltenen Mutationen sind in Tabelle 4 zusammengefasst. Es zeigt sich, dass auf diese Weise 10 verschiedene murE Mutationen ausgehend von Ausgangsstamm erhalten wurden, von denen neun zu einer erhöhten Lysinbildung gegenüber dem Ausgangsstamm führten. Die Sequenzen der erhaltenen murE Allele sind SEQ ID No. 33, murEG81W; SEQ ID No. 34, murEG81Y; SEQ ID No. 35, murEG81N; SEQ ID No. 36, murEG81C; SEQ ID No. 37, murEG81S; SEQ ID No. 38, murEG81F; SEQ ID No. 39, murEG81V; SEQ ID No. 40, murEG81L; SEQ ID No. 41, murEG81H; SEQ ID No. 42, murEG81I; SEQ ID No. 43, murEG81T; und SEQ ID No. 44, murEG81 R.

**Tabelle 1. Vergleich von Rekombinaseaktivitäten in Corynebacterium glutamicum**

| Vektor | cfu^{a} (rec) | cfu (spont) |
|---|---|---|
| pCLTON2-bet | 8 | 0 |
| pCLTON2-recT | 12513 | 31 |
| pCLTON2-gp43 | 97 | 57 |
| pCLTON2-gp61 | 306 | 1 |
| pCLTON2-rCau | 2475 | 7 |
| pEKEx3-recT | 20054 | 44 |
| pEKEx3-bet | 6491 | 12 |

| | | |
|---|---|---|
| ^{a} cfu (rec) gibt die Anzahl Kanamycin resistenter Klone die durch Rekombineering mit dem Kan* oligo entstanden sind, an; cfu (spont) ist die Anzahl spontan Kanamycin resistenter Klone die aus einem Kontrollansatz, der Wasser anstelle des Kan* oligos enthielt, hervorgegangen sind. Es ist deutlich zu erkennen, dass mit der Rekombinase recT in pEKEx3-recT oder auch pCLTON2-recT hohe Rekombinationeffizienz erreicht wird. Auch mit der Rekombinase rCau aus Corynebacterium aurimucosum wird sehr hohe Rekombinationeffizienz erreicht, die deutlich über derjenigen spontan resistenter Klone liegt. | | |

**Tabelle 2. Vergleich von Rekombinaseaktivitäten nach unterschiedlicher Induktionszeit**

| Vektor | Induktionszeit (h) | cfu^{a} (rec) | cfu (spont) |
|---|---|---|---|
| pCLTON2-recT | 0 | 101 | 2 |
| pCLTON2-recT | 1 | 816 | 1 |
| pCLTON2-recT | 4 | 8831 | 6 |
| pEKEx3-recT | 0 | 1238 | 6 |
| pEKEx3-recT | 1 | 53460 | 7 |
| pEKEx3-recT | 4 | 33165 | 7 |

| | | | |
|---|---|---|---|
| ^{a} cfu (rec) und cfu (spont) wie in Tabelle 1. Der Einfluss der Induktionszeit zur Expression der Rekombinase auf Rekombinationeffizienz ist deutlich zu erkennen. | | | |

**Tabelle 3. Vergleich von Rekombinaseaktivitäten bei Einsatz unterschiedlicher DNA Menge**

| Vektor | Menge (µg) | cfu (rec) |
|---|---|---|
| pEKEx3-recT | 0 | 13 |
| pEKEx3-recT | 0,05 | 1139 |
| pEKEx3-recT | 0,1 | 2673 |
| pEKEx3-recT | 0,5 | 25080 |
| pEKEx3-recT | 1 | 15840 |
| pEKEx3-recT | 5 | 301950 |
| pEKEx3-recT | 10 | 950400 |
| pEKEx3-recT | 25 | 940500 |
| pEKEx3-recT | 50 | 871200 |
| pEKEx3-recT | 100 | 831600 |

| | | |
|---|---|---|
| ^{a} cfu (rec) und cfu (spont) wie in Tabelle 1. Es ist deutlich zu erkennen, wie die zum Rekombineeringansatz zugegebene DNA Menge die Rekombineeringfrequenz erhöht. Bei etwa 10 Mikrogramm DNA ist die maximale Rekombineeringfrequenz erreicht. | | |

**Tabelle 4. Ergebnis der Sequenzierung von murE Allelen in Klonen, die durch Rekombineering und direkte zytometrische Produktanalyse durch den Nanosensor (Figur 3) und nach Verfizierung (Figur 3, C3.B) erhalten wurden, sowie deren Lysinbildung und Fluoreszenz in Kulturen.**

| Stamm | murE Codon 241-243 | MurE Aminosäure 81 | Fluoreszenz (AU) | Lysin (mM) |
|---|---|---|---|---|
| Ausgangsstamm | GGA | (G) Glycin | 0,07 | 0 |
| I.4 | TGG | (W) Tryptophan | 1,80 | 11 |
| I.6 | TAC | (Y) Tyrosin | 1,17 | 9 |
| I.7 | AAC | (N) Asparagin | 0,73 | 5 |
| I.24 | TTC | (F) Phenylalanin | 1,36 | 8 |
| I.25 | TGC | (C) Cystein | 1,08 | 7 |
| I.34 | CTG | (L) Leucin | 1,83 | 12 |
| II.1 | CAC | (H) Histidin | 0,46 | 1 |
| II.4 | GTG | (V) Valin | 1,47 | 9 |
| II.5 | ACC | (T) Threonin | 0,47 | 1 |
| II.24 | ATC | (I) Isoleucin | 1,85 | 10 |
| II.23 | CGC | (R) Arginin | 2,05 | 10 |

**Sequenzen gemäß Sequenzprotokoll:**

| SEQ ID | Name |
|---|---|
| SEQ ID No. 1 | Rekombinasegen |
| SEQ ID No. 2 | Rekombinase |
| SEQ ID No. 3 | pCLTON2-bet |
| SEQ ID No. 4 | pCLTON2-recT |
| SEQ ID No. 5 | pCLTON2-gp43 |
| SEQ ID No. 6 | pCLTON2-gp61 |
| SEQ ID No. 7 | pCLTON2-rCau |
| SEQ ID No. 8 | pEKEx3-recT |
| SEQ ID No. 9 | pEKEx3-bet |
| SEQ ID No. 10 | gp43 adaptiert |
| SEQ ID No. 11 | gp61 adaptiert |
| SEQ ID No. 12 | murEG81amb* |
| SEQ ID No. 13 | murEG81A* |
| SEQ ID No. 14 | murEG81C* |
| SEQ ID No. 15 | murEG81D* |
| SEQ ID No. 16 | murEG81E* |
| SEQ ID No. 17 | murEG81F* |
| SEQ ID No. 18 | murEG81H* |
| SEQ ID No. 19 | murEG81I* |
| SEQ ID No. 20 | murEG81K* |
| SEQ ID No. 21 | murEG81L* |
| SEQ ID No. 22 | murEG81M* |
| SEQ ID No. 23 | murEG81N* |
| SEQ ID No. 24 | murEG81P* |
| SEQ ID No. 25 | murEG81Q* |
| SEQ ID No. 26 | murEG81R* |
| SEQ ID No. 27 | murEG81S* |
| SEQ ID No. 28 | murEG81T* |
| SEQ ID No. 29 | murEG81V* |
| SEQ ID No. 30 | murEG81W* |
| SEQ ID No. 31 | murEG81Y* |
| SEQ ID No. 32 | lyse-100* |
| SEQ ID No. 33 | murEG81W |
| SEQ ID No. 34 | murEG81Y |
| SEQ ID No. 35 | murEG81N |
| SEQ ID No. 36 | murEG81C |
| SEQ ID No. 37 | murEG81S |
| SEQ ID No. 38 | murEG81F |
| SEQ ID No. 39 | murEG81V |
| SEQ ID No. 40 | murEG81L |
| SEQ ID No. 41 | murEG81H |
| SEQ ID No. 42 | murEG81I |
| SEQ ID No. 43 | murEG81T |
| SEQ ID No. 44 | murEG81R |

## Patentansprüche

1. Ein gegenüber seinem Wildtyp genetisch veränderter Mikroorganismus, der eine für einen Metabolitsensor kodierende Gensequenz umfasst, die für ein Protein kodiert, das eine Aminosäure, eine organische Säure, eine Fettsäure, ein Vitamin oder einen pflanzlichen Wirkstoff erkennt,
**dadurch gekennzeichnet**,
das der Mikroorganismus einen Vektor, enthaltend ein Gen kodierend für eine Rekombinase die nicht im Wildtyp des Mikroorganismus vorhanden ist nach einer der Sequenzen 8, 4, 9, 7, 6, 5 oder 3 enthält.

2. Mikroorganismus nach Anspruch 1,
**dadurch gekennzeichnet,**
**dass** die für eine Rekombinase kodierende Gensequenz eine Sequenz ist, die für ein Protein kodiert, das extrazellulär zugesetzte DNA mit zelleigner DNA rekombiniert.

3. Mikroorganismus nach einem der Ansprüche 1 oder 2,
**dadurch gekennzeichnet,**
**dass** der Mikroorganismus ein Mikroorganismus der Gattung Corynebacterium, Enterobakterium oder Escherichia ist.

4. Verfahren zum Identifizieren eines Mikroorganismus aus der Gruppe bestehend aus Corynebacterium, Enterobakterium oder Escherichia, enthaltend einen Vektor nach Sequenz 4 oder 8 mit gegenüber seinem Wildtyp erhöhter intrazellulärer Konzentration eines bestimmten Metaboliten aus der Gruppe Aminosäure, organische Säure, Fettsäure, Vitamine oder pflanzlicher Wirkstoff in einer Zellsuspension, beinhaltend die Verfahrensschritte:
i) Bereitstellen einer Zellsuspension beinhaltend den Mikroorganismus aus der Gruppe Corynebacterium, Enterobakterium oder Escherichia, der einen Vektor nach Sequenz 4 oder 8 und zusätzlich eine für einen Metabolitsensor kodierende Gensequenz enthält, die für einen Metabolitsensor kodiert, der Metabolite aus der Gruppe Aminosäure, organische Säure, Fettsäure, Vitamin oder pflanzlicher Wirkstoff erkennt;
ii) Genetisches Verändern der Zellen gemäß Schritt i) durch Rekombineering unter Zugabe von DNA, die mindestens ein verändertes Gen G1 bis Gn oder mindestens eine Mutation M1 bis Mm enthält, unter Erhalt einer Zellsuspension, in der sich die Zellen hinsichtlich der intrazellulären Konzentration des Metaboliten unterscheiden;
iii) Identifizieren einzelner Zellen in der Zellsuspension mit erhöhter intrazellulärer Konzentration des Metaboliten durch Fluoreszenzdetektion mittels eines Metabolitsensors für Aminosäuren, organische Säuren, Fettsäuren, Vitamine oder pflanzliche Wirkstoffe.

5. Verfahren zur Herstellung von einem gegenüber seinem Wildtyp genetisch veränderten Mikroorganismus aus der Gruppe bestehend aus Corynebacterium, Enterobakterium oder Escherichia mit optimierter Produktion eines Metaboliten aus der Gruppe Aminosäure, organische Säure, Fettsäure, Vitamine oder pflanzlicher Wirkstoff, beinhaltend folgende Schritte:
i) Bereitstellen einer Zellsuspension beinhaltend den Mikroorganismus aus der Gruppe Corynebacterium, Enterobakterium oder Escherichia, der einen Vektor nach Sequenz 4 oder 8 und zusätzlich eine für einen Metabolitsensor kodierende Gensequenz enthält, die für einen Metabolitsensor kodiert, der Metabolite aus der Gruppe Aminosäure, organische Säure, Fettsäure, Vitamine oder pflanzlicher Wirkstoff erkennt;
ii) Genetisches Verändern der Zellen gemäß Schritt i) durch Rekombineering unter Zugabe von DNA, die mindestens ein verändertes Gen G1 bis Gn oder mindestens eine Mutation M1 bis Mm enthält, unter Erhalt einer Zellsuspension, in der sich die Zellen hinsichtlich der intrazellulären Konzentration eines bestimmten Metaboliten unterscheiden;
iii) Identifizieren einzelner Zellen in der Zellsuspension mit erhöhter intrazellulärer Konzentration des Metaboliten durch Fluoreszenzdetektion mittels eines Metabolitsensors für Aminosäuren, organische Säuren, Fettsäuren, Vitamine oder pflanzliche Wirkstoffe;
iv) Abtrennen der identifizierten Zellen aus der Zellsuspension;
v) Identifizieren mindestens eines genetisch veränderten Gens G1 bis Gn oder mindestens einer Mutation M1 bis Mm in den identifizierten und abgetrennten Zellen, welche für die erhöhte intrazelluläre Konzentration des Metaboliten verantwortlich ist;
vi) Herstellung einer gegenüber ihrem Wildtyp genetisch veränderten Produktionszelle mit optimierter Produktion des Metaboliten, dessen Genom mindestens eines der Gene G1 bis Gm und/oder mindestens eine Mutation M1 bis Mm umfasst.

6. Verfahren nach einem der Ansprüche 4 bis 5,
**dadurch gekennzeichnet,**
**dass** die genetische Veränderung der Zelle gemäß Schritt ii) durch eine Rekombinase erfolgt, die eine oder mehrere in die Zelle eingebrachte DNA-Moleküle, die das veränderte Gen oder die veränderten Gene G1 bis Gn und/oder die Mutation oder die Mutationen M1 bis Mm enthält, in die zelleigene DNA einbaut, die als Chromosom oder Plasmid vorliegt.

7. Verfahren nach einem der Ansprüche 5 oder 6,
**dadurch gekennzeichnet,**
**dass** DNA für mindestens ein verändertes Gen G1 bis Gn und/oder mindestens eine Mutation M1 bis Mm eingesetzt wird, die für einen der Schritte aus dem Biosyntheseweg des Metaboliten kodieren.

8. Verfahren zur Herstellung von Metaboliten, beinhaltend die Verfahrensschritte:
a) Herstellung einer gegenüber ihrem Wildtyp genetisch veränderten Zelle mit optimierter Produktion eines bestimmten Metaboliten durch ein Verfahren nach den Ansprüchen 5 bis 7,
b) Kultivieren der Zelle in einem Kulturmedium, beinhaltend Nährstoffe unter Bedingungen, unter denen die Zelle aus den Nährstoffen den bestimmten Metaboliten produziert.

9. Verfahren nach Anspruch 8,
**dadurch gekennzeichnet,**
**dass** der Metabolit eine Komponente aus der Gruppe Aminosäuren, organische Säuren, Fettsäuren, Vitamine oder pflanzliche Wirkstoffe ist.

## Claims

1. A microorganism that has been genetically modified compared to the corresponding wildtype, which comprises a gene sequence coding for a metabolite sensor, which codes for a protein that recognises an amino acid, organic acid, fatty acid, vitamin or plant-based active substance,
**characterised in that**
the microorganism contains a vector containing a gene coding for a recombinase that is not present in the wildtype of the microorganism according to one of sequences 8, 4, 9, 7, 6, 5 or 3.

2. Microorganism according to claim 1,
**characterised in that**
the gene sequence coding for a recombinase is a sequence, which codes for a protein that recombines the extracellular added DNA with the cell's own DNA.

3. Microorganism according to one of claims 1 or 2,
**characterised in that**
the microorganism is a microorganism of the corynebacterium, enterobacterium or escherichia genus.

4. Method for identifying a microorganism from the group consisting of corynebacterium, enterobacterium or escherichia, containing a vector according to sequence 4 or 8 with increased concentration of a particular metabolite from the amino acid, organic acid, fatty acid, vitamin or plant-based active substance group, compared to the corresponding wildtype, in a cell suspension, including the process steps:
i) Providing a cell suspension containing the microorganism from the corynebacterium, enterobacterium or escherichia group, which contains a vector according to sequence 4 or 8 and also a gene sequence coding for a metabolite sensor, which codes for a metabolite sensor that recognises metabolites from the amino acid, organic acid, fatty acid, vitamin or plant-based active substance group;
ii) Genetically modifying the cells according to step i) through recombineering, adding DNA that contains at least one modified gene G1 to Gn or at least one mutation M1 to Mm, obtaining a cell suspension, in which the cells differ with regard to the intracellular concentration of the metabolite;
iii) Identifying individual cells in the cell suspension with increased intracellular concentration of the metabolite through fluorescence detection by means of a metabolite sensor for amino acids, organic acids, fatty acids, vitamins or plant-based active substances.

5. Method for producing a microorganism that has been genetically modified compared to the corresponding wildtype from the group consisting of corynebacterium, enterobacterium or escherichia with optimised production of a metabolite from the amino acid, organic acid, fatty acid, vitamin or plant-based active substance group, including the following steps:
i) Providing a cell suspension containing the microorganism from the corynebacterium, enterobacterium or escherichia group, which contains a vector according to sequence 4 or 8 and also a gene sequence coding for a metabolite sensor, which codes for a metabolite sensor that recognises metabolites from the amino acid, organic acid, fatty acid, vitamin or plant-based active substance group;
ii) Genetically modifying the cells according to step i) through recombineering, adding DNA that contains at least one modified gene G1 to Gn or at least one mutation M1 to Mm, obtaining a cell suspension, in which the cells differ with regard to the intracellular concentration of a particular metabolite;
iii) Identifying individual cells in the cell suspension with increased intracellular concentration of the metabolite through fluorescence detection by means of a metabolite sensor for amino acids, organic acids, fatty acids, vitamins or plant-based active substances;
iv) Separating the cells that have been identified from the cell suspension;
v) Identifying at least one genetically modified gene G1 to Gn or at least one mutation M1 to Mm in the cells that have been identified and separated, which is responsible for the increased intracellular concentration of the metabolite;
vi) Producing a production cell that has been genetically modified compared to the corresponding wildtype with optimised production of the metabolite, the genome of which comprises at least one of genes G1 to Gm and/or at least one mutation M1 to Mm.

6. Method according to one of claims 4 to 5,
**characterised in that**
the genetic modification of the cells according to step ii) is carried out through a recombinase, which incorporates one or more DNA molecules that have been put into the cell, which contains the modified gene or modified genes G1 to Gn and/or the mutation or mutations M1 to Mm, into the cell's own DNA, which is present as a chromosome or plasmid.

7. Method according to one of claims 5 or 6
**characterised in that**
DNA is used for at least one modified gene G1 to Gn and/or at least one mutation M1 to Mm, which code for one of the steps from the biosynthesis path of the metabolite.

8. Method for producing metabolites, including the process steps:
a) Producing a genetically modified cell compared to the corresponding wildtype with optimised production of a particular metabolite by a method according to claims 5 to 7.
b) Cultivating the cell in a culture medium containing nutrients under conditions in which the cell produces the particular metabolite from the nutrients.

9. Method according to claim 8,
**characterised in that**
the metabolite is a component from the amino acid, organic acid, fatty acid, vitamin or plant-based active substance group.

## Revendications

1. Microorganisme génétiquement modifié par rapport à son type sauvage, qui comprend une séquence génique codant pour un capteur de métabolites, qui code pour une protéine qui reconnaît un acide aminé, un acide organique, un acide gras, une vitamine ou un principe actif végétal,
**caractérisé en ce que**
le microorganisme contient un vecteur contenant un gène codant pour une recombinase qui n'est pas présente dans le type sauvage du microorganisme, selon l'une des séquences 8, 4, 9, 7, 6, 5 ou 3.

2. Microorganisme selon la revendication 1,
**caractérisé en ce que**
la séquence génique codant pour une recombinase est une séquence qui code pour une protéine qui recombine un ADN extracellulairement ajouté à un ADN naturellement produit dans la cellule.

3. Microorganisme selon l'une des revendications 1 ou 2,
**caractérisé en ce que**
le microorganisme est un microorganisme du genre Corynebacterium, Enterobacterium ou Escherichia.

4. Procédé pour l'identification d'un microorganisme du groupe consistant en Corynebacterium, Enterobacterium ou Escherichia, contenant un vecteur selon la séquence 4 ou 8, présentant une concentration intracellulaire, augmentée par rapport à son type sauvage, d'un certain métabolite du groupe acide aminé, acide organique, acide gras, vitamines ou principe actif végétal, dans une suspension cellulaire, comprenant les étapes de procédé :
i) mise à disposition d'une suspension cellulaire comprenant le microorganisme du groupe Corynebacterium, Enterobacterium ou Escherichia, qui contient un vecteur selon la séquence 4 ou 8 et en outre une séquence génique codant pour un capteur de métabolites, qui code pour un capteur de métabolites qui reconnaît les métabolites du groupe acide aminé, acide organique, acide gras, vitamine ou principe actif végétal ;
ii) modification génétique des cellules selon l'étape i), par recombinaison avec addition d'un ADN qui contient au moins un gène G1 à Gn modifié ou au moins une mutation M1 à Mm, avec obtention d'une suspension cellulaire dans laquelle les cellules se distinguent pour ce qui est de la concentration intracellulaire du métabolite ;
iii) identification des cellules individuelles, dans la suspension cellulaire, présentant une concentration intracellulaire augmentée du métabolite, par détection par fluorescence à l'aide d'un capteur de métabolites pour des acides aminés, des acides organiques, des acides gras, des vitamines ou des principes actifs végétaux.

5. Procédé pour la fabrication d'un microorganisme génétiquement modifié par rapport à son type sauvage, du groupe consistant en Corynebacterium, Enterobacterium ou Escherichia, avec production optimisée d'un métabolite du groupe acide aminé, acide organique, acide gras, vitamines ou principe actif végétal, comprenant les étapes suivantes :
i) mise à disposition d'une suspension cellulaire comprenant le microorganisme du groupe Corynebacterium, Enterobacterium ou Escherichia, qui contient un vecteur selon la séquence 4 ou 8 et en outre une séquence génique codant pour un capteur de métabolites, qui code pour un capteur de métabolites qui reconnaît les métabolites du groupe acide aminé, acide organique, acide gras, vitamines ou principe actif végétal ;
ii) modification génétique des cellules selon l'étape i), par recombinaison avec addition d'un ADN qui contient au moins un gène G1 à Gn modifié ou au moins une mutation M1 à Mm, avec obtention d'une suspension cellulaire dans laquelle les cellules se distinguent pour ce qui est de la concentration intracellulaire du métabolite ;
iii) identification des cellules individuelles, dans la suspension cellulaire, présentant une concentration intracellulaire augmentée du métabolite, par détection par fluorescence à l'aide d'un capteur de métabolites pour des acides aminés, des acides organiques, des acides gras, des vitamines ou des principes actifs végétaux ;
iv) séparation des cellules identifiées d'avec la suspension cellulaire ;
v) identification d'au moins un gène G1 à Gn génétiquement modifié ou d'au moins une mutation M1 à Mm dans les cellules identifiées et séparées, qui est responsable de la concentration intracellulaire augmentée du métabolite ;
vi) fabrication d'une cellule de production, génétiquement modifiée par rapport à son type sauvage, avec production optimisée du métabolite, dont le génome comprend au moins l'un des gènes G1 à Gm et/ou au moins une mutation M1 à Mm.

6. Procédé selon l'une des revendications 4 à 5,
**caractérisé en ce que**
la modification génétique de la cellule selon l'étape ii) est mise en oeuvre grâce à une recombinase, qui incorpore dans l'ADN naturellement produit dans la cellule, se présentant sous forme d'un chromosome ou d'un plasmide, une ou plusieurs molécules d'ADN insérées dans la cellule, qui contient le gène modifié ou les gènes modifiés G1 à Gn et/ou la mutation ou les mutations M1 à Mm.

7. Procédé selon l'une des revendications 5 ou 6,
**caractérisé en ce que**
l'ADN est utilisé pour au moins un gène modifié G1 à Gn et/ou pour au moins une mutation M1 à Mm, qui codent pour l'une des étapes de la voie de biosynthèse du métabolite.

8. Procédé pour la fabrication de métabolites, contenant les étapes de procédé :
a) fabrication d'une cellule génétiquement modifiée par rapport à son type sauvage, avec production optimisée d'un certain métabolite, par un procédé selon les revendications 5 à 7,
b) culture de la cellule dans un milieu de culture contenant des nutriments, dans des conditions dans lesquelles la cellule produit à partir des nutriments le métabolite considéré.

9. Procédé selon la revendication 8,
**caractérisé en ce que**
le métabolite est un composant du groupe des acides aminés, des acides organiques, des acides gras, des vitamines ou des principes actifs végétaux.


### SEQUENCE LISTING

<110> Forschungszentrum Jülich GmbH
<120> verfahren zur Identifizierung einer zelle mit gegenüber ihrem wildtyp erhöhten intrazellulären Konzentration eines bestimmten Metaboliten, wobei die veränderung der Zelle durch Rekombineering erreicht wird, sowie ein verfahren zur Herstellung einer gegenüber ihrem wildtyp genetisch veränderten Produktionszelle mit optimierter Produktion eines bestimmten Metaboliten, ein verfahren zur Herstellung dieses Metaboliten, sowie dafür geeignete Nukleinsäuren.
<130> PT1.2597
<160> 44
<170> PatentIn version 3.5
<210> 1
   <211> 819
   <212> DNA
   <213> Corynebacterium aurimucosum
<400> 1
   ctaggcggct tcgccctcag cgggcttact atccaccact tccccatcca cgtgctgcgg      60
   atagtcaagg gcggattcgg ataggtctac acgcactgtt tcgtccgctg cgataccccg     120
   atctaggtcg gtagatgacg gcatccactt cgcgagctgg cgtacacagg tcttgtgggc     180
   catagcgtca aaattgtcag cccacgggcc aaacacttca ccttgcttgt tcttagcctt     240
   ggcgaattca tcacggtagg ccagcatttc gtccttactc atgacgatga agctgtgtcc     300
   cccggtggtg aacttagcta ccgcgtagta ggcgattggg ttgcccttcg gaccgttcat     360
   gcatggcttg tggacaagct tgtcatccag cccatagtca acgtcaaagt ggtcgttggc     420
   gtacacggtg cgggcgataa gcgactgaat ctgcccagag cggtgagcca gctcaactaa     480
   gccacggtaa ccaaccacaa gttgagccac catgccgccc tgcttcctat cccagaatgg     540
   cagcaggtaa gcatggccta gtacgccggg acgcaaacca agctgggagc acgtcatgag     600
   tccacccagt acggactgtg gggtgcattc tgcaagcttc ggggtttggc gcaggcaggt     660
   aagcgcatca cgtactagct gctgtgcctc catgcccttt ggcattgcga gctggaattg     720
   tgattccatg cttcggattt ggtctgccag ggtgacgggg cgattctgct tagccggggc     780
   gttgttagcg gccatacgct gctctagatt gtttcccat                            819
<210> 2
   <211> 272
   <212> PRT
   <213> Corynebacterium aurimucosum
<400> 2
   Met Gly Asn Asn Leu Glu Gln Arg Met Ala Ala Asn Asn Ala Pro Ala
   1               5                   10                  15
   Lys Gln Asn Arg Pro Val Thr Leu Ala Asp Gln Ile Arg Ser Met Glu
               20                  25                  30
   Ser Gln Phe Gln Leu Ala Met Pro Lys Gly Met Glu Ala Gln Gln Leu
           35                  40                  45
   Val Arg Asp Ala Leu Thr Cys Leu Arg Gln Thr Pro Lys Leu Ala Glu
       50                  55                  60
   Cys Thr Pro Gln Ser Val Leu Gly Gly Leu Met Thr Cys Ser Gln Leu
   65                  70                  75                  80
   Gly Leu Arg Pro Gly Val Leu Gly His Ala Tyr Leu Leu Pro Phe Trp
                   85                  90                  95
   Asp Arg Lys Gln Gly Gly Met Val Ala Gln Leu Val Val Gly Tyr Arg
               100                 105                 110
   Gly Leu Val Glu Leu Ala His Arg Ser Gly Gln Ile Gln Ser Leu Ile
           115                 120                 125
   Ala Arg Thr Val Tyr Ala Asn Asp His Phe Asp Val Asp Tyr Gly Leu
       130                 135                 140
   Asp Asp Lys Leu Val His Lys Pro Cys Met Asn Gly Pro Lys Gly Asn
   145                 150                 155                 160
   Pro Ile Ala Tyr Tyr Ala Val Ala Lys Phe Thr Thr Gly Gly His Ser
                   165                 170                 175
   Phe Ile Val Met Ser Lys Asp Glu Met Leu Ala Tyr Arg Asp Glu Phe
               180                 185                 190
   Ala Lys Ala Lys Asn Lys Gln Gly Glu Val Phe Gly Pro Trp Ala Asp
           195                 200                 205
   Asn Phe Asp Ala Met Ala His Lys Thr Cys Val Arg Gln Leu Ala Lys
       210                 215                 220
   Trp Met Pro Ser Ser Thr Asp Leu Asp Arg Gly Ile Ala Ala Asp Glu
   225                 230                 235                 240
   Thr Val Arg Val Asp Leu Ser Glu Ser Ala Leu Asp Tyr Pro Gln His
                   245                 250                 255
   Val Asp Gly Glu Val Val Asp Ser Lys Pro Ala Glu Gly Glu Ala Ala
               260                 265                 270
<210> 3
   <211> 4100
   <212> DNA
   <213> Escherichia coli
<400> 3
   ggtcgactct agaggatccc caaggagata tagatatgag tactgcactc gcaacgctgg 60
   ctgggaagct ggctgaacgt gtcggcatgg attctgtcga cccacaggaa ctgatcacca 120
   ctcttcgcca gacggcattt aaaggtgatg ccagcgatgc gcagttcatc gcattactga 180
   tcgttgccaa ccagtacggc cttaatccgt ggacgaaaga aatttacgcc tttcctgata 240
   agcagaatgg catcgttccg gtggtgggcg ttgatggctg gtcccgcatc atcaatgaaa 300
   accagcagtt tgatggcatg gactttgagc aggacaatga atcctgtaca tgccggattt 360
   accgcaagga ccgtaatcat ccgatctgcg ttaccgaatg gatggatgaa tgccgccgcg 420
   aaccattcaa aactcgcgaa ggcagagaaa tcacggggcc gtggcagtcg catcccaaac 480
   ggatgttacg tcataaagcc atgattcagt gtgcccgtct ggccttcgga tttgctggta 540
   tctatgacaa ggatgaagcc gagcgcattg tcgaaaatac tgcatacact gcagaacgtc 600
   agccggaacg cgacatcact ccggttaacg atgaaaccat gcaggagatt aacactctgc 660
   tgatcgccct ggataaaaca tgggatgacg acttattgcc gctctgttcc cagatatttc 720
   gccgcgacat tcgtgcatcg tcagaactga cacaggccga agcagtaaaa gctcttggat 780
   tcctgaaaca gaaagccgca gagcagaagg tggcagcatg ataccgagct cgaattcact 840
   ggccgtcgtt ttacagccaa gcttggctgt tttggcggat gagagaagat tttcagcctg 900
   atacagatta aatcagaacg cagaagcggt ctgataaaac agaatttgcc tggcggcagt 960
   agcgcggtgg tcccacctga ccccatgccg aactcagaag tgaaacgccg tagcgccgat 1020
   ggtagtgtgg ggtctcccca tgcgagagta gggaactgcc aggcatcaaa taaaacgaaa 1080
   ggctcagtcg aaagactggg cctttcgttt tatctgttgt ttgtcggtga acgctctcct 1140
   gagtaggaca aatccgccgg gagcggattt gaacgttgcg aagcaacggc ccggagggtg 1200
   gcgggcagga cgcccgccat aaactgccag gcatcaaatt aagcagaagg ccatcctgac 1260
   ggatggcctt tttgcgtttc tacaaactct tttgtttatt tttctaaata cattcaaata 1320
   tgtatccgct catgagacaa taaccctgat aaatgcttca ataatattga aaaaggaaga 1380
   gtatgagtat tcaacatttc cgtgtcgccc ttattccctt ttttgcggca ttttgccttc 1440
   ctgtttttgc tcacccagaa acgctggtga aagtaaaaga tgctgaagat cagttgggtg 1500
   cacgagtggg ttacatcgaa ctggatctca acagcggtaa gatccttgag agttttcgcc 1560
   ccgaagaacg ttttccaatg atgagcactt ttaaagttct gctatgtggc gcggtattat 1620
   cccgtgttga cgccgggcaa gagcaactcg gtcgccgcat acactattct cagaatgact 1680
   tggttgagta attcgtaatc atgtcatagc tgtttcctgt gtgaaattgt tatccgctca 1740
   caattccaca caacatacga gccggaagca taaagtgtaa agcctggggt gcctaatgag 1800
   tgagctaact cacattaatt gcgttgcgct cactgcccgc tttccagtcg ggaaacctgt 1860
   cgtgccagct gcattaatga atcggccaac gcgcggggag aggcggtttg cgtattgggc 1920
   gctcttccgc ttcctcgctc actgactcgc tgcgctcggt cgttcggctg cggcgagcgg 1980
   tatcagctca ctcaaaggcg gtaatacggt tatccacaga atcaggggat aacgcaggaa 2040
   agaacatgtg agcaaaaggc cagcaaaagg ccaggaaccg taaaaaggcc gcgttgctgg 2100
   cgtttttcca taggctccgc ccccctgacg agcatcacaa aaatcgacgc tcaagtcaga    2160
   ggtggcgaaa cccgacagga ctataaagat accaggcgtt tccccctgga agctccctcg    2220
   tgcgctctcc tgttccgacc ctgccgctta ccggatacct gtccgccttt ctcccttcgg    2280
   gaagcgtggc gctttctcat agctcacgct gtaggtatct cagttcggtg taggtcgttc    2340
   gctccaagct gggctgtgtg cacgaacccc ccgttcagcc cgaccgctgc gccttatccg    2400
   gtaactatcg tcttgagtcc aacccggtaa gacacgactt atcgccactg gcagcagcca    2460
   ctggtaacag gattagcaga gcgaggtatg taggcggtgc tacagagttc ttgaagtggt    2520
   ggcctaacta cggctacact agaagaacag tatttggtat ctgcgctctg ctgaagccag    2580
   ttaccttcgg aaaaagagtt ggtagctctt gatccggcaa acaaaccacc gctggtagcg    2640
   gtggtttttt tgtttgcaag cagcagatta cgcgcagaaa aaaaggatct caagaagatc    2700
   ctttgatctt ttctacgggg tctgacgctc agtggaacga aaactcacgt taagggattt    2760
   tggtcatgag attatcaaaa aggatcttca cctagatcct tttggggggg gggggaaagc    2820
   cacgttgtgt ctcaaaatct ctgatgttac attgcacaag ataaaaatat atcatcatga    2880
   acaataaaac tgtctgctta cataaacagt aatacaaggg gtgttatgag ccatattcaa    2940
   cgggaaacgt cttgctcgag atctatcgat tttcgttcgt gaatacatgt tataataact    3000
   ataactaata acgtaacgtg actggcaaga gatattttta aaacaatgaa taggtttaca    3060
   cttactttag ttttatggaa atgaaagatc atatcatata taatctagaa taaaattaac    3120
   taaaataatt attatctaga taaaaaattt agaagccaat gaaatctata aataaactaa    3180
   attaagttta tttaattaac aactatggat ataaaatagg tactaatcaa aatagtgagg    3240
   aggatatatt tgaatacata cgaacaaatt aataaagtga aaaaaatact tcggaaacat    3300
   ttaaaaaata accttattgg tacttacatg tttggatcag gagttgagag tggactaaaa    3360
   ccaaatagtg atcttgactt tttagtcgtc gtatctgaac cattgacaga tcaaagtaaa    3420
   gaaatactta tacaaaaaat tagacctatt tcaaaaaaaa taggagataa aagcaactta    3480
   cgatatattg aattaacaat tattattcag caagaaatgg taccgtggaa tcatcctccc    3540
   aaacaagaat ttatttatgg agaatggtta caagagcttt atgaacaagg atacattcct    3600
   cagaaggaat taaattcaga tttaaccata atgctttacc aagcaaaacg aaaaaataaa    3660
   agaatatacg gaaattatga cttagaggaa ttactacctg atattccatt ttctgatgtg    3720
   agaagagcca ttatggattc gtcagaggaa ttaatagata attatcagga tgatgaaacc    3780
   aactctatat taactttatg ccgtatgatt ttaactatgg acacgggtaa aatcatacca    3840
   aaagatattg cgggaaatgc agtggctgaa tcttctccat tagaacatag ggagagaatt    3900
   ttgttagcag ttcgtagtta tcttggagag aatattgaat ggactaatga aaatgtaaat    3960
   ttaactataa actatttaaa taacagatta aaaaaattat aaaaaaattg aaaaaatggt    4020
   ggaaacactt ttttcaattt ttttgtttta ttatttaata tttgggaaat attcattcta    4080
   attggtaatc agattttaga                                                4100
<210> 4
   <211> 3700
   <212> DNA
   <213> Escherichia coli
<400> 4
   ggtcgactct agaggatccc caaggagata tacatatgac taagcaacca ccaatcgcaa      60
   aagccgatct gcaaaaaact cagggaaacc gtgcaccagc agcagttaaa aatagcgacg     120
   tgattagttt tattaaccag ccatcaatga aagagcaact ggcagcagct cttccacgcc     180
   atatgacggc tgaacgtatg atccgtatcg ccaccacaga aattcgtaaa gttccggcgt     240
   taggaaactg tgacactatg agttttgtca gtgcgatcgt acagtgttca cagctcggac     300
   ttgagccagg tagcgccctc ggtcatgcat atttactgcc ttttggtaat aaaaacgaaa     360
   agagcggtaa aaagaacgtt cagctaatca ttggctatcg cggcatgatt gatctggctc     420
   gccgttctgg tcaaatcgcc agcctgtcag cccgtgttgt ccgtgaaggt gacgagttta     480
   gcttcgaatt tggccttgat gaaaagttaa tacaccgccc gggagaaaac gaagatgccc     540
   cggttaccca cgtctatgct gtcgcaagac tgaaagacgg aggtactcag tttgaagtta     600
   tgacgcgcaa acagattgag ctggtgcgca gcctgagtaa agctggtaat aacgggccgt     660
   gggtaactca ctgggaagaa atggcaaaga aaacggctat tcgtcgcctg ttcaaatatt     720
   tgcccgtatc aattgagatc cagcgtgcag tatcaatgga tgaaaaggaa ccactgacaa     780
   tcgatcctgc agattcctct gtattaaccg gggaatacag tgtaatcgat aattcagagg     840
   aataagggta ccgagctcga attcactggc cgtcgtttta cagccaagct tggctgtttt     900
   ggcggatgag agaagatttt cagcctgata cagattaaat cagaacgcag aagcggtctg     960
   ataaaacaga atttgcctgg cggcagtagc gcggtggtcc cacctgaccc catgccgaac    1020
   tcagaagtga aacgccgtag cgccgatggt agtgtggggt ctccccatgc gagagtaggg    1080
   aactgccagg catcaaataa aacgaaaggc tcagtcgaaa gactgggcct ttcgttttat    1140
   ctgttgtttg tcggtgaacg ctctcctgag taggacaaat ccgccgggag cggatttgaa    1200
   cgttgcgaag caacggcccg gagggtggcg ggcaggacgc ccgccataaa ctgccaggca    1260
   tcaaattaag cagaaggcca tcctgacgga tggccttttt gcgtttctac aaactctttt    1320
   gtttattttt ctaaatacat tcaaatatgt atccgctcat gagacaataa ccctgataaa    1380
   tgcttcaata atattgaaaa aggaagagta tgagtattca acatttccgt gtcgccctta    1440
   ttcccttttt tgcggcattt tgccttcctg tttttgctca cccagaaacg ctggtgaaag    1500
   taaaagatgc tgaagatcag ttgggtgcac gagtgggtta catcgaactg gatctcaaca    1560
   gcggtaagat ccttgagagt tttcgccccg aagaacgttt tccaatgatg agcactttta    1620
   aagttctgct atgtggcgcg gtattatccc gtgttgacgc cgggcaagag caactcggtc    1680
   gccgcataca ctattctcag aatgacttgg ttgagtaatt cgtaatcatg tcatagctgt    1740
   ttcctgtgtg aaattgttat ccgctcacaa ttccacacaa catacgagcc ggaagcataa    1800
   agtgtaaagc ctggggtgcc taatgagtga gctaactcac attaattgcg ttgcgctcac 1860
   tgcccgcttt ccagtcggga aacctgtcgt gccagctgca ttaatgaatc ggccaacgcg 1920
   cggggagagg cggtttgcgt attgggcgct cttccgcttc ctcgctcact gactcgctgc 1980
   gctcggtcgt tcggctgcgg cgagcggtat cagctcactc aaaggcggta atacggttat 2040
   ccacagaatc aggggataac gcaggaaaga acatgtgagc aaaaggccag caaaaggcca 2100
   ggaaccgtaa aaaggccgcg ttgctggcgt ttttccatag gctccgcccc cctgacgagc 2160
   atcacaaaaa tcgacgctca agtcagaggt ggcgaaaccc gacaggacta taaagatacc 2220
   aggcgtttcc ccctggaagc tccctcgtgc gctctcctgt tccgaccctg ccgcttaccg 2280
   gatacctgtc cgcctttctc ccttcgggaa gcgtggcgct ttctcatagc tcacgctgta 2340
   ggtatctcag ttcggtgtag gtcgttcgct ccaagctggg ctgtgtgcac gaaccccccg 2400
   ttcagcccga ccgctgcgcc ttatccggta actatcgtct tgagtccaac ccggtaagac 2460
   acgacttatc gccactggca gcagccactg gtaacaggat tagcagagcg aggtatgtag 2520
   gcggtgctac agagttcttg aagtggtggc ctaactacgg ctacactaga agaacagtat 2580
   ttggtatctg cgctctgctg aagccagtta ccttcggaaa aagagttggt agctcttgat 2640
   ccggcaaaca aaccaccgct ggtagcggtg gtttttttgt ttgcaagcag cagattacgc 2700
   gcagaaaaaa aggatctcaa gaagatcctt tgatcttttc tacggggtct gacgctcagt 2760
   ggaacgaaaa ctcacgttaa gggattttgg tcatgagatt atcaaaaagg atcttcacct 2820
   agatcctttt gggggggggg ggaaagccac gttgtgtctc aaaatctctg atgttacatt 2880
   gcacaagata aaaatatatc atcatgaaca ataaaactgt ctgcttacat aaacagtaat 2940
   acaaggggtg ttatgagcca tattcaacgg gaaacgtctt gctcgagatc tatcgatttt 3000
   cgttcgtgaa tacatgttat aataactata actaataacg taacgtgact ggcaagagat 3060
   atttttaaaa caatgaatag gtttacactt actttagttt tatggaaatg aaagatcata 3120
   tcatatataa tctagaataa aattaactaa aataattatt atctagataa aaaatttaga 3180
   agccaatgaa atctataaat aaactaaatt aagtttattt aattaacaac tatggatata 3240
   aaataggtac taatcaaaat agtgaggagg atatatttga atacatacga acaaattaat 3300
   aaagtgaaaa aaatacttcg gaaacattta aaaaataacc ttattggtac ttacatgttt 3360
   ggatcaggag ttgagagtgg actaaaacca aatagtgatc ttgacttttt agtcgtcgta 3420
   tctgaaccat tgacagatca aagtaaagaa atacttatac aaaaaattag acctatttca 3480
   aaaaaaatag gagataaaag caacttacga tatattgaat taacaattat tattcagcaa 3540
   gaaatggtac cgtggaatca tcctcccaaa caagaattta tttatggaga atggttacaa 3600
   gagctttatg aacaaggata cattcctcag aaggaattaa attcagattt aaccataatg 3660
   ctttaccaag caaaacgaaa aaataaaaga atatacggaa 3700
<210> 5
   <211> 3650
   <212> DNA
   <213> Mycobakterium
<400> 5
   ggtcgactct agaggatccc gatctaagga gatatacata tgacctcctc ggagatcgac 60
   aagatcgatc cggacgcagg cactcttgag tggcttcctg cggcacaggc gccttccgca 120
   gtctcacgcg cgatgttgcg agaacacgct cagatgatgg cggacgctta ccaactcgcg 180
   aagggtatct gcggagatgg acggaatggc actggtctga tggttccaca gcgctttcgc 240
   ggtaaacccg aagaagcggc agctgcaatg atttacggct ctgagctcgg tctgaaccca 300
   cttcaggctg tgcaacgagt agtacccatc catggcatgc caaccctgga agctcggacc 360
   atggtgggac tgcttaaagc tcgcggttac aagatccgca ccgtcgaaca gtcggacact 420
   tccgtcacag tcgaaggcgt cgcaccagac ggcgaaacgg ctagctcaac ctggaccatc 480
   gaacgcgcac aacgcgccgg ctacgttcca accccatcat caccggactc caagcgtcga 540
   cccgacgtcg acgatgactg ggttaccgtg tcgaaaacat gggatggaaa ggtcaagaag 600
   tccgttgttg ggaacatgaa gtacatcacc gacccacagg cgatgctgaa agctaaagcc 660
   cagtctgagg tgtgtcgcga tctggcgccc gacgttctga tgggcatctc ctatacccgg 720
   gaggatctgg aatctgaacg ccaggatcag ttcgaggatc gtcgtcccgc agcagcccca 780
   gcacgcactc agcgtgtaac cgttgacgaa atcttcgccg aggaagtgcc gttgtcctcc 840
   gatggctttg ccggtgataa ccctccgacg gcagctcaag ctgcaccgga gcctgccccg 900
   gaacctgaca cgcaccaagc tcctgccggg gatgtgatcg aggaccaagg cgatggtgcg 960
   ccggatcccg gtgaaccttc cccagcggaa cagagcgaga cagttgtcga accagcccca 1020
   gagcccgccc ctgcaccgga taccgctgcc gctgctaagt ccacgtccaa gaaagtgaac 1080
   gctaaacgct ctaccgcgcc agcagctaac cctgacaaac caaagacccg catgcgtcaa 1140
   gctctggaga agcgcttgtt cgcactcttg ggcgatgccg gacttagcgg agcggccgac 1200
   cgcgacggtc ggattgccgt gtaccgttcc gtgctcgaaa acgaggcaat tggctccact 1260
   gacgatctgg atgaccctgc gattgggaag gtggcagatc agctctatgc atggcagcag 1320
   cagaatgaac tggatgacaa gattgccggt atcctcagcg atgctgcccg tgctgagtct 1380
   gagtcggtcg ccccagcagc agatccgact tctgagggca atgagtgaat tcgtaccgag 1440
   ctcgaattca ctggccgtcg ttttacagcc aagcttggct gttttggcgg atgagagaag 1500
   attttcagcc tgatacagat taaatcagaa cgcagaagcg gtctgataaa acagaatttg 1560
   cctggcggca gtagcgcggt ggtcccacct gaccccatgc cgaactcaga agtgaaacgc 1620
   cgtagcgccg atggtagtgt ggggtctccc catgcgagag tagggaactg ccaggcatca 1680
   aataaaacga aaggctcagt cgaaagactg ggcctttcgt tttatctgtt gtttgtcggt 1740
   gaacgctctc ctgagtagga caaatccgcc gggagcggat ttgaacgttg cgaagcaacg 1800
   gcccggaggg tggcgggcag gacgcccgcc ataaactgcc aggcatcaaa ttaagcagaa 1860
   ggccatcctg acggatggcc tttttgcgtt tctacaaact cttttgttta tttttctaaa 1920
   tacattcaaa tatgtatccg ctcatgagac aataaccctg ataaatgctt caataatatt 1980
   gaaaaaggaa gagtatgagt attcaacatt tccgtgtcgc ccttattccc ttttttgcgg 2040
   cattttgcct tcctgttttt gctcacccag aaacgctggt gaaagtaaaa gatgctgaag 2100
   atcagttggg tgcacgagtg ggttacatcg aactggatct caacagcggt aagatccttg 2160
   agagttttcg ccccgaagaa cgttttccaa tgatgagcac ttttaaagtt ctgctatgtg 2220
   gcgcggtatt atcccgtgtt gacgccgggc aagagcaact cggtcgccgc atacactatt 2280
   ctcagaatga cttggttgag taattcgtaa tcatgtcata gctgtttcct gtgtgaaatt 2340
   gttatccgct cacaattcca cacaacatac gagccggaag cataaagtgt aaagcctggg 2400
   gtgcctaatg agtgagctaa ctcacattaa ttgcgttgcg ctcactgccc gctttccagt 2460
   cgggaaacct gtcgtgccag ctgcattaat gaatcggcca acgcgcgggg agaggcggtt 2520
   tgcgtattgg gcgctcttcc gcttcctcgc tcactgactc gctgcgctcg gtcgttcggc 2580
   tgcggcgagc ggtatcagct cactcaaagg cggtaatacg gttatccaca gaatcagggg 2640
   ataacgcagg aaagaacatg tgagcaaaag gccagcaaaa ggccaggaac cgtaaaaagg 2700
   ccgcgttgct ggcgtttttc cataggctcc gcccccctga cgagcatcac aaaaatcgac 2760
   gctcaagtca gaggtggcga aacccgacag gactataaag ataccaggcg tttccccctg 2820
   gaagctccct cgtgcgctct cctgttccga ccctgccgct taccggatac ctgtccgcct 2880
   ttctcccttc gggaagcgtg gcgctttctc atagctcacg ctgtaggtat ctcagttcgg 2940
   tgtaggtcgt tcgctccaag ctgggctgtg tgcacgaacc ccccgttcag cccgaccgct 3000
   gcgccttatc cggtaactat cgtcttgagt ccaacccggt aagacacgac ttatcgccac 3060
   tggcagcagc cactggtaac aggattagca gagcgaggta tgtaggcggt gctacagagt 3120
   tcttgaagtg gtggcctaac tacggctaca ctagaagaac agtatttggt atctgcgctc 3180
   tgctgaagcc agttaccttc ggaaaaagag ttggtagctc ttgatccggc aaacaaacca 3240
   ccgctggtag cggtggtttt tttgtttgca agcagcagat tacgcgcaga aaaaaaggat 3300
   ctcaagaaga tcctttgatc ttttctacgg ggtctgacgc tcagtggaac gaaaactcac 3360
   gttaagggat tttggtcatg agattatcaa aaaggatctt cacctagatc cttttggggg 3420
   gggggggaaa gccacgttgt gtctcaaaat ctctgatgtt acattgcaca agataaaaat 3480
   atatcatcat gaacaataaa actgtctgct tacataaaca gtaatacaag gggtgttatg 3540
   agccatattc aacgggaaac gtcttgctcg agatctatcg attttcgttc gtgaatacat 3600
   gttataataa ctataactaa taacgtaacg tgactggcaa gagatatttt 3650
<210> 6
   <211> 3706
   <212> DNA
   <213> Mycobakterium
<400> 6
   taggagggtc gactctagag gatccccgat ctaggagata tacatatggc tgagaatgcg 60
   gtaacaaagc aggacagccc gaaagcaccg gaaaccatct ctcaggttct ccaagtcctg 120
   gttcctcagt tggctcgcgc agtgcctaaa ggcatggacc cagatcgcat tgcacgcatc 180
   gtacagaccg aaattcgcaa gtcacgcaac gcgaaagcgg caggcattgc caagcagtcc 240
   ttggacgatt gcacccaaga gagctttgcg ggagctctcc tcacttcggc cgcactcggt 300
   ttggaacctg gagtgaacgg cgaatgctac ctggtgccct atcgcgatac tcgacgcggc 360
   gtggtagagt gtcagctgat catcgggtat cagggtatcg tcaagctgtt ttggcaacat 420
   ccgcgcgcat cgcgcattga tgcgcagtgg gttggcgcaa acgatgaatt ccactacacc 480
   atgggcctga atcccacgct caaacacgtc aaggcaaagg gagatcgggg caatccagtc 540
   tacttctacg cgatcgtgga agttaccggt gccgagcctc tgtgggacgt tttcaccgcc 600
   gacgagatcc gtgaactgcg tcgtgggaag gttggttcct ccggcgatat caaggatcca 660
   cagcggtgga tggaacgtaa gactgccttg aaacaggtcc tgaagcttgc tcccaagacg 720
   acacgccttg acgctgctat ccgagctgat gaccgccctg gtaccgacct ctctcagtcc 780
   caagcacttg ctcttccctc cacggtgaaa ccgaccgctg actacatcga tggcgagatt 840
   gccgaaccac acgaagtcga taccccaccg aagtcatccc gtgcccaacg agcacaacgc 900
   gcaactgccc cagcaccaga cgttcagatg gcaaacccag accagctgaa acggcttggt 960
   gagattcaga aggcggagaa gtacaacgat gctgactggt tcaagtttct ggccgattct 1020
   gcgggagtga aggcaacccg tgctgccgat ctgaccttcg atgaggctaa agctgtcatc 1080
   gacatgttcg acggtcctaa cgcgtaacaa tttaccgagc tcgaattcac tggccgtcgt 1140
   tttacagcca agcttggctg ttttggcgga tgagagaaga ttttcagcct gatacagatt 1200
   aaatcagaac gcagaagcgg tctgataaaa cagaatttgc ctggcggcag tagcgcggtg 1260
   gtcccacctg accccatgcc gaactcagaa gtgaaacgcc gtagcgccga tggtagtgtg 1320
   gggtctcccc atgcgagagt agggaactgc caggcatcaa ataaaacgaa aggctcagtc 1380
   gaaagactgg gcctttcgtt ttatctgttg tttgtcggtg aacgctctcc tgagtaggac 1440
   aaatccgccg ggagcggatt tgaacgttgc gaagcaacgg cccggagggt ggcgggcagg 1500
   acgcccgcca taaactgcca ggcatcaaat taagcagaag gccatcctga cggatggcct 1560
   ttttgcgttt ctacaaactc ttttgtttat ttttctaaat acattcaaat atgtatccgc 1620
   tcatgagaca ataaccctga taaatgcttc aataatattg aaaaaggaag agtatgagta 1680
   ttcaacattt ccgtgtcgcc cttattccct tttttgcggc attttgcctt cctgtttttg 1740
   ctcacccaga aacgctggtg aaagtaaaag atgctgaaga tcagttgggt gcacgagtgg 1800
   gttacatcga actggatctc aacagcggta agatccttga gagttttcgc cccgaagaac 1860
   gttttccaat gatgagcact tttaaagttc tgctatgtgg cgcggtatta tcccgtgttg 1920
   acgccgggca agagcaactc ggtcgccgca tacactattc tcagaatgac ttggttgagt 1980
   aattcgtaat catgtcatag ctgtttcctg tgtgaaattg ttatccgctc acaattccac 2040
   acaacatacg agccggaagc ataaagtgta aagcctgggg tgcctaatga gtgagctaac 2100
   tcacattaat tgcgttgcgc tcactgcccg ctttccagtc gggaaacctg tcgtgccagc 2160
   tgcattaatg aatcggccaa cgcgcgggga gaggcggttt gcgtattggg cgctcttccg 2220
   cttcctcgct cactgactcg ctgcgctcgg tcgttcggct gcggcgagcg gtatcagctc 2280
   actcaaaggc ggtaatacgg ttatccacag aatcagggga taacgcagga aagaacatgt 2340
   gagcaaaagg ccagcaaaag gccaggaacc gtaaaaaggc cgcgttgctg gcgtttttcc 2400
   ataggctccg cccccctgac gagcatcaca aaaatcgacg ctcaagtcag aggtggcgaa 2460
   acccgacagg actataaaga taccaggcgt ttccccctgg aagctccctc gtgcgctctc 2520
   ctgttccgac cctgccgctt accggatacc tgtccgcctt tctcccttcg ggaagcgtgg 2580
   cgctttctca tagctcacgc tgtaggtatc tcagttcggt gtaggtcgtt cgctccaagc 2640
   tgggctgtgt gcacgaaccc cccgttcagc ccgaccgctg cgccttatcc ggtaactatc 2700
   gtcttgagtc caacccggta agacacgact tatcgccact ggcagcagcc actggtaaca 2760
   ggattagcag agcgaggtat gtaggcggtg ctacagagtt cttgaagtgg tggcctaact 2820
   acggctacac tagaagaaca gtatttggta tctgcgctct gctgaagcca gttaccttcg 2880
   gaaaaagagt tggtagctct tgatccggca aacaaaccac cgctggtagc ggtggttttt 2940
   ttgtttgcaa gcagcagatt acgcgcagaa aaaaaggatc tcaagaagat cctttgatct 3000
   tttctacggg gtctgacgct cagtggaacg aaaactcacg ttaagggatt ttggtcatga 3060
   gattatcaaa aaggatcttc acctagatcc ttttgggggg ggggggaaag ccacgttgtg 3120
   tctcaaaatc tctgatgtta cattgcacaa gataaaaata tatcatcatg aacaataaaa 3180
   ctgtctgctt acataaacag taatacaagg ggtgttatga gccatattca acgggaaacg 3240
   tcttgctcga gatctatcga ttttcgttcg tgaatacatg ttataataac tataactaat 3300
   aacgtaacgt gactggcaag agatattttt aaaacaatga ataggtttac acttacttta 3360
   gttttatgga aatgaaagat catatcatat ataatctaga ataaaattaa ctaaaataat 3420
   tattatctag ataaaaaatt tagaagccaa tgaaatctat aaataaacta aattaagttt 3480
   atttaattaa caactatgga tataaaatag gtactaatca aaatagtgag gaggatatat 3540
   ttgaatacat acgaacaaat taataaagtg aaaaaaatac ttcggaaaca tttaaaaaat 3600
   aaccttattg gtacttacat gtttggatca ggagttgaga gtggactaaa accaaatagt 3660
   gatcttgact ttttagtcgt cgtatctgaa ccattgacag atcaaa 3706
<210> 7
   <211> 3950
   <212> DNA
   <213> Corynebacterium aurimucosum
<400> 7
   ggtcgactct agaggatccc gatccaagga gatatacata tgggaaacaa tctagagcag      60
   cgtatggccg ctaacaacgc cccggctaag cagaatcgcc ccgtcaccct ggcagaccaa     120
   atccgaagca tggaatcaca attccagctc gcaatgccaa agggcatgga ggcacagcag     180
   ctagtacgtg atgcgcttac ctgcctgcgc caaaccccga agcttgcaga atgcacccca     240
   cagtccgtac tgggtggact catgacgtgc tcccagcttg gtttgcgtcc cggcgtacta     300
   ggccatgctt acctgctgcc attctgggat aggaagcagg gcggcatggt ggctcaactt 360
   gtggttggtt accgtggctt agttgagctg gctcaccgct ctgggcagat tcagtcgctt 420
   atcgcccgca ccgtgtacgc caacgaccac tttgacgttg actatgggct ggatgacaag 480
   cttgtccaca agccatgcat gaacggtccg aagggcaacc caatcgccta ctacgcggta 540
   gctaagttca ccaccggggg acacagcttc atcgtcatga gtaaggacga aatgctggcc 600
   taccgtgatg aattcgccaa ggctaagaac aagcaaggtg aagtgtttgg cccgtgggct 660
   gacaattttg acgctatggc ccacaagacc tgtgtacgcc agctcgcgaa gtggatgccg 720
   tcatctaccg acctagatcg gggtatcgca gcggacgaaa cagtgcgtgt agacctatcc 780
   gaatccgccc ttgactatcc gcagcacgtg gatggggaag tggtggatag taagcccgct 840
   gagggcgaag ccgcctagca attggtaccg agctcgaatt cactggccgt cgttttacag 900
   ccaagcttgg ctgttttggc ggatgagaga agattttcag cctgatacag attaaatcag 960
   aacgcagaag cggtctgata aaacagaatt tgcctggcgg cagtagcgcg gtggtcccac 1020
   ctgaccccat gccgaactca gaagtgaaac gccgtagcgc cgatggtagt gtggggtctc 1080
   cccatgcgag agtagggaac tgccaggcat caaataaaac gaaaggctca gtcgaaagac 1140
   tgggcctttc gttttatctg ttgtttgtcg gtgaacgctc tcctgagtag gacaaatccg 1200
   ccgggagcgg atttgaacgt tgcgaagcaa cggcccggag ggtggcgggc aggacgcccg 1260
   ccataaactg ccaggcatca aattaagcag aaggccatcc tgacggatgg cctttttgcg 1320
   tttctacaaa ctcttttgtt tatttttcta aatacattca aatatgtatc cgctcatgag 1380
   acaataaccc tgataaatgc ttcaataata ttgaaaaagg aagagtatga gtattcaaca 1440
   tttccgtgtc gcccttattc ccttttttgc ggcattttgc cttcctgttt ttgctcaccc 1500
   agaaacgctg gtgaaagtaa aagatgctga agatcagttg ggtgcacgag tgggttacat 1560
   cgaactggat ctcaacagcg gtaagatcct tgagagtttt cgccccgaag aacgttttcc 1620
   aatgatgagc acttttaaag ttctgctatg tggcgcggta ttatcccgtg ttgacgccgg 1680
   gcaagagcaa ctcggtcgcc gcatacacta ttctcagaat gacttggttg agtaattcgt 1740
   aatcatgtca tagctgtttc ctgtgtgaaa ttgttatccg ctcacaattc cacacaacat 1800
   acgagccgga agcataaagt gtaaagcctg gggtgcctaa tgagtgagct aactcacatt 1860
   aattgcgttg cgctcactgc ccgctttcca gtcgggaaac ctgtcgtgcc agctgcatta 1920
   atgaatcggc caacgcgcgg ggagaggcgg tttgcgtatt gggcgctctt ccgcttcctc 1980
   gctcactgac tcgctgcgct cggtcgttcg gctgcggcga gcggtatcag ctcactcaaa 2040
   ggcggtaata cggttatcca cagaatcagg ggataacgca ggaaagaaca tgtgagcaaa 2100
   aggccagcaa aaggccagga accgtaaaaa ggccgcgttg ctggcgtttt tccataggct 2160
   ccgcccccct gacgagcatc acaaaaatcg acgctcaagt cagaggtggc gaaacccgac 2220
   aggactataa agataccagg cgtttccccc tggaagctcc ctcgtgcgct ctcctgttcc 2280
   gaccctgccg cttaccggat acctgtccgc ctttctccct tcgggaagcg tggcgctttc 2340
   tcatagctca cgctgtaggt atctcagttc ggtgtaggtc gttcgctcca agctgggctg 2400
   tgtgcacgaa ccccccgttc agcccgaccg ctgcgcctta tccggtaact atcgtcttga 2460
   gtccaacccg gtaagacacg acttatcgcc actggcagca gccactggta acaggattag 2520
   cagagcgagg tatgtaggcg gtgctacaga gttcttgaag tggtggccta actacggcta 2580
   cactagaaga acagtatttg gtatctgcgc tctgctgaag ccagttacct tcggaaaaag 2640
   agttggtagc tcttgatccg gcaaacaaac caccgctggt agcggtggtt tttttgtttg 2700
   caagcagcag attacgcgca gaaaaaaagg atctcaagaa gatcctttga tcttttctac 2760
   ggggtctgac gctcagtgga acgaaaactc acgttaaggg attttggtca tgagattatc 2820
   aaaaaggatc ttcacctaga tccttttggg ggggggggga aagccacgtt gtgtctcaaa 2880
   atctctgatg ttacattgca caagataaaa atatatcatc atgaacaata aaactgtctg 2940
   cttacataaa cagtaataca aggggtgtta tgagccatat tcaacgggaa acgtcttgct 3000
   cgagatctat cgattttcgt tcgtgaatac atgttataat aactataact aataacgtaa 3060
   cgtgactggc aagagatatt tttaaaacaa tgaataggtt tacacttact ttagttttat 3120
   ggaaatgaaa gatcatatca tatataatct agaataaaat taactaaaat aattattatc 3180
   tagataaaaa atttagaagc caatgaaatc tataaataaa ctaaattaag tttatttaat 3240
   taacaactat ggatataaaa taggtactaa tcaaaatagt gaggaggata tatttgaata 3300
   catacgaaca aattaataaa gtgaaaaaaa tacttcggaa acatttaaaa aataacctta 3360
   ttggtactta catgtttgga tcaggagttg agagtggact aaaaccaaat agtgatcttg 3420
   actttttagt cgtcgtatct gaaccattga cagatcaaag taaagaaata cttatacaaa 3480
   aaattagacc tatttcaaaa aaaataggag ataaaagcaa cttacgatat attgaattaa 3540
   caattattat tcagcaagaa atggtaccgt ggaatcatcc tcccaaacaa gaatttattt 3600
   atggagaatg gttacaagag ctttatgaac aaggatacat tcctcagaag gaattaaatt 3660
   cagatttaac cataatgctt taccaagcaa aacgaaaaaa taaaagaata tacggaaatt 3720
   atgacttaga ggaattacta cctgatattc cattttctga tgtgagaaga gccattatgg 3780
   attcgtcaga ggaattaata gataattatc aggatgatga aaccaactct atattaactt 3840
   tatgccgtat gattttaact atggacacgg gtaaaatcat accaaaagat attgcgggaa 3900
   atgcagtggc tgaatcttct ccattagaac atagggagag aattttgtta 3950
<210> 8
   <211> 9137
   <212> DNA
   <213> Escherichia coli
<400> 8
   aagcttgcat gcctgcaggt cgactctaga ggatctaagg agatatacat atgactaagc 60
   aaccaccaat cgcaaaagcc gatctgcaaa aaactcaggg aaaccgtgca ccagcagcag 120
   ttaaaaatag cgacgtgatt agttttatta accagccatc aatgaaagag caactggcag 180
   cagctcttcc acgccatatg acggctgaac gtatgatccg tatcgccacc acagaaattc 240
   gtaaagttcc ggcgttagga aactgtgaca ctatgagttt tgtcagtgcg atcgtacagt 300
   gttcacagct cggacttgag ccaggtagcg ccctcggtca tgcatattta ctgccttttg 360
   gtaataaaaa cgaaaagagc ggtaaaaaga acgttcagct aatcattggc tatcgcggca 420
   tgattgatct ggctcgccgt tctggtcaaa tcgccagcct gtcagcccgt gttgtccgtg 480
   aaggtgacga gtttagcttc gaatttggcc ttgatgaaaa gttaatacac cgcccgggag 540
   aaaacgaaga tgccccggtt acccacgtct atgctgtcgc aagactgaaa gacggaggta 600
   ctcagtttga agttatgacg cgcaaacaga ttgagctggt gcgcagcctg agtaaagctg 660
   gtaataacgg gccgtgggta actcactggg aagaaatggc aaagaaaacg gctattcgtc 720
   gcctgttcaa atatttgccc gtatcaattg agatccagcg tgcagtatca atggatgaaa 780
   aggaaccact gacaatcgat cctgcagatt cctctgtatt aaccggggaa tacagtgtaa 840
   tcgataattc agaggaataa ttcagcctgg aattcactgg ccgtcgtttt acagccaagc 900
   ttggctgttt tggcggatga gagaagattt tcagcctgat acagattaaa tcagaacgca 960
   gaagcggtct gataaaacag aatttgcctg gcggcagtag cgcggtggtc ccacctgacc 1020
   ccatgccgaa ctcagaagtg aaacgccgta gcgccgatgg tagtgtgggg tctccccatg 1080
   cgagagtagg gaactgccag gcatcaaata aaacgaaagg ctcagtcgaa agactgggcc 1140
   tttcgtttta tctgttgttt gtcggtgaac gctctcctga gtaggacaaa tccgccggga 1200
   gcggatttga acgttgcgaa gcaacggccc ggagggtggc gggcaggacg cccgccataa 1260
   actgccaggc atcaaattaa gcagaaggcc atcctgacgg atggcctttt tgcgtttcta 1320
   caaactcttt tgtttatttt tctaaataca ttcaaatatg tatccgctca tgagacaata 1380
   accctgataa atgcttcaat aatattgaaa aaggaagagt atgagtattc aacatttccg 1440
   tgtcgccctt attccctttt ttgcggcatt ttgccttcct gtttttgctc acccagaaac 1500
   gctggtgaaa gtaaaagatg ctgaagatca gttgggtgca cgagtgggtt acatcgaact 1560
   ggatctcaac agcggtaaga tccttgagag ttttcgcccc gaagaacgtt ttccaatgat 1620
   gagcactttt aaagttctgc tatgtggcgc ggtattatcc cgtgttgacg ccgggcaaga 1680
   gcaactcggt cgccgcatac actattctca gaatgacttg gttgagtaat tcgtaatcat 1740
   gtcatagctg tttcctgtgt gaaattgtta tccgctcaca attccacaca acatacgagc 1800
   cggaagcata aagtgtaaag cctggggtgc ctaatgagtg agctaactca cattaattgc 1860
   gttgcgctca ctgcccgctt tccagtcggg aaacctgtcg tgccagctgc attaatgaat 1920
   cggccaacgc gcggggagag gcggtttgcg tattgggcgc tcttccgctt cctcgctcac 1980
   tgactcgctg cgctcggtcg ttcggctgcg gcgagcggta tcagctcact caaaggcggt 2040
   aatacggtta tccacagaat caggggataa cgcaggaaag aacatgtgag caaaaggcca 2100
   gcaaaaggcc aggaaccgta aaaaggccgc gttgctggcg tttttccata ggctccgccc 2160
   ccctgacgag catcacaaaa atcgacgctc aagtcagagg tggcgaaacc cgacaggact 2220
   ataaagatac caggcgtttc cccctggaag ctccctcgtg cgctctcctg ttccgaccct 2280
   gccgcttacc ggatacctgt ccgcctttct cccttcggga agcgtggcgc tttctcatag 2340
   ctcacgctgt aggtatctca gttcggtgta ggtcgttcgc tccaagctgg gctgtgtgca 2400
   cgaacccccc gttcagcccg accgctgcgc cttatccggt aactatcgtc ttgagtccaa 2460
   cccggtaaga cacgacttat cgccactggc agcagccact ggtaacagga ttagcagagc 2520
   gaggtatgta ggcggtgcta cagagttctt gaagtggtgg cctaactacg gctacactag 2580
   aagaacagta tttggtatct gcgctctgct gaagccagtt accttcggaa aaagagttgg 2640
   tagctcttga tccggcaaac aaaccaccgc tggtagcggt ggtttttttg tttgcaagca 2700
   gcagattacg cgcagaaaaa aaggatctca agaagatcct ttgatctttt ctacggggtc 2760
   tgacgctcag tggaacgaaa actcacgtta agggattttg gtcatgagat tatcaaaaag 2820
   gatcttcacc tagatccttt tggggggggg gggaaagcca cgttgtgtct caaaatctct 2880
   gatgttacat tgcacaagat aaaaatatat catcatgaac aataaaactg tctgcttaca 2940
   taaacagtaa tacaaggggt gttatgagcc atattcaacg ggaaacgtct tgctcgagat 3000
   ctatcgattt tcgttcgtga atacatgtta taataactat aactaataac gtaacgtgac 3060
   tggcaagaga tatttttaaa acaatgaata ggtttacact tactttagtt ttatggaaat 3120
   gaaagatcat atcatatata atctagaata aaattaacta aaataattat tatctagata 3180
   aaaaatttag aagccaatga aatctataaa taaactaaat taagtttatt taattaacaa 3240
   ctatggatat aaaataggta ctaatcaaaa tagtgaggag gatatatttg aatacatacg 3300
   aacaaattaa taaagtgaaa aaaatacttc ggaaacattt aaaaaataac cttattggta 3360
   cttacatgtt tggatcagga gttgagagtg gactaaaacc aaatagtgat cttgactttt 3420
   tagtcgtcgt atctgaacca ttgacagatc aaagtaaaga aatacttata caaaaaatta 3480
   gacctatttc aaaaaaaata ggagataaaa gcaacttacg atatattgaa ttaacaatta 3540
   ttattcagca agaaatggta ccgtggaatc atcctcccaa acaagaattt atttatggag 3600
   aatggttaca agagctttat gaacaaggat acattcctca gaaggaatta aattcagatt 3660
   taaccataat gctttaccaa gcaaaacgaa aaaataaaag aatatacgga aattatgact 3720
   tagaggaatt actacctgat attccatttt ctgatgtgag aagagccatt atggattcgt 3780
   cagaggaatt aatagataat tatcaggatg atgaaaccaa ctctatatta actttatgcc 3840
   gtatgatttt aactatggac acgggtaaaa tcataccaaa agatattgcg ggaaatgcag 3900
   tggctgaatc ttctccatta gaacataggg agagaatttt gttagcagtt cgtagttatc 3960
   ttggagagaa tattgaatgg actaatgaaa atgtaaattt aactataaac tatttaaata 4020
   acagattaaa aaaattataa aaaaattgaa aaaatggtgg aaacactttt ttcaattttt 4080
   ttgttttatt atttaatatt tgggaaatat tcattctaat tggtaatcag attttagaaa 4140
   acaataaacc cttgcatatg atatcgatgt acagatccct ggtatgagtc agcaacacct 4200
   tcttcacgag gcagacctca gcgccccccc ccccctagct tgtctacgtc tgatgctttg 4260
   aatcggacgg acttgccgat cttgtatgcg gtgatttttc cctcgtttgc ccacttttta     4320
   atggtggccg gggtgagagc tacgcgggcg gcgacctgct gcgctgtgat ccaatattcg     4380
   gggtcgttca ctggttcccc tttctgattt ctggcataga agaacccccg tgaactgtgt     4440
   ggttccgggg gttgctgatt tttgcgagac ttctcgcgca attccctagc ttaggtgaaa     4500
   acaccatgaa acactaggga aacacccatg aaacacccat tagggcagta gggcggcttc     4560
   ttcgtctagg gcttgcattt gggcggtgat ctggtcttta gcgtgtgaaa gtgtgtcgta     4620
   ggtggcgtgc tcaatgcact cgaacgtcac gtcatttacc gggtcacggt gggcaaagag     4680
   aactagtggg ttagacattg ttttcctcgt tgtcggtggt ggtgagcttt tctagccgct     4740
   cggtaaacgc ggcgatcatg aactcttgga ggttttcacc gttctgcatg cctgcgcgct     4800
   tcatgtcctc acgtagtgcc aaaggaacgc gtgcggtgac cacgacgggc ttagcctttg     4860
   cctgcgcttc tagtgcttcg atggtggctt gtgcctgcgc ttgctgcgcc tgtagtgcct     4920
   gttgagcttc ttgtagttgc tgttctagct gtgccttggt tgccatgctt taagactcta     4980
   gtagctttcc tgcgatatgt catgcgcatg cgtagcaaac attgtcctgc aactcattca     5040
   ttatgtgcag tgctcctgtt actagtcgta catactcata tttacctagt ctgcatgcag     5100
   tgcatgcaca tgcagtcatg tcgtgctaat gtgtaaaaca tgtacatgca gattgctggg     5160
   ggtgcagggg gcggagccac cctgtccatg cggggtgtgg ggcttgcccc gccggtacag     5220
   acagtgagca ccggggcacc tagtcgcgga taccccccct aggtatcgga cacgtaaccc     5280
   tcccatgtcg atgcaaatct ttaacattga gtacgggtaa gctggcacgc atagccaagc     5340
   taggcggcca ccaaacacca ctaaaaatta atagttccta gacaagacaa acccccgtgc     5400
   gagctaccaa ctcatatgca cgggggccac ataacccgaa ggggtttcaa ttgacaacca     5460
   tagcactagc taagacaacg ggcacaacac ccgcacaaac tcgcactgcg caaccccgca     5520
   caacatcggg tctaggtaac actgaaatag aagtgaacac ctctaaggaa ccgcaggtca     5580
   atgagggttc taaggtcact cgcgctaggg cgtggcgtag gcaaaacgtc atgtacaaga     5640
   tcaccaatag taaggctctg gcggggtgcc ataggtggcg cagggacgaa gctgttgcgg     5700
   tgtcctggtc gtctaacggt gcttcgcagt ttgagggtct gcaaaactct cactctcgct     5760
   gggggtcacc tctggctgaa ttggaagtca tgggcgaacg ccgcattgag ctggctattg     5820
   ctactaagaa tcacttggcg gcgggtggcg cgctcatgat gtttgtgggc actgttcgac     5880
   acaaccgctc acagtcattt gcgcaggttg aagcgggtat taagactgcg tactcttcga     5940
   tggtgaaaac atctcagtgg aagaaagaac gtgcacggta cggggtggag cacacctata     6000
   gtgactatga ggtcacagac tcttgggcga acggttggca cttgcaccgc aacatgctgt     6060
   tgttcttgga tcgtccactg tctgacgatg aactcaaggc gtttgaggat tccatgtttt     6120
   cccgctggtc tgctggtgtg gttaaggccg gtatggacgc gccactgcgt gagcacgggg     6180
   tcaaacttga tcaggtgtct acctggggtg gagacgctgc gaaaatggca acctacctcg     6240
   ctaagggcat gtctcaggaa ctgactggct ccgctactaa aaccgcgtct aaggggtcgt     6300
   acacgccgtt tcagatgttg gatatgttgg ccgatcaaag cgacgccggc gaggatatgg 6360
   acgctgtttt ggtggctcgg tggcgtgagt atgaggttgg ttctaaaaac ctgcgttcgt 6420
   cctggtcacg tggggctaag cgtgctttgg gcattgatta catagacgct gatgtacgtc 6480
   gtgaaatgga agaagaactg tacaagctcg ccggtctgga agcaccggaa cgggtcgaat 6540
   caacccgcgt tgctgttgct ttggtgaagc ccgatgattg gaaactgatt cagtctgatt 6600
   tcgcggttag gcagtacgtt ctagattgcg tggataaggc taaggacgtg gccgctgcgc 6660
   aacgtgtcgc taatgaggtg ctggcaagtc tgggtgtgga ttccaccccg tgcatgatcg 6720
   ttatggatga tgtggacttg gacgcggttc tgcctactca tggggacgct actaagcgtg 6780
   atctgaatgc ggcggtgttc gcgggtaatg agcagactat tcttcgcacc cactaaaagc 6840
   ggcataaacc ccgttcgata ttttgtgcga tgaatttatg gtcaatgtcg cgggggcaaa 6900
   ctatgatggg tcttgttgtt gacaatggct gatttcatca ggaatggaac tgtcatgctg 6960
   ttatgtgcct ggctcctaat caaagctggg gacaatgggt tgccccgttg atctgatcta 7020
   gttcggattg gcggggcttc actgtatctg ggggtggcat cgtgaataga ttgcacaccg 7080
   tagtgggcag tgtgcacacc atagtggcca tgagcaccac cacccccagg gacgccgacg 7140
   gcgcgaagct ctgcgcctgg tgcggctcgg agatcaagca atccggcgtc ggccggagcc 7200
   gggactactg ccgccgctcc tgccgccagc gggcgtacga ggcccggcgc cagcgcgagg 7260
   cgatcgtgtc cgccgtggcg tcggcagtcg ctcgccgaga tacgtcacgt gacgaaatgc 7320
   agcagccttc cattccgtca cgtgacgaaa ctcgggccgc aggtcagagc acggttccgc 7380
   ccgctccggc cctgccggac ccccggcatc ccgcaagagg cccggcagta ccggcataac 7440
   caagcctatg cctacagcat ccagggtgac ggtgccgagg atgacgatga gcgcattgtt 7500
   agatttcata cacggtgcct gactgcgtta gcaatttaac tgtgataaac taccgcatta 7560
   aagcttatcg atgataagct gtcaaacatg gcctgtcgct tgcggtattc ggaatcttgc 7620
   acgccctcgc tcaagccttc gtcactggtc ccgccaccaa acgtttcggc gagaagcagg 7680
   ccattatcgc cggcatggcg gccgacgcgc ggggagaggc ggtttgcgta ttgggcgcca 7740
   gggtggtttt tcttttcacc agtgagacgg gcaacagctg attgcccttc accgcctggc 7800
   cctgagagag ttgcagcaag cggtccacgc tggtttgccc cagcaggcga aaatcctgtt 7860
   tgatggtggt taacggcggg atataacatg agctgtcttc ggtatcgtcg tatcccacta 7920
   ccgagatatc cgcaccaacg cgcagcccgg actcggtaat ggcgcgcatt gcgcccagcg 7980
   ccatctgatc gttggcaacc agcatcgcag tgggaacgat gccctcattc agcatttgca 8040
   tggtttgttg aaaaccggac atggcactcc agtcgccttc ccgttccgct atcggctgaa 8100
   tttgattgcg agtgagatat ttatgccagc cagccagacg cagacgcgcc gagacagaac 8160
   ttaatgggcc cgctaacagc gcgatttgct ggtgacccaa tgcgaccaga tgctccacgc 8220
   ccagtcgcgt accgtcttca tgggagaaaa taatactgtt gatgggtgtc tggtcagaga 8280
   catcaagaaa taacgccgga acattagtgc aggcagcttc cacagcaatg gcatcctggt 8340
   catccagcgg atagttaatg atcagcccac tgacgcgttg cgcgagaaga ttgtgcaccg 8400
   ccgctttaca ggcttcgacg ccgcttcgtt ctaccatcga caccaccacg ctggcaccca 8460
   gttgatcggc gcgagattta atcgccgcga caatttgcga cggcgcgtgc agggccagac 8520
   tggaggtggc aacgccaatc agcaacgact gtttgcccgc cagttgttgt gccacgcggt 8580
   tgggaatgta attcagctcc gccatcgccg cttccacttt ttcccgcgtt ttcgcagaaa 8640
   cgtggctggc ctggttcacc acgcgggaaa cggtctgata agagacaccg gcatactctg 8700
   cgacatcgta taacgttact ggtttcacat tcaccaccct gaattgactc tcttccgggc 8760
   gctatcatgc cataccgcga aaggttttgc accattcgat ggtgtcaacg taaatgcatg 8820
   ccgcttcgcc ttcgcgcgcg aattgcaagc tgatccgggc ttatcgactg cacggtgcac 8880
   caatgcttct ggcgtcaggc agccatcgga agctgtggta tggctgtgca ggtcgtaaat 8940
   cactgcataa ttcgtgtcgc tcaaggcgca ctcccgttct ggataatgtt ttttgcgccg 9000
   acatcataac ggttctggca aatattctga aatgagctgt tgacaattaa tcatcggctc 9060
   gtataatgtg tggaattgtg agcggataac aatttcacac aggaaacaga attaaaagat 9120
   atgaccatga ttacgcc 9137
<210> 9
   <211> 9100
   <212> DNA
   <213> Escherichia coli
<400> 9
   aagcttgcat gcctgcaggt cgactctaga ggatctaagg agatatacat atgagtactg     60
   cactcgcaac gctggctggg aagctggctg aacgtgtcgg catggattct gtcgacccac    120
   aggaactgat caccactctt cgccagacgg catttaaagg tgatgccagc gatgcgcagt    180
   tcatcgcatt actgatcgtt gccaaccagt acggccttaa tccgtggacg aaagaaattt    240
   acgcctttcc tgataagcag aatggcatcg ttccggtggt gggcgttgat ggctggtccc    300
   gcatcatcaa tgaaaaccag cagtttgatg gcatggactt tgagcaggac aatgaatcct    360
   gtacatgccg gatttaccgc aaggaccgta atcatccgat ctgcgttacc gaatggatgg    420
   atgaatgccg ccgcgaacca ttcaaaactc gcgaaggcag agaaatcacg gggccgtggc    480
   agtcgcatcc caaacggatg ttacgtcata aagccatgat tcagtgtgcc cgtctggcct    540
   tcggatttgc tggtatctat gacaaggatg aagccgagcg cattgtcgaa aatactgcat    600
   acactgcaga acgtcagccg gaacgcgaca tcactccggt taacgatgaa accatgcagg    660
   agattaacac tctgctgatc gccctggata aaacatggga tgacgactta ttgccgctct    720
   gttcccagat atttcgccgc gacattcgtg catcgtcaga actgacacag gccgaagcag    780
   taaaagctct tggattcctg aaacagaaag ccgcagagca gaaggtggca gcatgaattc    840
   actggccgtc gttttacagc caagcttggc tgttttggcg gatgagagaa gattttcagc    900
   ctgatacaga ttaaatcaga acgcagaagc ggtctgataa aacagaattt gcctggcggc    960
   agtagcgcgg tggtcccacc tgaccccatg ccgaactcag aagtgaaacg ccgtagcgcc   1020
   gatggtagtg tggggtctcc ccatgcgaga gtagggaact gccaggcatc aaataaaacg 1080
   aaaggctcag tcgaaagact gggcctttcg ttttatctgt tgtttgtcgg tgaacgctct 1140
   cctgagtagg acaaatccgc cgggagcgga tttgaacgtt gcgaagcaac ggcccggagg 1200
   gtggcgggca ggacgcccgc cataaactgc caggcatcaa attaagcaga aggccatcct 1260
   gacggatggc ctttttgcgt ttctacaaac tcttttgttt atttttctaa atacattcaa 1320
   atatgtatcc gctcatgaga caataaccct gataaatgct tcaataatat tgaaaaagga 1380
   agagtatgag tattcaacat ttccgtgtcg cccttattcc cttttttgcg gcattttgcc 1440
   ttcctgtttt tgctcaccca gaaacgctgg tgaaagtaaa agatgctgaa gatcagttgg 1500
   gtgcacgagt gggttacatc gaactggatc tcaacagcgg taagatcctt gagagttttc 1560
   gccccgaaga acgttttcca atgatgagca cttttaaagt tctgctatgt ggcgcggtat 1620
   tatcccgtgt tgacgccggg caagagcaac tcggtcgccg catacactat tctcagaatg 1680
   acttggttga gtaattcgta atcatgtcat agctgtttcc tgtgtgaaat tgttatccgc 1740
   tcacaattcc acacaacata cgagccggaa gcataaagtg taaagcctgg ggtgcctaat 1800
   gagtgagcta actcacatta attgcgttgc gctcactgcc cgctttccag tcgggaaacc 1860
   tgtcgtgcca gctgcattaa tgaatcggcc aacgcgcggg gagaggcggt ttgcgtattg 1920
   ggcgctcttc cgcttcctcg ctcactgact cgctgcgctc ggtcgttcgg ctgcggcgag 1980
   cggtatcagc tcactcaaag gcggtaatac ggttatccac agaatcaggg gataacgcag 2040
   gaaagaacat gtgagcaaaa ggccagcaaa aggccaggaa ccgtaaaaag gccgcgttgc 2100
   tggcgttttt ccataggctc cgcccccctg acgagcatca caaaaatcga cgctcaagtc 2160
   agaggtggcg aaacccgaca ggactataaa gataccaggc gtttccccct ggaagctccc 2220
   tcgtgcgctc tcctgttccg accctgccgc ttaccggata cctgtccgcc tttctccctt 2280
   cgggaagcgt ggcgctttct catagctcac gctgtaggta tctcagttcg gtgtaggtcg 2340
   ttcgctccaa gctgggctgt gtgcacgaac cccccgttca gcccgaccgc tgcgccttat 2400
   ccggtaacta tcgtcttgag tccaacccgg taagacacga cttatcgcca ctggcagcag 2460
   ccactggtaa caggattagc agagcgaggt atgtaggcgg tgctacagag ttcttgaagt 2520
   ggtggcctaa ctacggctac actagaagaa cagtatttgg tatctgcgct ctgctgaagc 2580
   cagttacctt cggaaaaaga gttggtagct cttgatccgg caaacaaacc accgctggta 2640
   gcggtggttt ttttgtttgc aagcagcaga ttacgcgcag aaaaaaagga tctcaagaag 2700
   atcctttgat cttttctacg gggtctgacg ctcagtggaa cgaaaactca cgttaaggga 2760
   ttttggtcat gagattatca aaaaggatct tcacctagat ccttttgggg ggggggggaa 2820
   agccacgttg tgtctcaaaa tctctgatgt tacattgcac aagataaaaa tatatcatca 2880
   tgaacaataa aactgtctgc ttacataaac agtaatacaa ggggtgttat gagccatatt 2940
   caacgggaaa cgtcttgctc gagatctatc gattttcgtt cgtgaataca tgttataata 3000
   actataacta ataacgtaac gtgactggca agagatattt ttaaaacaat gaataggttt 3060
   acacttactt tagttttatg gaaatgaaag atcatatcat atataatcta gaataaaatt 3120
   aactaaaata attattatct agataaaaaa tttagaagcc aatgaaatct ataaataaac 3180
   taaattaagt ttatttaatt aacaactatg gatataaaat aggtactaat caaaatagtg 3240
   aggaggatat atttgaatac atacgaacaa attaataaag tgaaaaaaat acttcggaaa 3300
   catttaaaaa ataaccttat tggtacttac atgtttggat caggagttga gagtggacta 3360
   aaaccaaata gtgatcttga ctttttagtc gtcgtatctg aaccattgac agatcaaagt 3420
   aaagaaatac ttatacaaaa aattagacct atttcaaaaa aaataggaga taaaagcaac 3480
   ttacgatata ttgaattaac aattattatt cagcaagaaa tggtaccgtg gaatcatcct 3540
   cccaaacaag aatttattta tggagaatgg ttacaagagc tttatgaaca aggatacatt 3600
   cctcagaagg aattaaattc agatttaacc ataatgcttt accaagcaaa acgaaaaaat 3660
   aaaagaatat acggaaatta tgacttagag gaattactac ctgatattcc attttctgat 3720
   gtgagaagag ccattatgga ttcgtcagag gaattaatag ataattatca ggatgatgaa 3780
   accaactcta tattaacttt atgccgtatg attttaacta tggacacggg taaaatcata 3840
   ccaaaagata ttgcgggaaa tgcagtggct gaatcttctc cattagaaca tagggagaga 3900
   attttgttag cagttcgtag ttatcttgga gagaatattg aatggactaa tgaaaatgta 3960
   aatttaacta taaactattt aaataacaga ttaaaaaaat tataaaaaaa ttgaaaaaat 4020
   ggtggaaaca cttttttcaa tttttttgtt ttattattta atatttggga aatattcatt 4080
   ctaattggta atcagatttt agaaaacaat aaacccttgc atatgatatc gatgtacaga 4140
   tccctggtat gagtcagcaa caccttcttc acgaggcaga cctcagcgcc cccccccccc 4200
   tagcttgtct acgtctgatg ctttgaatcg gacggacttg ccgatcttgt atgcggtgat 4260
   ttttccctcg tttgcccact ttttaatggt ggccggggtg agagctacgc gggcggcgac 4320
   ctgctgcgct gtgatccaat attcggggtc gttcactggt tcccctttct gatttctggc 4380
   atagaagaac ccccgtgaac tgtgtggttc cgggggttgc tgatttttgc gagacttctc 4440
   gcgcaattcc ctagcttagg tgaaaacacc atgaaacact agggaaacac ccatgaaaca 4500
   cccattaggg cagtagggcg gcttcttcgt ctagggcttg catttgggcg gtgatctggt 4560
   ctttagcgtg tgaaagtgtg tcgtaggtgg cgtgctcaat gcactcgaac gtcacgtcat 4620
   ttaccgggtc acggtgggca aagagaacta gtgggttaga cattgttttc ctcgttgtcg 4680
   gtggtggtga gcttttctag ccgctcggta aacgcggcga tcatgaactc ttggaggttt 4740
   tcaccgttct gcatgcctgc gcgcttcatg tcctcacgta gtgccaaagg aacgcgtgcg 4800
   gtgaccacga cgggcttagc ctttgcctgc gcttctagtg cttcgatggt ggcttgtgcc 4860
   tgcgcttgct gcgcctgtag tgcctgttga gcttcttgta gttgctgttc tagctgtgcc 4920
   ttggttgcca tgctttaaga ctctagtagc tttcctgcga tatgtcatgc gcatgcgtag 4980
   caaacattgt cctgcaactc attcattatg tgcagtgctc ctgttactag tcgtacatac 5040
   tcatatttac ctagtctgca tgcagtgcat gcacatgcag tcatgtcgtg ctaatgtgta 5100
   aaacatgtac atgcagattg ctgggggtgc agggggcgga gccaccctgt ccatgcgggg 5160
   tgtggggctt gccccgccgg tacagacagt gagcaccggg gcacctagtc gcggataccc 5220
   cccctaggta tcggacacgt aaccctccca tgtcgatgca aatctttaac attgagtacg 5280
   ggtaagctgg cacgcatagc caagctaggc ggccaccaaa caccactaaa aattaatagt 5340
   tcctagacaa gacaaacccc cgtgcgagct accaactcat atgcacgggg gccacataac 5400
   ccgaaggggt ttcaattgac aaccatagca ctagctaaga caacgggcac aacacccgca 5460
   caaactcgca ctgcgcaacc ccgcacaaca tcgggtctag gtaacactga aatagaagtg 5520
   aacacctcta aggaaccgca ggtcaatgag ggttctaagg tcactcgcgc tagggcgtgg 5580
   cgtaggcaaa acgtcatgta caagatcacc aatagtaagg ctctggcggg gtgccatagg 5640
   tggcgcaggg acgaagctgt tgcggtgtcc tggtcgtcta acggtgcttc gcagtttgag 5700
   ggtctgcaaa actctcactc tcgctggggg tcacctctgg ctgaattgga agtcatgggc 5760
   gaacgccgca ttgagctggc tattgctact aagaatcact tggcggcggg tggcgcgctc 5820
   atgatgtttg tgggcactgt tcgacacaac cgctcacagt catttgcgca ggttgaagcg 5880
   ggtattaaga ctgcgtactc ttcgatggtg aaaacatctc agtggaagaa agaacgtgca 5940
   cggtacgggg tggagcacac ctatagtgac tatgaggtca cagactcttg ggcgaacggt 6000
   tggcacttgc accgcaacat gctgttgttc ttggatcgtc cactgtctga cgatgaactc 6060
   aaggcgtttg aggattccat gttttcccgc tggtctgctg gtgtggttaa ggccggtatg 6120
   gacgcgccac tgcgtgagca cggggtcaaa cttgatcagg tgtctacctg gggtggagac 6180
   gctgcgaaaa tggcaaccta cctcgctaag ggcatgtctc aggaactgac tggctccgct 6240
   actaaaaccg cgtctaaggg gtcgtacacg ccgtttcaga tgttggatat gttggccgat 6300
   caaagcgacg ccggcgagga tatggacgct gttttggtgg ctcggtggcg tgagtatgag 6360
   gttggttcta aaaacctgcg ttcgtcctgg tcacgtgggg ctaagcgtgc tttgggcatt 6420
   gattacatag acgctgatgt acgtcgtgaa atggaagaag aactgtacaa gctcgccggt 6480
   ctggaagcac cggaacgggt cgaatcaacc cgcgttgctg ttgctttggt gaagcccgat 6540
   gattggaaac tgattcagtc tgatttcgcg gttaggcagt acgttctaga ttgcgtggat 6600
   aaggctaagg acgtggccgc tgcgcaacgt gtcgctaatg aggtgctggc aagtctgggt 6660
   gtggattcca ccccgtgcat gatcgttatg gatgatgtgg acttggacgc ggttctgcct 6720
   actcatgggg acgctactaa gcgtgatctg aatgcggcgg tgttcgcggg taatgagcag 6780
   actattcttc gcacccacta aaagcggcat aaaccccgtt cgatattttg tgcgatgaat 6840
   ttatggtcaa tgtcgcgggg gcaaactatg atgggtcttg ttgttgacaa tggctgattt 6900
   catcaggaat ggaactgtca tgctgttatg tgcctggctc ctaatcaaag ctggggacaa 6960
   tgggttgccc cgttgatctg atctagttcg gattggcggg gcttcactgt atctgggggt 7020
   ggcatcgtga atagattgca caccgtagtg ggcagtgtgc acaccatagt ggccatgagc 7080
   accaccaccc ccagggacgc cgacggcgcg aagctctgcg cctggtgcgg ctcggagatc 7140
   aagcaatccg gcgtcggccg gagccgggac tactgccgcc gctcctgccg ccagcgggcg 7200
   tacgaggccc ggcgccagcg cgaggcgatc gtgtccgccg tggcgtcggc agtcgctcgc 7260
   cgagatacgt cacgtgacga aatgcagcag ccttccattc cgtcacgtga cgaaactcgg 7320
   gccgcaggtc agagcacggt tccgcccgct ccggccctgc cggacccccg gcatcccgca 7380
   agaggcccgg cagtaccggc ataaccaagc ctatgcctac agcatccagg gtgacggtgc 7440
   cgaggatgac gatgagcgca ttgttagatt tcatacacgg tgcctgactg cgttagcaat 7500
   ttaactgtga taaactaccg cattaaagct tatcgatgat aagctgtcaa acatggcctg 7560
   tcgcttgcgg tattcggaat cttgcacgcc ctcgctcaag ccttcgtcac tggtcccgcc 7620
   accaaacgtt tcggcgagaa gcaggccatt atcgccggca tggcggccga cgcgcgggga 7680
   gaggcggttt gcgtattggg cgccagggtg gtttttcttt tcaccagtga gacgggcaac 7740
   agctgattgc ccttcaccgc ctggccctga gagagttgca gcaagcggtc cacgctggtt 7800
   tgccccagca ggcgaaaatc ctgtttgatg gtggttaacg gcgggatata acatgagctg 7860
   tcttcggtat cgtcgtatcc cactaccgag atatccgcac caacgcgcag cccggactcg 7920
   gtaatggcgc gcattgcgcc cagcgccatc tgatcgttgg caaccagcat cgcagtggga 7980
   acgatgccct cattcagcat ttgcatggtt tgttgaaaac cggacatggc actccagtcg 8040
   ccttcccgtt ccgctatcgg ctgaatttga ttgcgagtga gatatttatg ccagccagcc 8100
   agacgcagac gcgccgagac agaacttaat gggcccgcta acagcgcgat ttgctggtga 8160
   cccaatgcga ccagatgctc cacgcccagt cgcgtaccgt cttcatggga gaaaataata 8220
   ctgttgatgg gtgtctggtc agagacatca agaaataacg ccggaacatt agtgcaggca 8280
   gcttccacag caatggcatc ctggtcatcc agcggatagt taatgatcag cccactgacg 8340
   cgttgcgcga gaagattgtg caccgccgct ttacaggctt cgacgccgct tcgttctacc 8400
   atcgacacca ccacgctggc acccagttga tcggcgcgag atttaatcgc cgcgacaatt 8460
   tgcgacggcg cgtgcagggc cagactggag gtggcaacgc caatcagcaa cgactgtttg 8520
   cccgccagtt gttgtgccac gcggttggga atgtaattca gctccgccat cgccgcttcc 8580
   actttttccc gcgttttcgc agaaacgtgg ctggcctggt tcaccacgcg ggaaacggtc 8640
   tgataagaga caccggcata ctctgcgaca tcgtataacg ttactggttt cacattcacc 8700
   accctgaatt gactctcttc cgggcgctat catgccatac cgcgaaaggt tttgcaccat 8760
   tcgatggtgt caacgtaaat gcatgccgct tcgccttcgc gcgcgaattg caagctgatc 8820
   cgggcttatc gactgcacgg tgcaccaatg cttctggcgt caggcagcca tcggaagctg 8880
   tggtatggct gtgcaggtcg taaatcactg cataattcgt gtcgctcaag gcgcactccc 8940
   gttctggata atgttttttg cgccgacatc ataacggttc tggcaaatat tctgaaatga 9000
   gctgttgaca attaatcatc ggctcgtata atgtgtggaa ttgtgagcgg ataacaattt 9060
   cacacaggaa acagaattaa aagatatgac catgattacg 9100
<210> 10
   <211> 1429
   <212> DNA
   <213> Mycobakterium
<400> 10
   ctgcaggtcg accggcagat ctaaggagat atacatatga cctcctcgga gatcgacaag      60
   atcgatccgg acgcaggcac tcttgagtgg cttcctgcgg cacaggcgcc ttccgcagtc     120
   tcacgcgcga tgttgcgaga acacgctcag atgatggcgg acgcttacca actcgcgaag     180
   ggtatctgcg gagatggacg gaatggcact ggtctgatgg ttccacagcg ctttcgcggt     240
   aaacccgaag aagcggcagc tgcaatgatt tacggctctg agctcggtct gaacccactt     300
   caggctgtgc aacgagtagt acccatccat ggcatgccaa ccctggaagc tcggaccatg     360
   gtgggactgc ttaaagctcg cggttacaag atccgcaccg tcgaacagtc ggacacttcc     420
   gtcacagtcg aaggcgtcgc accagacggc gaaacggcta gctcaacctg gaccatcgaa     480
   cgcgcacaac gcgccggcta cgttccaacc ccatcatcac cggactccaa gcgtcgaccc     540
   gacgtcgacg atgactgggt taccgtgtcg aaaacatggg atggaaaggt caagaagtcc     600
   gttgttggga acatgaagta catcaccgac ccacaggcga tgctgaaagc taaagcccag     660
   tctgaggtgt gtcgcgatct ggcgcccgac gttctgatgg gcatctccta tacccgggag     720
   gatctggaat ctgaacgcca ggatcagttc gaggatcgtc gtcccgcagc agccccagca     780
   cgcactcagc gtgtaaccgt tgacgaaatc ttcgccgagg aagtgccgtt gtcctccgat     840
   ggctttgccg gtgataaccc tccgacggca gctcaagctg caccggagcc tgccccggaa     900
   cctgacacgc accaagctcc tgccggggat gtgatcgagg accaaggcga tggtgcgccg     960
   gatcccggtg aaccttcccc agcggaacag agcgagacag ttgtcgaacc agccccagag    1020
   cccgcccctg caccggatac cgctgccgct gctaagtcca cgtccaagaa agtgaacgct    1080
   aaacgctcta ccgcgccagc agctaaccct gacaaaccaa agacccgcat gcgtcaagct    1140
   ctggagaagc gcttgttcgc actcttgggc gatgccggac ttagcggagc ggccgaccgc    1200
   gacggtcgga ttgccgtgta ccgttccgtg ctcgaaaacg aggcaattgg ctccactgac    1260
   gatctggatg accctgcgat tgggaaggtg gcagatcagc tctatgcatg gcagcagcag    1320
   aatgaactgg atgacaagat tgccggtatc ctcagcgatg ctgcccgtgc tgagtctgag    1380
   tcggtcgccc cagcagcaga tccgacttct gagggcaatg agtgaattc                1429
<210> 11
   <211> 1093
   <212> DNA
   <213> Mycobakterium
<400> 11
   gcagatctaa ggagatatac atatggctga gaatgcggta acaaagcagg acagcccgaa 60
   agcaccggaa accatctctc aggttctcca agtcctggtt cctcagttgg ctcgcgcagt 120
   gcctaaaggc atggacccag atcgcattgc acgcatcgta cagaccgaaa ttcgcaagtc 180
   acgcaacgcg aaagcggcag gcattgccaa gcagtccttg gacgattgca cccaagagag 240
   ctttgcggga gctctcctca cttcggccgc actcggtttg gaacctggag tgaacggcga 300
   atgctacctg gtgccctatc gcgatactcg acgcggcgtg gtagagtgtc agctgatcat 360
   cgggtatcag ggtatcgtca agctgttttg gcaacatccg cgcgcatcgc gcattgatgc 420
   gcagtgggtt ggcgcaaacg atgaattcca ctacaccatg ggcctgaatc ccacgctcaa 480
   acacgtcaag gcaaagggag atcggggcaa tccagtctac ttctacgcga tcgtggaagt 540
   taccggtgcc gagcctctgt gggacgtttt caccgccgac gagatccgtg aactgcgtcg 600
   tgggaaggtt ggttcctccg gcgatatcaa ggatccacag cggtggatgg aacgtaagac 660
   tgccttgaaa caggtcctga agcttgctcc caagacgaca cgccttgacg ctgctatccg 720
   agctgatgac cgccctggta ccgacctctc tcagtcccaa gcacttgctc ttccctccac 780
   ggtgaaaccg accgctgact acatcgatgg cgagattgcc gaaccacacg aagtcgatac 840
   cccaccgaag tcatcccgtg cccaacgagc acaacgcgca actgccccag caccagacgt 900
   tcagatggca aacccagacc agctgaaacg gcttggtgag attcagaagg cggagaagta 960
   caacgatgct gactggttca agtttctggc cgattctgcg ggagtgaagg caacccgtgc 1020
   tgccgatctg accttcgatg aggctaaagc tgtcatcgac atgttcgacg gtcctaacgc 1080
   gtaacaattg ata 1093
<210> 12
   <211> 100
   <212> DNA
   <213> artificial
<220>
   <223> oligonukleotid
<400> 12
   aacaacgatg actgggcggg tctctcctgc ttcgttgagc acctcaagtc aagctgcgtc 60
   agtcaaaatg gccacagctt tcgcagcgtt atccgtacct 100
<210> 13
   <211> 100
   <212> DNA
   <213> artificial
<220>
   <223> oligonukleotid
<400> 13
   aacaacgatg actgggcggg tctctcctgc ttcgttgagc acctcaagtg cagcggcgtc 60
   agtcaaaatg gccacagctt tcgcagcgtt atccgtacct 100
<210> 14
   <211> 100
   <212> DNA
   <213> artificial
<220>
   <223> oligonukleotid
<400> 14
   aacaacgatg actgggcggg tctctcctgc ttcgttgagc acctcaaggc aagctgcgtc 60
   agtcaaaatg gccacagctt tcgcagcgtt atccgtacct 100
<210> 15
   <211> 100
   <212> DNA
   <213> artificial
<220>
   <223> oligonukleotid
<400> 15
   aacaacgatg actgggcggg tctctcctgc ttcgttgagc acctcaagtg cagcggcgtc 60
   agtcaaaatg gccacagctt tcgcagcgtt atccgtacct 100
<210> 16
   <211> 100
   <212> DNA
   <213> artificial
<220>
   <223> oligonukleotid
<400> 16
   aacaacgatg actgggcggg tctctcctgc ttcgttgagc acctcaagtt cagcggcgtc 60
   agtcaaaatg gccacagctt tcgcagcgtt atccgtacct 100
<210> 17
   <211> 100
   <212> DNA
   <213> artificial
<220>
   <223> oligonukleotid
<400> 17
   aacaacgatg actgggcggg tctctcctgc ttcgttgagc acctcaagga aagctgcgtc 60
   agtcaaaatg gccacagctt tcgcagcgtt atccgtacct 100
<210> 18
   <211> 100
   <212> DNA
   <213> artificial
<220>
   <223> oligonukleotid
<400> 18
   aacaacgatg actgggcggg tctctcctgc ttcgttgagc acctcaaggt gagctgcgtc 60
   agtcaaaatg gccacagctt tcgcagcgtt atccgtacct 100
<210> 19
   <211> 100
   <212> DNA
   <213> artificial
<220>
   <223> oligonukleotid
<400> 19
   aacaacgatg actgggcggg tctctcctgc ttcgttgagc acctcaagga tagctgcgtc 60
   agtcaaaatg gccacagctt tcgcagcgtt atccgtacct 100
<210> 20
   <211> 100
   <212> DNA
   <213> artificial
<220>
   <223> oligonukleotid
<400> 20
   aacaacgatg actgggcggg tctctcctgc ttcgttgagc acctcaagct tagctgcgtc 60
   agtcaaaatg gccacagctt tcgcagcgtt atccgtacct 100
<210> 21
   <211> 100
   <212> DNA
   <213> artificial
<220>
   <223> oligonukleotid
<400> 21
   aacaacgatg actgggcggg tctctcctgc ttcgttgagc acctcaagca gagctgcgtc 60
   agtcaaaatg gccacagctt tcgcagcgtt atccgtacct 100
<210> 22
   <211> 100
   <212> DNA
   <213> artificial
<220>
   <223> oligonukleotid
<400> 22
   aacaacgatg actgggcggg tctctcctgc ttcgttgagc acctcaagca tagctgcgtc 60
   agtcaaaatg gccacagctt tcgcagcgtt atccgtacct 100
<210> 23
   <211> 100
   <212> DNA
   <213> artificial
<220>
   <223> oligonukleotid
<400> 23
   aacaacgatg actgggcggg tctctcctgc ttcgttgagc acctcaaggt tagctgcgtc 60
   agtcaaaatg gccacagctt tcgcagcgtt atccgtacct 100
<210> 24
   <211> 100
   <212> DNA
   <213> artificial
<220>
   <223> oligonukleotid
<400> 24
   aacaacgatg actgggcggg tctctcctgc ttcgttgagc acctcaagtg gagctgcgtc 60
   agtcaaaatg gccacagctt tcgcagcgtt atccgtacct 100
<210> 25
   <211> 100
   <212> DNA
   <213> artificial
<220>
   <223> oligonukleotid
<400> 25
   aacaacgatg actgggcggg tctctcctgc ttcgttgagc acctcaagct gagctgcgtc 60
   agtcaaaatg gccacagctt tcgcagcgtt atccgtacct 100
<210> 26
   <211> 100
   <212> DNA
   <213> artificial
<220>
   <223> oligonukleotid
<400> 26
   aacaacgatg actgggcggg tctctcctgc ttcgttgagc acctcaaggc gagctgcgtc 60
   agtcaaaatg gccacagctt tcgcagcgtt atccgtacct 100
<210> 27
   <211> 100
   <212> DNA
   <213> artificial
<220>
   <223> oligonukleotid
<400> 27
   aacaacgatg actgggcggg tctctcctgc ttcgttgagc acctcaaggg aagctgcgtc 60
   agtcaaaatg gccacagctt tcgcagcgtt atccgtacct 100
<210> 28
   <211> 100
   <212> DNA
   <213> artificial
<220>
   <223> oligonukleotid
<400> 28
   aacaacgatg actgggcggg tctctcctgc ttcgttgagc acctcaaggg tagctgcgtc 60
   agtcaaaatg gccacagctt tcgcagcgtt atccgtacct 100
<210> 29
   <211> 100
   <212> DNA
   <213> artificial
<220>
   <223> oligonukleotid
<400> 29
   aacaacgatg actgggcggg tctctcctgc ttcgttgagc acctcaagca cagcggcgtc 60
   agtcaaaatg gccacagctt tcgcagcgtt atccgtacct 100
<210> 30
   <211> 100
   <212> DNA
   <213> artificial
<220>
   <223> oligonukleotid
<400> 30
   aacaacgatg actgggcggg tctctcctgc ttcgttgagc acctcaagcc aagctgcgtc 60
   agtcaaaatg gccacagctt tcgcagcgtt atccgtacct 100
<210> 31
   <211> 100
   <212> DNA
   <213> artificial
<220>
   <223> oligonukleotid
<400> 31
   aacaacgatg actgggcggg tctctcctgc ttcgttgagc acctcaaggt aagctgcgtc 60
   agtcaaaatg gccacagctt tcgcagcgtt atccgtacct 100
<210> 32
   <211> 100
   <212> DNA
   <213> artificial
<220>
   <223> oligonukleotid
<400> 32
   tcttcaagat ctccatcgcg cggcggccgt cggaacgagg gcaggtgaag atgatatcgg 60
   tggtgccgtc ttctacagaa gagacgttct gcagaaccat 100
<210> 33
   <211> 1566
   <212> DNA
   <213> Corynebacterium glutamicum
<400> 33
   atggcaacca cgttgctgga cctcaccaaa cttatcgatg gcatcctcaa gggctctgcc 60
   cagggcgttc ccgctcacgc agtaggggaa caagcaatcg cggctattgg tcttgactcc 120
   tccagcttac ctacctcgga cgctattttt gctgcagttc caggaacccg cactcacggc 180
   gcacagtttg caggtacgga taacgctgcg aaagctgtgg ccattttgac tgacgcagct 240
   tggcttgagg tgctcaacga agcaggagag acccgcccag tcatcgttgt tgatgatgtc 300
   cgcgcagtac ttggcgcagc atcatcaagc atttatggcg atccttcaaa agatttcacg 360
   ctcattggag tcactggaac ctcaggtaaa accaccacca gctacctctt ggaaaaagga 420
   ctcatggagg caggccacaa agttggtttg atcggcacca caggtacacg tattgacggg 480
   gaagaagtac ccacaaagct caccactcca gaagcgccga ctctgcaggc attgtttgct 540
   cgaatgcgcg atcacggtgt cacccacgtg gtgatggaag tatccagcca tgcattgtca 600
   ttgggcagag ttgcgggttc ccactttgat gtagctgcgt ttaccaacct gtcgcaggat 660
   caccttgatt tccaccccac catggatgat tactttgacg cgaaggcatt gttcttccgc 720
   gcagattctc cacttgtggc tgacaaacag gtcgtgtgcg tggatgattc ttggggtcag 780
   cgcatggcca gcgtggcagc ggatgtgcaa acagtatcca cccttgggca agaagcagac 840
   ttcagcgcta cagacatcaa tgtcagcgac tctggcgccc agagttttaa gatcaacgcc 900
   ccctcaaacc agtcctacca ggtcgagcta gctcttccag gtgcgttcaa cgttgctaac 960
   gccacgttgg catttgccgc tgcggcacgc gtgggtgttg atggcgaagc gtttgctcga 1020
   ggcatgtcca aggtcgcggt tccaggccgt atggaacgca ttgatgaggg acaagacttc 1080
   cttgcagtgg tggattatgc ccacaagcct gctgcagtgg ctgctgtgtt ggatacgttg 1140
   aggacccaga ttgacgggcg cctcggagtg gttatcggtg ctggtggaga ccgcgattcc 1200
   accaagcgtg gccccatggg gcagttgtcc gcacagcgtg ctgatctagt tattgtcact 1260
   gatgacaacc ctcgttcaga ggtgcctgcc acgattcgcg cagcagtcac tgcaggagca 1320
   cagcagggtg cttcagagtc cgaacgaccg gtggaagtcc tagaaattgg tgaccgtgca 1380
   gaagcaattc gcgttttggt cgagtgggca cagcctggag atggcattgt agtagctgga 1440
   aaaggccatg aagttggaca actagttgct ggtgtcaccc accattttga tgaccgcgaa 1500
   gaagttcgcg ctgctttgac agaaaagctc aacaataaac ttccccttac tacggaagaa 1560
   ggatag 1566
<210> 34
   <211> 1566
   <212> DNA
   <213> Corynebacterium glutamicum
<400> 34
   atggcaacca cgttgctgga cctcaccaaa cttatcgatg gcatcctcaa gggctctgcc      60
   cagggcgttc ccgctcacgc agtaggggaa caagcaatcg cggctattgg tcttgactcc     120
   tccagcttac ctacctcgga cgctattttt gctgcagttc caggaacccg cactcacggc     180
   gcacagtttg caggtacgga taacgctgcg aaagctgtgg ccattttgac tgacgcagct     240
   taccttgagg tgctcaacga agcaggagag acccgcccag tcatcgttgt tgatgatgtc     300
   cgcgcagtac ttggcgcagc atcatcaagc atttatggcg atccttcaaa agatttcacg     360
   ctcattggag tcactggaac ctcaggtaaa accaccacca gctacctctt ggaaaaagga     420
   ctcatggagg caggccacaa agttggtttg atcggcacca caggtacacg tattgacggg     480
   gaagaagtac ccacaaagct caccactcca gaagcgccga ctctgcaggc attgtttgct     540
   cgaatgcgcg atcacggtgt cacccacgtg gtgatggaag tatccagcca tgcattgtca     600
   ttgggcagag ttgcgggttc ccactttgat gtagctgcgt ttaccaacct gtcgcaggat     660
   caccttgatt tccaccccac catggatgat tactttgacg cgaaggcatt gttcttccgc     720
   gcagattctc cacttgtggc tgacaaacag gtcgtgtgcg tggatgattc ttggggtcag     780
   cgcatggcca gcgtggcagc ggatgtgcaa acagtatcca cccttgggca agaagcagac     840
   ttcagcgcta cagacatcaa tgtcagcgac tctggcgccc agagttttaa gatcaacgcc     900
   ccctcaaacc agtcctacca ggtcgagcta gctcttccag gtgcgttcaa cgttgctaac     960
   gccacgttgg catttgccgc tgcggcacgc gtgggtgttg atggcgaagc gtttgctcga    1020
   ggcatgtcca aggtcgcggt tccaggccgt atggaacgca ttgatgaggg acaagacttc    1080
   cttgcagtgg tggattatgc ccacaagcct gctgcagtgg ctgctgtgtt ggatacgttg    1140
   aggacccaga ttgacgggcg cctcggagtg gttatcggtg ctggtggaga ccgcgattcc    1200
   accaagcgtg gccccatggg gcagttgtcc gcacagcgtg ctgatctagt tattgtcact    1260
   gatgacaacc ctcgttcaga ggtgcctgcc acgattcgcg cagcagtcac tgcaggagca    1320
   cagcagggtg cttcagagtc cgaacgaccg gtggaagtcc tagaaattgg tgaccgtgca    1380
   gaagcaattc gcgttttggt cgagtgggca cagcctggag atggcattgt agtagctgga    1440
   aaaggccatg aagttggaca actagttgct ggtgtcaccc accattttga tgaccgcgaa    1500
   gaagttcgcg ctgctttgac agaaaagctc aacaataaac ttccccttac tacggaagaa    1560
   ggatag                                                               1566
<210> 35
   <211> 1566
   <212> DNA
   <213> Corynebacterium glutamicum
<400> 35
   atggcaacca cgttgctgga cctcaccaaa cttatcgatg gcatcctcaa gggctctgcc      60
   cagggcgttc ccgctcacgc agtaggggaa caagcaatcg cggctattgg tcttgactcc     120
   tccagcttac ctacctcgga cgctattttt gctgcagttc caggaacccg cactcacggc     180
   gcacagtttg caggtacgga taacgctgcg aaagctgtgg ccattttgac tgacgcagct     240
   aaccttgagg tgctcaacga agcaggagag acccgcccag tcatcgttgt tgatgatgtc     300
   cgcgcagtac ttggcgcagc atcatcaagc atttatggcg atccttcaaa agatttcacg     360
   ctcattggag tcactggaac ctcaggtaaa accaccacca gctacctctt ggaaaaagga     420
   ctcatggagg caggccacaa agttggtttg atcggcacca caggtacacg tattgacggg     480
   gaagaagtac ccacaaagct caccactcca gaagcgccga ctctgcaggc attgtttgct     540
   cgaatgcgcg atcacggtgt cacccacgtg gtgatggaag tatccagcca tgcattgtca     600
   ttgggcagag ttgcgggttc ccactttgat gtagctgcgt ttaccaacct gtcgcaggat     660
   caccttgatt tccaccccac catggatgat tactttgacg cgaaggcatt gttcttccgc     720
   gcagattctc cacttgtggc tgacaaacag gtcgtgtgcg tggatgattc ttggggtcag     780
   cgcatggcca gcgtggcagc ggatgtgcaa acagtatcca cccttgggca agaagcagac     840
   ttcagcgcta cagacatcaa tgtcagcgac tctggcgccc agagttttaa gatcaacgcc     900
   ccctcaaacc agtcctacca ggtcgagcta gctcttccag gtgcgttcaa cgttgctaac     960
   gccacgttgg catttgccgc tgcggcacgc gtgggtgttg atggcgaagc gtttgctcga    1020
   ggcatgtcca aggtcgcggt tccaggccgt atggaacgca ttgatgaggg acaagacttc    1080
   cttgcagtgg tggattatgc ccacaagcct gctgcagtgg ctgctgtgtt ggatacgttg    1140
   aggacccaga ttgacgggcg cctcggagtg gttatcggtg ctggtggaga ccgcgattcc    1200
   accaagcgtg gccccatggg gcagttgtcc gcacagcgtg ctgatctagt tattgtcact    1260
   gatgacaacc ctcgttcaga ggtgcctgcc acgattcgcg cagcagtcac tgcaggagca    1320
   cagcagggtg cttcagagtc cgaacgaccg gtggaagtcc tagaaattgg tgaccgtgca    1380
   gaagcaattc gcgttttggt cgagtgggca cagcctggag atggcattgt agtagctgga    1440
   aaaggccatg aagttggaca actagttgct ggtgtcaccc accattttga tgaccgcgaa    1500
   gaagttcgcg ctgctttgac agaaaagctc aacaataaac ttccccttac tacggaagaa    1560
   ggatag                                                               1566
<210> 36
   <211> 1566
   <212> DNA
   <213> Corynebacterium glutamicum
<400> 36
   atggcaacca cgttgctgga cctcaccaaa cttatcgatg gcatcctcaa gggctctgcc       60
   cagggcgttc ccgctcacgc agtaggggaa caagcaatcg cggctattgg tcttgactcc      120
   tccagcttac ctacctcgga cgctattttt gctgcagttc caggaacccg cactcacggc      180
   gcacagtttg caggtacgga taacgctgcg aaagctgtgg ccattttgac tgacgcagct      240
   tgccttgagg tgctcaacga agcaggagag acccgcccag tcatcgttgt tgatgatgtc      300
   cgcgcagtac ttggcgcagc atcatcaagc atttatggcg atccttcaaa agatttcacg      360
   ctcattggag tcactggaac ctcaggtaaa accaccacca gctacctctt ggaaaaagga      420
   ctcatggagg caggccacaa agttggtttg atcggcacca caggtacacg tattgacggg      480
   gaagaagtac ccacaaagct caccactcca gaagcgccga ctctgcaggc attgtttgct      540
   cgaatgcgcg atcacggtgt cacccacgtg gtgatggaag tatccagcca tgcattgtca      600
   ttgggcagag ttgcgggttc ccactttgat gtagctgcgt ttaccaacct gtcgcaggat      660
   caccttgatt tccaccccac catggatgat tactttgacg cgaaggcatt gttcttccgc      720
   gcagattctc cacttgtggc tgacaaacag gtcgtgtgcg tggatgattc ttggggtcag      780
   cgcatggcca gcgtggcagc ggatgtgcaa acagtatcca cccttgggca agaagcagac      840
   ttcagcgcta cagacatcaa tgtcagcgac tctggcgccc agagttttaa gatcaacgcc      900
   ccctcaaacc agtcctacca ggtcgagcta gctcttccag gtgcgttcaa cgttgctaac      960
   gccacgttgg catttgccgc tgcggcacgc gtgggtgttg atggcgaagc gtttgctcga     1020
   ggcatgtcca aggtcgcggt tccaggccgt atggaacgca ttgatgaggg acaagacttc     1080
   cttgcagtgg tggattatgc ccacaagcct gctgcagtgg ctgctgtgtt ggatacgttg     1140
   aggacccaga ttgacgggcg cctcggagtg gttatcggtg ctggtggaga ccgcgattcc     1200
   accaagcgtg gccccatggg gcagttgtcc gcacagcgtg ctgatctagt tattgtcact     1260
   gatgacaacc ctcgttcaga ggtgcctgcc acgattcgcg cagcagtcac tgcaggagca     1320
   cagcagggtg cttcagagtc cgaacgaccg gtggaagtcc tagaaattgg tgaccgtgca     1380
   gaagcaattc gcgttttggt cgagtgggca cagcctggag atggcattgt agtagctgga     1440
   aaaggccatg aagttggaca actagttgct ggtgtcaccc accattttga tgaccgcgaa     1500
   gaagttcgcg ctgctttgac agaaaagctc aacaataaac ttccccttac tacggaagaa     1560
   ggatag                                                                1566
<210> 37
   <211> 1566
   <212> DNA
   <213> corynebacterium glutamicum
<400> 37
   atggcaacca cgttgctgga cctcaccaaa cttatcgatg gcatcctcaa gggctctgcc 60
   cagggcgttc ccgctcacgc agtaggggaa caagcaatcg cggctattgg tcttgactcc 120
   tccagcttac ctacctcgga cgctattttt gctgcagttc caggaacccg cactcacggc 180
   gcacagtttg caggtacgga taacgctgcg aaagctgtgg ccattttgac tgacgcagct 240
   tcccttgagg tgctcaacga agcaggagag acccgcccag tcatcgttgt tgatgatgtc 300
   cgcgcagtac ttggcgcagc atcatcaagc atttatggcg atccttcaaa agatttcacg 360
   ctcattggag tcactggaac ctcaggtaaa accaccacca gctacctctt ggaaaaagga 420
   ctcatggagg caggccacaa agttggtttg atcggcacca caggtacacg tattgacggg 480
   gaagaagtac ccacaaagct caccactcca gaagcgccga ctctgcaggc attgtttgct 540
   cgaatgcgcg atcacggtgt cacccacgtg gtgatggaag tatccagcca tgcattgtca 600
   ttgggcagag ttgcgggttc ccactttgat gtagctgcgt ttaccaacct gtcgcaggat 660
   caccttgatt tccaccccac catggatgat tactttgacg cgaaggcatt gttcttccgc 720
   gcagattctc cacttgtggc tgacaaacag gtcgtgtgcg tggatgattc ttggggtcag 780
   cgcatggcca gcgtggcagc ggatgtgcaa acagtatcca cccttgggca agaagcagac 840
   ttcagcgcta cagacatcaa tgtcagcgac tctggcgccc agagttttaa gatcaacgcc 900
   ccctcaaacc agtcctacca ggtcgagcta gctcttccag gtgcgttcaa cgttgctaac 960
   gccacgttgg catttgccgc tgcggcacgc gtgggtgttg atggcgaagc gtttgctcga 1020
   ggcatgtcca aggtcgcggt tccaggccgt atggaacgca ttgatgaggg acaagacttc 1080
   cttgcagtgg tggattatgc ccacaagcct gctgcagtgg ctgctgtgtt ggatacgttg 1140
   aggacccaga ttgacgggcg cctcggagtg gttatcggtg ctggtggaga ccgcgattcc 1200
   accaagcgtg gccccatggg gcagttgtcc gcacagcgtg ctgatctagt tattgtcact 1260
   gatgacaacc ctcgttcaga ggtgcctgcc acgattcgcg cagcagtcac tgcaggagca 1320
   cagcagggtg cttcagagtc cgaacgaccg gtggaagtcc tagaaattgg tgaccgtgca 1380
   gaagcaattc gcgttttggt cgagtgggca cagcctggag atggcattgt agtagctgga 1440
   aaaggccatg aagttggaca actagttgct ggtgtcaccc accattttga tgaccgcgaa 1500
   gaagttcgcg ctgctttgac agaaaagctc aacaataaac ttccccttac tacggaagaa 1560
   ggatag 1566
<210> 38
   <211> 1566
   <212> DNA
   <213> Corynebacterium glutamicum
<400> 38
   atggcaacca cgttgctgga cctcaccaaa cttatcgatg gcatcctcaa gggctctgcc 60
   cagggcgttc ccgctcacgc agtaggggaa caagcaatcg cggctattgg tcttgactcc 120
   tccagcttac ctacctcgga cgctattttt gctgcagttc caggaacccg cactcacggc 180
   gcacagtttg caggtacgga taacgctgcg aaagctgtgg ccattttgac tgacgcagct 240
   ttccttgagg tgctcaacga agcaggagag acccgcccag tcatcgttgt tgatgatgtc 300
   cgcgcagtac ttggcgcagc atcatcaagc atttatggcg atccttcaaa agatttcacg 360
   ctcattggag tcactggaac ctcaggtaaa accaccacca gctacctctt ggaaaaagga 420
   ctcatggagg caggccacaa agttggtttg atcggcacca caggtacacg tattgacggg 480
   gaagaagtac ccacaaagct caccactcca gaagcgccga ctctgcaggc attgtttgct 540
   cgaatgcgcg atcacggtgt cacccacgtg gtgatggaag tatccagcca tgcattgtca 600
   ttgggcagag ttgcgggttc ccactttgat gtagctgcgt ttaccaacct gtcgcaggat 660
   caccttgatt tccaccccac catggatgat tactttgacg cgaaggcatt gttcttccgc 720
   gcagattctc cacttgtggc tgacaaacag gtcgtgtgcg tggatgattc ttggggtcag 780
   cgcatggcca gcgtggcagc ggatgtgcaa acagtatcca cccttgggca agaagcagac 840
   ttcagcgcta cagacatcaa tgtcagcgac tctggcgccc agagttttaa gatcaacgcc 900
   ccctcaaacc agtcctacca ggtcgagcta gctcttccag gtgcgttcaa cgttgctaac 960
   gccacgttgg catttgccgc tgcggcacgc gtgggtgttg atggcgaagc gtttgctcga 1020
   ggcatgtcca aggtcgcggt tccaggccgt atggaacgca ttgatgaggg acaagacttc 1080
   cttgcagtgg tggattatgc ccacaagcct gctgcagtgg ctgctgtgtt ggatacgttg 1140
   aggacccaga ttgacgggcg cctcggagtg gttatcggtg ctggtggaga ccgcgattcc 1200
   accaagcgtg gccccatggg gcagttgtcc gcacagcgtg ctgatctagt tattgtcact 1260
   gatgacaacc ctcgttcaga ggtgcctgcc acgattcgcg cagcagtcac tgcaggagca 1320
   cagcagggtg cttcagagtc cgaacgaccg gtggaagtcc tagaaattgg tgaccgtgca 1380
   gaagcaattc gcgttttggt cgagtgggca cagcctggag atggcattgt agtagctgga 1440
   aaaggccatg aagttggaca actagttgct ggtgtcaccc accattttga tgaccgcgaa 1500
   gaagttcgcg ctgctttgac agaaaagctc aacaataaac ttccccttac tacggaagaa 1560
   ggatag                                                            1566
<210> 39
   <211> 1566
   <212> DNA
   <213> Corynebacterium glutamicum
<400> 39
   atggcaacca cgttgctgga cctcaccaaa cttatcgatg gcatcctcaa gggctctgcc      60
   cagggcgttc ccgctcacgc agtaggggaa caagcaatcg cggctattgg tcttgactcc     120
   tccagcttac ctacctcgga cgctattttt gctgcagttc caggaacccg cactcacggc     180
   gcacagtttg caggtacgga taacgctgcg aaagctgtgg ccattttgac tgacgccgct     240
   gtgcttgagg tgctcaacga agcaggagag acccgcccag tcatcgttgt tgatgatgtc     300
   cgcgcagtac ttggcgcagc atcatcaagc atttatggcg atccttcaaa agatttcacg     360
   ctcattggag tcactggaac ctcaggtaaa accaccacca gctacctctt ggaaaaagga     420
   ctcatggagg caggccacaa agttggtttg atcggcacca caggtacacg tattgacggg     480
   gaagaagtac ccacaaagct caccactcca gaagcgccga ctctgcaggc attgtttgct     540
   cgaatgcgcg atcacggtgt cacccacgtg gtgatggaag tatccagcca tgcattgtca     600
   ttgggcagag ttgcgggttc ccactttgat gtagctgcgt ttaccaacct gtcgcaggat     660
   caccttgatt tccaccccac catggatgat tactttgacg cgaaggcatt gttcttccgc     720
   gcagattctc cacttgtggc tgacaaacag gtcgtgtgcg tggatgattc ttggggtcag     780
   cgcatggcca gcgtggcagc ggatgtgcaa acagtatcca cccttgggca agaagcagac     840
   ttcagcgcta cagacatcaa tgtcagcgac tctggcgccc agagttttaa gatcaacgcc     900
   ccctcaaacc agtcctacca ggtcgagcta gctcttccag gtgcgttcaa cgttgctaac     960
   gccacgttgg catttgccgc tgcggcacgc gtgggtgttg atggcgaagc gtttgctcga    1020
   ggcatgtcca aggtcgcggt tccaggccgt atggaacgca ttgatgaggg acaagacttc    1080
   cttgcagtgg tggattatgc ccacaagcct gctgcagtgg ctgctgtgtt ggatacgttg    1140
   aggacccaga ttgacgggcg cctcggagtg gttatcggtg ctggtggaga ccgcgattcc    1200
   accaagcgtg gccccatggg gcagttgtcc gcacagcgtg ctgatctagt tattgtcact    1260
   gatgacaacc ctcgttcaga ggtgcctgcc acgattcgcg cagcagtcac tgcaggagca    1320
   cagcagggtg cttcagagtc cgaacgaccg gtggaagtcc tagaaattgg tgaccgtgca    1380
   gaagcaattc gcgttttggt cgagtgggca cagcctggag atggcattgt agtagctgga    1440
   aaaggccatg aagttggaca actagttgct ggtgtcaccc accattttga tgaccgcgaa    1500
   gaagttcgcg ctgctttgac agaaaagctc aacaataaac ttccccttac tacggaagaa    1560
   ggatag                                                               1566
<210> 40
   <211> 1566
   <212> DNA
   <213> Corynebacterium glutamicum
<400> 40
   atggcaacca cgttgctgga cctcaccaaa cttatcgatg gcatcctcaa gggctctgcc 60
   cagggcgttc ccgctcacgc agtaggggaa caagcaatcg cggctattgg tcttgactcc 120
   tccagcttac ctacctcgga cgctattttt gctgcagttc caggaacccg cactcacggc 180
   gcacagtttg caggtacgga taacgctgcg aaagctgtgg ccattttgac tgacgcagct 240
   ctgcttgagg tgctcaacga agcaggagag acccgcccag tcatcgttgt tgatgatgtc 300
   cgcgcagtac ttggcgcagc atcatcaagc atttatggcg atccttcaaa agatttcacg 360
   ctcattggag tcactggaac ctcaggtaaa accaccacca gctacctctt ggaaaaagga 420
   ctcatggagg caggccacaa agttggtttg atcggcacca caggtacacg tattgacggg 480
   gaagaagtac ccacaaagct caccactcca gaagcgccga ctctgcaggc attgtttgct 540
   cgaatgcgcg atcacggtgt cacccacgtg gtgatggaag tatccagcca tgcattgtca 600
   ttgggcagag ttgcgggttc ccactttgat gtagctgcgt ttaccaacct gtcgcaggat 660
   caccttgatt tccaccccac catggatgat tactttgacg cgaaggcatt gttcttccgc 720
   gcagattctc cacttgtggc tgacaaacag gtcgtgtgcg tggatgattc ttggggtcag 780
   cgcatggcca gcgtggcagc ggatgtgcaa acagtatcca cccttgggca agaagcagac 840
   ttcagcgcta cagacatcaa tgtcagcgac tctggcgccc agagttttaa gatcaacgcc 900
   ccctcaaacc agtcctacca ggtcgagcta gctcttccag gtgcgttcaa cgttgctaac 960
   gccacgttgg catttgccgc tgcggcacgc gtgggtgttg atggcgaagc gtttgctcga 1020
   ggcatgtcca aggtcgcggt tccaggccgt atggaacgca ttgatgaggg acaagacttc 1080
   cttgcagtgg tggattatgc ccacaagcct gctgcagtgg ctgctgtgtt ggatacgttg 1140
   aggacccaga ttgacgggcg cctcggagtg gttatcggtg ctggtggaga ccgcgattcc 1200
   accaagcgtg gccccatggg gcagttgtcc gcacagcgtg ctgatctagt tattgtcact 1260
   gatgacaacc ctcgttcaga ggtgcctgcc acgattcgcg cagcagtcac tgcaggagca 1320
   cagcagggtg cttcagagtc cgaacgaccg gtggaagtcc tagaaattgg tgaccgtgca 1380
   gaagcaattc gcgttttggt cgagtgggca cagcctggag atggcattgt agtagctgga 1440
   aaaggccatg aagttggaca actagttgct ggtgtcaccc accattttga tgaccgcgaa 1500
   gaagttcgcg ctgctttgac agaaaagctc aacaataaac ttccccttac tacggaagaa 1560
   ggatag 1566
<210> 41
   <211> 1566
   <212> DNA
   <213> Corynebacterium glutamicum
<400> 41
   atggcaacca cgttgctgga cctcaccaaa cttatcgatg gcatcctcaa gggctctgcc      60
   cagggcgttc ccgctcacgc agtaggggaa caagcaatcg cggctattgg tcttgactcc     120
   tccagcttac ctacctcgga cgctattttt gctgcagttc caggaacccg cactcacggc     180
   gcacagtttg caggtacgga taacgctgcg aaagctgtgg ccattttgac tgacgcagct     240
   caccttgagg tgctcaacga agcaggagag acccgcccag tcatcgttgt tgatgatgtc     300
   cgcgcagtac ttggcgcagc atcatcaagc atttatggcg atccttcaaa agatttcacg     360
   ctcattggag tcactggaac ctcaggtaaa accaccacca gctacctctt ggaaaaagga     420
   ctcatggagg caggccacaa agttggtttg atcggcacca caggtacacg tattgacggg     480
   gaagaagtac ccacaaagct caccactcca gaagcgccga ctctgcaggc attgtttgct     540
   cgaatgcgcg atcacggtgt cacccacgtg gtgatggaag tatccagcca tgcattgtca     600
   ttgggcagag ttgcgggttc ccactttgat gtagctgcgt ttaccaacct gtcgcaggat     660
   caccttgatt tccaccccac catggatgat tactttgacg cgaaggcatt gttcttccgc     720
   gcagattctc cacttgtggc tgacaaacag gtcgtgtgcg tggatgattc ttggggtcag     780
   cgcatggcca gcgtggcagc ggatgtgcaa acagtatcca cccttgggca agaagcagac     840
   ttcagcgcta cagacatcaa tgtcagcgac tctggcgccc agagttttaa gatcaacgcc     900
   ccctcaaacc agtcctacca ggtcgagcta gctcttccag gtgcgttcaa cgttgctaac     960
   gccacgttgg catttgccgc tgcggcacgc gtgggtgttg atggcgaagc gtttgctcga    1020
   ggcatgtcca aggtcgcggt tccaggccgt atggaacgca ttgatgaggg acaagacttc    1080
   cttgcagtgg tggattatgc ccacaagcct gctgcagtgg ctgctgtgtt ggatacgttg    1140
   aggacccaga ttgacgggcg cctcggagtg gttatcggtg ctggtggaga ccgcgattcc    1200
   accaagcgtg gccccatggg gcagttgtcc gcacagcgtg ctgatctagt tattgtcact    1260
   gatgacaacc ctcgttcaga ggtgcctgcc acgattcgcg cagcagtcac tgcaggagca    1320
   cagcagggtg cttcagagtc cgaacgaccg gtggaagtcc tagaaattgg tgaccgtgca    1380
   gaagcaattc gcgttttggt cgagtgggca cagcctggag atggcattgt agtagctgga    1440
   aaaggccatg aagttggaca actagttgct ggtgtcaccc accattttga tgaccgcgaa    1500
   gaagttcgcg ctgctttgac agaaaagctc aacaataaac ttccccttac tacggaagaa    1560
   ggatag                                                               1566
<210> 42
   <211> 1566
   <212> DNA
   <213> Corynebacterium glutamicum
<400> 42
   atggcaacca cgttgctgga cctcaccaaa cttatcgatg gcatcctcaa gggctctgcc      60
   cagggcgttc ccgctcacgc agtaggggaa caagcaatcg cggctattgg tcttgactcc     120
   tccagcttac ctacctcgga cgctattttt gctgcagttc caggaacccg cactcacggc      180
   gcacagtttg caggtacgga taacgctgcg aaagctgtgg ccattttgac tgacgcagct      240
   atccttgagg tgctcaacga agcaggagag acccgcccag tcatcgttgt tgatgatgtc      300
   cgcgcagtac ttggcgcagc atcatcaagc atttatggcg atccttcaaa agatttcacg      360
   ctcattggag tcactggaac ctcaggtaaa accaccacca gctacctctt ggaaaaagga      420
   ctcatggagg caggccacaa agttggtttg atcggcacca caggtacacg tattgacggg      480
   gaagaagtac ccacaaagct caccactcca gaagcgccga ctctgcaggc attgtttgct      540
   cgaatgcgcg atcacggtgt cacccacgtg gtgatggaag tatccagcca tgcattgtca      600
   ttgggcagag ttgcgggttc ccactttgat gtagctgcgt ttaccaacct gtcgcaggat      660
   caccttgatt tccaccccac catggatgat tactttgacg cgaaggcatt gttcttccgc      720
   gcagattctc cacttgtggc tgacaaacag gtcgtgtgcg tggatgattc ttggggtcag      780
   cgcatggcca gcgtggcagc ggatgtgcaa acagtatcca cccttgggca agaagcagac      840
   ttcagcgcta cagacatcaa tgtcagcgac tctggcgccc agagttttaa gatcaacgcc      900
   ccctcaaacc agtcctacca ggtcgagcta gctcttccag gtgcgttcaa cgttgctaac      960
   gccacgttgg catttgccgc tgcggcacgc gtgggtgttg atggcgaagc gtttgctcga     1020
   ggcatgtcca aggtcgcggt tccaggccgt atggaacgca ttgatgaggg acaagacttc     1080
   cttgcagtgg tggattatgc ccacaagcct gctgcagtgg ctgctgtgtt ggatacgttg     1140
   aggacccaga ttgacgggcg cctcggagtg gttatcggtg ctggtggaga ccgcgattcc     1200
   accaagcgtg gccccatggg gcagttgtcc gcacagcgtg ctgatctagt tattgtcact     1260
   gatgacaacc ctcgttcaga ggtgcctgcc acgattcgcg cagcagtcac tgcaggagca     1320
   cagcagggtg cttcagagtc cgaacgaccg gtggaagtcc tagaaattgg tgaccgtgca     1380
   gaagcaattc gcgttttggt cgagtgggca cagcctggag atggcattgt agtagctgga     1440
   aaaggccatg aagttggaca actagttgct ggtgtcaccc accattttga tgaccgcgaa     1500
   gaagttcgcg ctgctttgac agaaaagctc aacaataaac ttccccttac tacggaagaa     1560
   ggatag                                                                1566
<210> 43
   <211> 1566
   <212> DNA
   <213> Corynebacterium glutamicum
<400> 43
   atggcaacca cgttgctgga cctcaccaaa cttatcgatg gcatcctcaa gggctctgcc       60
   cagggcgttc ccgctcacgc agtaggggaa caagcaatcg cggctattgg tcttgactcc      120
   tccagcttac ctacctcgga cgctattttt gctgcagttc caggaacccg cactcacggc      180
   gcacagtttg caggtacgga taacgctgcg aaagctgtgg ccattttgac tgacgcagct      240
   acccttgagg tgctcaacga agcaggagag acccgcccag tcatcgttgt tgatgatgtc      300
   cgcgcagtac ttggcgcagc atcatcaagc atttatggcg atccttcaaa agatttcacg        360
   ctcattggag tcactggaac ctcaggtaaa accaccacca gctacctctt ggaaaaagga        420
   ctcatggagg caggccacaa agttggtttg atcggcacca caggtacacg tattgacggg        480
   gaagaagtac ccacaaagct caccactcca gaagcgccga ctctgcaggc attgtttgct        540
   cgaatgcgcg atcacggtgt cacccacgtg gtgatggaag tatccagcca tgcattgtca        600
   ttgggcagag ttgcgggttc ccactttgat gtagctgcgt ttaccaacct gtcgcaggat        660
   caccttgatt tccaccccac catggatgat tactttgacg cgaaggcatt gttcttccgc        720
   gcagattctc cacttgtggc tgacaaacag gtcgtgtgcg tggatgattc ttggggtcag        780
   cgcatggcca gcgtggcagc ggatgtgcaa acagtatcca cccttgggca agaagcagac        840
   ttcagcgcta cagacatcaa tgtcagcgac tctggcgccc agagttttaa gatcaacgcc        900
   ccctcaaacc agtcctacca ggtcgagcta gctcttccag gtgcgttcaa cgttgctaac        960
   gccacgttgg catttgccgc tgcggcacgc gtgggtgttg atggcgaagc gtttgctcga       1020
   ggcatgtcca aggtcgcggt tccaggccgt atggaacgca ttgatgaggg acaagacttc       1080
   cttgcagtgg tggattatgc ccacaagcct gctgcagtgg ctgctgtgtt ggatacgttg       1140
   aggacccaga ttgacgggcg cctcggagtg gttatcggtg ctggtggaga ccgcgattcc       1200
   accaagcgtg gccccatggg gcagttgtcc gcacagcgtg ctgatctagt tattgtcact       1260
   gatgacaacc ctcgttcaga ggtgcctgcc acgattcgcg cagcagtcac tgcaggagca       1320
   cagcagggtg cttcagagtc cgaacgaccg gtggaagtcc tagaaattgg tgaccgtgca       1380
   gaagcaattc gcgttttggt cgagtgggca cagcctggag atggcattgt agtagctgga       1440
   aaaggccatg aagttggaca actagttgct ggtgtcaccc accattttga tgaccgcgaa       1500
   gaagttcgcg ctgctttgac agaaaagctc aacaataaac ttccccttac tacggaagaa       1560
   ggatag                                                                  1566
<210> 44
   <211> 1566
   <212> DNA
   <213> Corynebacterium glutamicum
<400> 44
   atggcaacca cgttgctgga cctcaccaaa cttatcgatg gcatcctcaa gggctctgcc      60
   cagggcgttc ccgctcacgc agtaggggaa caagcaatcg cggctattgg tcttgactcc     120
   tccagcttac ctacctcgga cgctattttt gctgcagttc caggaacccg cactcacggc     180
   gcacagtttg caggtacgga taacgctgcg aaagctgtgg ccattttgac tgacgcagct     240
   cgccttgagg tgctcaacga agcaggagag acccgcccag tcatcgttgt tgatgatgtc     300
   cgcgcagtac ttggcgcagc atcatcaagc atttatggcg atccttcaaa agatttcacg     360
   ctcattggag tcactggaac ctcaggtaaa accaccacca gctacctctt ggaaaaagga     420
   ctcatggagg caggccacaa agttggtttg atcggcacca caggtacacg tattgacggg     480
   gaagaagtac ccacaaagct caccactcca gaagcgccga ctctgcaggc attgtttgct     540
   cgaatgcgcg atcacggtgt cacccacgtg gtgatggaag tatccagcca tgcattgtca     600
   ttgggcagag ttgcgggttc ccactttgat gtagctgcgt ttaccaacct gtcgcaggat     660
   caccttgatt tccaccccac catggatgat tactttgacg cgaaggcatt gttcttccgc     720
   gcagattctc cacttgtggc tgacaaacag gtcgtgtgcg tggatgattc ttggggtcag     780
   cgcatggcca gcgtggcagc ggatgtgcaa acagtatcca cccttgggca agaagcagac     840
   ttcagcgcta cagacatcaa tgtcagcgac tctggcgccc agagttttaa gatcaacgcc     900
   ccctcaaacc agtcctacca ggtcgagcta gctcttccag gtgcgttcaa cgttgctaac     960
   gccacgttgg catttgccgc tgcggcacgc gtgggtgttg atggcgaagc gtttgctcga    1020
   ggcatgtcca aggtcgcggt tccaggccgt atggaacgca ttgatgaggg acaagacttc    1080
   cttgcagtgg tggattatgc ccacaagcct gctgcagtgg ctgctgtgtt ggatacgttg    1140
   aggacccaga ttgacgggcg cctcggagtg gttatcggtg ctggtggaga ccgcgattcc    1200
   accaagcgtg gccccatggg gcagttgtcc gcacagcgtg ctgatctagt tattgtcact    1260
   gatgacaacc ctcgttcaga ggtgcctgcc acgattcgcg cagcagtcac tgcaggagca    1320
   cagcagggtg cttcagagtc cgaacgaccg gtggaagtcc tagaaattgg tgaccgtgca    1380
   gaagcaattc gcgttttggt cgagtgggca cagcctggag atggcattgt agtagctgga    1440
   aaaggccatg aagttggaca actagttgct ggtgtcaccc accattttga tgaccgcgaa    1500
   gaagttcgcg ctgctttgac agaaaagctc aacaataaac ttccccttac tacggaagaa    1560
   ggatag                                                               1566